(19) Europäisches Patentamt / European Patent Office / Office européen des brevets

(11) **EP 4 589 282 A1**

(12) **EUROPEAN PATENT APPLICATION**

(43) Date of publication:
   **23.07.2025 Bulletin 2025/30**

(21) Application number: **24222775.9**

(22) Date of filing: **23.12.2024**

(51) International Patent Classification (IPC):
   **G01N 15/1429** *(2024.01)*   **G01N 15/149** *(2024.01)*
   **G01N 21/64** *(2006.01)*

(52) Cooperative Patent Classification (CPC):
   **G01N 15/1429; G01N 15/1459; G01N 15/149;**
   **G01N 21/64; G01N 21/6486;** G01N 2021/6419;
   G01N 2021/6421

(84) Designated Contracting States:
   **AL AT BE BG CH CY CZ DE DK EE ES FI FR GB**
   **GR HR HU IE IS IT LI LT LU LV MC ME MK MT NL**
   **NO PL PT RO RS SE SI SK SM TR**
   Designated Extension States:
   **BA**
   Designated Validation States:
   **GE KH MA MD TN**

(30) Priority: **18.01.2024 US 202463622370 P**

(71) Applicant: **Becton, Dickinson and Company**
   **Franklin Lakes, New Jersey 07417-1880 (US)**

(72) Inventor: **Ludington Mage, Peter**
   **San Jose, 95124 (US)**

(74) Representative: **Bals & Vogel Patentanwälte**
   **PartGmbB**
   **Konrad-Zuse-Str. 4**
   **44801 Bochum (DE)**

(54) **METHODS FOR SPECTRALLY RESOLVING FLUOROPHORES OF A SAMPLE BY GENERALIZED LEAST SQUARES AND SYSTEMS FOR SAME**

(57)   Aspects of the present disclosure include methods for spectrally resolving light from fluorophores in a sample. Methods according to certain embodiments include detecting light with a light detection system from a sample having a plurality of fluorophores having overlapping fluorescence spectra and spectrally resolving light from each fluorophore in the sample with a generalized least squares algorithm. In some embodiments, methods include estimating the abundance of one or more of the fluorophores in the sample, such as on a particle. In certain instances, methods include identifying the particle in the sample based on the abundance of each fluorophore and sorting the particle. Methods according to some embodiments includes spectrally resolving the light from each fluorophore by calculating a spectral unmixing matrix for the fluorescence spectra of each fluorophore. Systems and integrated circuit devices (e.g., a field programmable gate array) for practicing the subject methods are also provided.

Figure 1A

**Description**

**CROSS-REFERENCE TO RELATED APPLICATION**

Pursuant to 35 U.S.C. § 119 (e), this application claims priority to the filing dates of United States Provisional Patent Application Serial No 63/622,370 filed January 18, 2024, the disclosure of which application is incorporated herein by reference in their entirety.

**INTRODUCTION**

Flow-type particle sorting systems, such as sorting flow cytometers, are used to sort particles in a fluid sample based on at least one measured characteristic of the particles. In a flow-type particle sorting system, particles, such as molecules, analyte-bound beads, or individual cells, in a fluid suspension are passed in a stream by a detection region in which a sensor detects particles contained in the stream of the type to be sorted. The sensor, upon detecting a particle of the type to be sorted, triggers a sorting mechanism that selectively isolates the particle of interest.

Particle sensing typically is carried out by passing the fluid stream by a detection region in which the particles are exposed to irradiating light, from one or more lasers, and the light scattering and fluorescence properties of the particles are measured. Particles or components thereof can be labeled with fluorescent dyes to facilitate detection, and a multiplicity of different particles or components may be simultaneously detected by using spectrally distinct fluorescent dyes to label the different particles or components. Detection is carried out using one or more photosensors to facilitate the independent measurement of the fluorescence of each distinct fluorescent dye.

To sort particles in the sample, a drop charging mechanism charges droplets of the flow stream containing a particle type to be sorted with an electrical charge at the break-off point of the flow stream. Droplets are passed through an electrostatic field and are deflected based on polarity and magnitude of charge on the droplet into one or more collection containers. Uncharged droplets are not deflected by the electrostatic field.

**SUMMARY**

Aspects of the present disclosure include methods for spectrally resolving light from fluorophores in a sample. Methods according to certain embodiments include detecting light with a light detection system from a sample having a plurality of fluorophores having overlapping fluorescence spectra and spectrally resolving light from each fluorophore in the sample with a generalized least squares algorithm. In some embodiments, methods include estimating the abundance of one or more of the fluorophores in the sample, such as on a particle. In certain instances, methods include identifying the particle in the sample based on the abundance of each fluorophore and sorting the particle. Methods according to some embodiments includes spectrally resolving the light from each fluorophore by calculating a spectral unmixing matrix for the fluorescence spectra of each fluorophore. Systems and integrated circuit devices (e.g., a field programmable gate array) for practicing the subject methods are also provided.

In some embodiments, samples of interest include a plurality of fluorophores where the fluorescence spectra of each fluorophore overlaps with the fluorescence spectra of at least one other fluorophore in the sample. In certain instances, the fluorescence spectra of each fluorophore overlaps with the fluorescence spectra of at least one other fluorophore in the sample by 10 nm or more, such as 25 nm or more and including by 50 nm or more. In some instances, the fluorescence spectra of one or more fluorophores in the sample overlaps with the fluorescence spectra of two different fluorophores in the sample, such as by 10 nm or more, such as by 25 nm or more and including by 50 nm or more. In other embodiments, samples of interest include a plurality of fluorophores having non-overlapping fluorescence spectra. In these embodiments, the fluorescence spectra of each fluorophore is adjacent to at least one other fluorophore within 10 nm or less, such as 9 nm or less, such as 8 nm or less, such as 7 nm or less, such as 6 nm or less, such as 5 nm or less, such as 4 nm or less, such as 3 nm or less, such as 2 nm or less and including 1 nm or less.

In some embodiments, light from the sample is detected by the light detection system in one or more photodetector channels, such as in a plurality of photodetection channels. In some instances, the light is detected with a plurality of photodetectors. In some instances, data signals are generated in each of the photodetector channels in response to the detected light. In some embodiments, light is spectrally resolved for each fluorophore in real time. In some instances, methods include spectrally resolving the light in real time with an integrated circuit, such as a field programmable gate array (FPGA).

In some embodiments, data signal covariance is determined in each photodetector channel. In some instances, the data signal covariance in each photodetector channel includes one or more of an intrinsic sample variability component and a measurement variability component. In some instances, the data signal covariance includes electronic noise in each photodetector channel. In some instances, the data signal covariance includes shot noise in each photodetector channel. In some instances, the data signal covariance varies linearly with the generated data signals. In some instances, the data signal covariance varies quadratically with the generated data signals. In some instances, the data signal covariance is correlated across two or more of the plurality of photodetector channels. In some instances, the data signal covariance is calculated according to:

$$V[y_i] = \sigma_{B,i}^2 + Q_i y_i + (CV_{0,i})^2 y_i^2$$

where:

$y_i$ is a measured detector signal;

$\sigma_{B,i}^2$ is a baseline noise component;
$Q_i y_i$ is a Poisson noise component;
$Q_i$ is a Poisson noise coefficient;

$(CV_{0,i})^2 y_i^2$ is a quadratic noise component; and
$CV_{0,i}$ is a quadratic noise coefficient.

In some embodiments, the data signal covariance is calculated using a covariance matrix. In some instances, the covariance matrix contains non-zero diagonal values. In certain instances, the covariance is calculated with a covariance matrix according to:

$$V[y] = \Sigma^y = \begin{bmatrix} V[y_1] & cov(y_1, y_2) & \cdots & cov(y_1, y_m) \\ cov(y_2, y_1) & V[y_2] & \cdots & cov(y_2, y_m) \\ \vdots & \vdots & \ddots & \vdots \\ cov(y_m, y_1) & cov(y_m, y_2) & \cdots & V[y_m] \end{bmatrix}$$

$$= diag(\sigma_B^2) + diag(Q)diag(y) + \begin{bmatrix} CV_{0,1}^2 y_1^2 & cov(y_1, y_2) & \cdots & cov(y_1, y_m) \\ cov(y_2, y_1) & CV_{0,2}^2 y_2^2 & \cdots & cov(y_2, y_m) \\ \vdots & \vdots & \ddots & \vdots \\ cov(y_m, y_1) & cov(y_m, y_2) & \cdots & CV_{0,m}^2 y_m^2 \end{bmatrix}$$

wherein *diag(x)* indicates generating a diagonal matrix from the column vector *x,* and *cov(a, b)* denotes the covariance between *a* and *b*.

In some instances, the generalized least squares problem is calculated by multiplying by the inverse of the calculated covariance matrix. In some instances, methods include estimating data signal covariance based on fluorescence intensity across two or more photodetectors of the light detection system. In some instances, methods include estimating data signal covariance based on fluorescence intensity across each of the photodetection channels. In some embodiments, the generalized least squares problem is calculated according to:

$$\hat{f} = \arg\min_f (Xf - y)^T G(Xf - y)$$

wherein:

$G = (\Sigma^y)^{-1}$ is a weight matrix;
$\Sigma^y$ is a covariance matrix;
$X$ is a spectral matrix (spillover matrix);
$y$ is measured detector values from the plurality of photodetectors of the light detection system for each cell;
$f$ is true fluorophore abundances for each cell; and
$\hat{f}$ is estimated (unmixed) fluorophore abundances for each cell.

In some instances, the method further includes generating *a priori* an estimated covariance matrix. In certain instances, the generalized least squares problem is applied in real time (e.g., using an integrated circuit such as a field programmable gate array). In some instances, an *a priori* noise model is used to estimate each event covariance (e.g., calculate each event covariance matrix in real time). In certain instances, the *a priori* noise model uses only data collected for each specific event. In some instances, the covariance matrix includes estimations from an entire collected dataset. In some instances, the covariance matrix is generated through iterative optimization methods which empirically tune the covariance matrix to minimize the variance of the unmixed data.

In some embodiments, data signal covariance is determined by estimating by iterative optimization with a covariance matrix that minimizes variance of unmixed data signals. In certain instances, the generalized least squares problem is characterized by Cholesky decomposition of the covariance matrix. In certain instances, the generalized least squares problem is characterized by computing the Cholesky decomposition of the covariance matrix $\Sigma^y = CC^T$ and solving triangular systems to generate transformed inputs for an ordinary least squares algorithm according to:

Solving $C\overline{y} = y$ to generate $\overline{y}$, a decorrelated and whitened version of the data vector $y$.
Solving $C\overline{X} = X$ to generate $\overline{X}$, the corresponding transformed version of the spectral matrix $X$.
The transformed system $\overline{X}f = \overline{y}$ is solved by ordinary least squares, for example by solving the so-called normal equations $\overline{X^T}\overline{X}\hat{f} = \overline{X^T}\overline{y}$.

In some embodiments, methods include finding the least-squares solution of the generalized least squares problem. In some instances, the least-squares solution of the generalized least squares problem is found by one or more of matrix decomposition, matrix factorization, QR factorization, Cholesky decomposition, singular value decomposition, LDL decomposition, and forward- and back-substitution. In some instances, methods include finding the least-squares solution of the generalized least squares problem by solving the so-called normal equations via Cholesky or LDL decomposition, such as by Cholesky or LDL decomposition according to:

$$X^T G X \hat{f} = X^T G y$$

Where $y$ is measured detector values from the plurality of photodetectors of the light detection system for each cell; $X$ is spillover; and

$$G \text{ is } \begin{bmatrix} G_{1,1} & G_{1,2} & \cdots & G_{1,m} \\ G_{2,1} & G_{2,2} & \cdots & G_{2,m} \\ \vdots & \vdots & \ddots & \vdots \\ G_{m,1} & G_{m,2} & \cdots & G_{m,m} \end{bmatrix}$$

In some instances, methods include finding the least-squares solution of the generalized least squares problem by Cholesky or LDL decomposition according to:

$$X^T G X \hat{f} = X^T G y$$

$A\hat{f} = B$
$LDL^T\hat{f} = B$                      LDL decomposition
$Lz = B$ where $z = DL^T\hat{f}$        Lower-triangular matrix solution
$Dx = z$ where $x = L^T\hat{f}$         Diagonal matrix solution
$L^T\hat{f} = x$, solve for $\hat{f}$            Upper-triangular matrix solution

In some embodiments, methods include finding the least-squares solution of the generalized least squares problem by matrix decomposition.

In some embodiments, methods include finding the least-squares solution of the generalized least squares problem by matrix factorization. In some instances, the least-squares solution of the generalized least squares problem is found by QR factorization. In some instances, the generalized least squares problem is calculated using transformed $\overline{X}$ and $\overline{y}$ (as calculated via Cholesky decomposition of the covariance matrix described above) using QR factorization according to:

$\overline{X^T}\overline{X}f = \overline{X^T}\overline{y}$            Normal equations for transformed GLS problem
$QR = \overline{X}$                    QR decomposition of transformed $X$
$(QR)^T QR\hat{f} = (QR)^T\overline{y}$           Substitution
$R^T Q^T QR\hat{f} = R^T Q^T\overline{y}$         Expand transpose of $QR$
$R^T R\hat{f} = R^T Q^T\overline{y}Q^T Q$      cancels due to orthogonality

(continued)

$$R\hat{f} = Q^T\overline{y}.$$ Triangular solve for $\hat{f}$

In some embodiments, methods include finding the least-squares solution of the generalized least squares problem by singular value decomposition. In some instances, the singular value decomposition is the matrix that is the product $\overline{X} = U\Sigma V^T$ where $U$ and $V$ are orthogonal matrices and $\Sigma$ is a diagonal matrix containing singular values of $\overline{X}$. In some instances, the generalized least squares problem is calculated using singular value decomposition according to:

$$z = U^T\overline{y}$$

$$\Sigma w = z$$

$$\hat{f} = Vw,$$

where $U$ and $V$ are orthogonal matrices and $\Sigma$ is a diagonal matrix containing singular values of $\overline{X}$. In some embodiments, methods include finding the least-squares solution of the generalized least squares problem by LDL decomposition. In certain embodiments, methods include finding the least-squares solution of the generalized least squares problem by forward- and back-substitution.

Systems for practicing the subject methods are also provided. Systems according to certain embodiments include a light source configured to irradiate a sample having a plurality of fluorophores that have overlapping fluorescence spectra; a light detection system comprising a plurality of photodetectors; and a processor with memory operably coupled to the processor where the memory includes instructions stored thereon, which when executed by the processor, cause the processor to spectrally resolve light from each fluorophore in the sample using a generalized least squares problem. In some instances, the system is configured detect light by the light detection system in one or more photodetector channels, such as in a plurality of photodetector channels. In some instances, systems include a plurality of photodetectors. In some instances, the photodetectors include one or more photomultiplier tubes. In some instances, the light detection system includes a photodetector array. In some instances, the photodetector array includes charged coupled devices.

In some embodiments, the memory includes instructions stored thereon, which when executed by the processor, cause the processor to determine data signal covariance in each photodetector channel. In some instances, the data signal covariance in each photodetector channel includes an intrinsic sample variability component and a measurement variability component. In some instances, the data signal covariance includes electronic noise in each photodetector channel. In some instances, the data signal covariance includes shot noise in each photodetector channel. In some instances, the data signal covariance varies linearly with the generated data signals. In some instances, the data signal covariance varies quadratically with the generated data signals. In some instances, the data signal covariance is correlated across two or more of the plurality of photodetector channels. In some instances, the memory includes instructions to calculate the data signal covariance according to:

$$V[y_i] = \sigma_{B,i}^2 + Q_i y_i + (CV_{0,i})^2 y_i^2$$

where:

$y_i$ is a measured detector signal;

$\sigma_{B,i}^2$ is a baseline noise component;

$Q_i y_i$ is a Poisson noise component;

$Q_i$ is a Poisson noise coefficient;

$(CV_{0,i})^2 y_i^2$ is a quadratic noise component; and

$CV_{0,i}$ is a quadratic noise coefficient.

In some instances, the memory includes instructions to calculate the data signal covariance using a covariance matrix. In some instances, the covariance matrix contains non-zero diagonal values. In some instances, the memory includes instructions to calculate the covariance with a covariance matrix according to:

$$V[y] = \Sigma^y = \begin{bmatrix} V[y_1] & cov(y_1, y_2) & \cdots & cov(y_1, y_m) \\ cov(y_2, y_1) & V[y_2] & \cdots & cov(y_2, y_m) \\ \vdots & \vdots & \ddots & \vdots \\ cov(y_m, y_1) & cov(y_m, y_2) & \cdots & V[y_m] \end{bmatrix}$$

$$= diag(\sigma_B^2) + diag(Q)diag(y) + \begin{bmatrix} CV_{0,1}^2 y_1^2 & cov(y_1, y_2) & \cdots & cov(y_1, y_m) \\ cov(y_2, y_1) & CV_{0,2}^2 y_2^2 & \cdots & cov(y_2, y_m) \\ \vdots & \vdots & \ddots & \vdots \\ cov(y_m, y_1) & cov(y_m, y_2) & \cdots & CV_{0,m}^2 y_m^2 \end{bmatrix}$$

wherein *diag(x)* indicates generating a diagonal matrix from the column vector *x*, and *cov(a, b)* denotes the covariance between *a* and *b*.

In some instances, the memory includes instructions to calculate the generalized least squares problem by multiplying by the inverse of the calculated covariance matrix. In some instances, the memory includes instructions to estimate data signal covariance based on fluorescence intensity across two or more photodetectors of the light detection system. In some instances, the memory includes instructions to estimate data signal covariance based on fluorescence intensity across each of the photodetection channels.

In some embodiments, the memory includes instructions to calculate the generalized least squares problem according to:

$$\hat{f} = \arg\min_{f}(Xf - y)^T G(Xf - y)$$

wherein:

$G = (\Sigma^y)^{-1}$ is a weight matrix;
$\Sigma^y$ is a covariance matrix;
*X* is a spectral matrix (spillover matrix);
*y* is measured detector values from the plurality of photodetectors of the light detection system for each cell;
*f* is true fluorophore abundances for each cell; and
$\hat{f}$ is estimated (unmixed) fluorophore abundances for each cell.

In some instances, the memory includes instructions to estimate a covariance matrix *a priori*. In certain instances, the memory includes instructions to apply the generalized least squares problem in real time (e.g., using an integrated circuit such as a field programmable gate array). In some instances, an *a priori* noise model is used to estimate each event covariance (e.g., calculate each event covariance matrix in real time). In certain instances, the *a priori* noise model uses only data collected for each specific event. In some instances, the covariance matrix includes estimations from an entire collected dataset.

In some embodiments, the memory includes instructions to determine data signal covariance by estimating by iterative optimization with a covariance matrix that minimizes variance of unmixed data signals. In certain instances, the memory includes instructions to calculate the generalized least squares problem by Cholesky decomposition of the covariance matrix. In certain instances, the generalized least squares problem is characterized by computing the Cholesky decomposition of the covariance matrix $\Sigma^y = CC^T$ and solving triangular systems to generate transformed inputs for an ordinary least squares algorithm according to:

Solving $C\overline{y} = y$ to generate $\overline{y}$, a decorrelated and whitened version of the data vector *y*.
Solving $C\overline{X} = X$ to generate $\overline{X}$, the corresponding transformed version of the spectral matrix *X*.
The transformed system $\overline{X}f = \overline{y}$ is solved by ordinary least squares, for example by solving the so-called normal equations $\overline{X}^T\overline{X}\hat{f} = \overline{X}^T\overline{y}$.

In some embodiments, the memory includes instructions to find the least-squares solution of the generalized least squares problem. In some instances, the memory includes instructions to find the least-squares solution of the generalized least squares problem by one or more of matrix decomposition, matrix factorization, QR factorization, Cholesky decomposition, singular value decomposition, LDL decomposition, and forward- and back-substitution. In some instances, the memory includes instructions to find the least-squares solution of the generalized least squares problem by solving the so-called normal equations via Cholesky or LDL decomposition, such as by Cholesky or LDL decomposition according to:

$$X^T G X \hat{f} = X^T G y$$

where $y$ is measured detector values from the plurality of photodetectors of the light detection system for each cell; $X$ is spillover; and

$$G \text{ is } \begin{bmatrix} G_{1,1} & G_{1,2} & \cdots & G_{1,m} \\ G_{2,1} & G_{2,2} & \cdots & G_{2,m} \\ \vdots & \vdots & \ddots & \vdots \\ G_{m,1} & G_{m,2} & \cdots & G_{m,m} \end{bmatrix}$$

In some instances, the memory includes instructions to find the least-squares solution of the generalized least squares problem by Cholesky or LDL decomposition according to:

$$X^T G X \hat{f} = X^T G y$$

$A\hat{f} = B$
$LDL^T \hat{f} = B$            LDL decomposition
$Lz = B$ where $z = DL^T \hat{f}$      Lower-triangular matrix solution
$Dx = z$ where $x = L^T \hat{f}$      Diagonal matrix solution
$L^T \hat{f} = x$, solve for $\hat{f}$       Upper-triangular matrix solution

In some embodiments, the memory includes instructions to find the least-squares solution of the generalized least squares problem by matrix decomposition.

In some embodiments, the memory includes instructions to find the least-squares solution of the generalized least squares problem by matrix factorization. In some instances, the memory includes instructions to find the least-squares solution of the generalized least squares problem by QR factorization. In some instances, the memory includes instructions to calculate the generalized least squares problem using transformed $\overline{X}$ and $\overline{y}$ (as calculated via Cholesky decomposition of the covariance matrix described above) using QR factorization according to:

$\overline{X}^T \overline{X} f = \overline{X}^T \overline{y}$         Normal equations for transformed GLS problem
$QR = \overline{X}$              QR decomposition of transformed $X$
$(QR)^T QR \hat{f} = (QR)^T \overline{y}$      Substitution
$R^T Q^T QR \hat{f} = R^T Q^T \overline{y}$      Expand transpose of $QR$
$R^T R \hat{f} = R^T Q^T \overline{y} Q^T Q$     cancels due to orthogonality
$R \hat{f} = Q^T \overline{y}$.             Triangular solve for $\hat{f}$

In some embodiments, the memory includes instructions to find the least-squares solution of the generalized least squares problem by singular value decomposition. In some instances, the singular value decomposition is the matrix that is the product $\overline{X} = U\Sigma V^T$ where $U$ and $V$ are orthogonal matrices and $\Sigma$ is a diagonal matrix containing singular values of $\overline{X}$. In some instances, the memory includes instructions to calculate the generalized least squares problem using singular value decomposition according to:

$$z = U^T \overline{y}$$

$$\Sigma W = z$$

$$\hat{f} = Vw,$$

where $U$ and $V$ are orthogonal matrices and $\Sigma$ is a diagonal matrix containing singular values of $\overline{X}$. In some embodiments, the memory includes instructions to find the least-squares solution of the generalized least squares problem by LDL decomposition. In certain embodiments, the memory includes instructions to find the least-squares solution of the generalized least squares problem by forward- and back-substitution.

In some embodiments, systems include a processor with memory operably coupled to the processor where the memory includes instructions stored thereon, which when executed by the processor, cause the processor to estimate an abundance of one or more of the fluorophores in the sample. In certain instances, the memory includes instructions for estimating the abundance of one or more fluorophores on a particle in the sample. In some embodiments the memory includes instructions for identifying a particle in the sample based on the estimated abundance of each fluorophore on the particle. In certain instances, systems include a particle sorter for sorting identified particles in the sample.

Integrated circuit devices programmed to spectrally resolve light from a plurality of fluorophores in a sample using a generalized least squares problem are also provided. In embodiments, the integrated circuit device may be a field programmable gated array (FPGA), an application specific integrated circuit (ASIC) or a complex programmable logic device (CPLD), or some other integrated circuit device.

In some embodiments, the integrated circuit device is programmed to determine data signal covariance in each photodetector channel. In some instances, the data signal covariance in each photodetector channel includes an intrinsic sample variability component and a measurement variability component. In some instances, the data signal covariance includes electronic noise in each photodetector channel. In some instances, the data signal covariance includes shot noise in each photodetector channel. In some instances, the data signal covariance varies linearly with the generated data signals. In some instances, the data signal covariance varies quadratically with the generated data signals. In some instances, the data signal covariance is correlated across two or more of the plurality of photodetector channels. In some instances, the integrated circuit device is programmed to calculate the data signal covariance according to:

$$V[y_i] = \sigma_{B,i}^2 + Q_i y_i + (CV_{0,i})^2 y_i^2$$

where:

$y_i$ is a measured detector signal;

$\sigma_{B,i}^2$ is a baseline noise component;

$Q_i y_i$ is a Poisson noise component;

$Q_i$ is a Poisson noise coefficient;

$(CV_{0,i})^2 y_i^2$ is a quadratic noise component; and

$CV_{0,i}$ is a quadratic noise coefficient.

In some instances, the integrated circuit device is programmed to calculate the data signal covariance using a covariance matrix. In some instances, the covariance matrix contains non-zero diagonal values. In certain instances, the integrated circuit device is programmed to calculate the covariance with a covariance matrix according to:

$$V[y] = \Sigma^y = \begin{bmatrix} V[y_1] & cov(y_1, y_2) & \cdots & cov(y_1, y_m) \\ cov(y_2, y_1) & V[y_2] & \cdots & cov(y_2, y_m) \\ \vdots & \vdots & \ddots & \vdots \\ cov(y_m, y_1) & cov(y_m, y_2) & \cdots & V[y_m] \end{bmatrix}$$

$$= diag(\sigma_B^2) + diag(Q)diag(y) + \begin{bmatrix} CV_{0,1}^2 y_1^2 & cov(y_1, y_2) & \cdots & cov(y_1, y_m) \\ cov(y_2, y_1) & CV_{0,2}^2 y_2^2 & \cdots & cov(y_2, y_m) \\ \vdots & \vdots & \ddots & \vdots \\ cov(y_m, y_1) & cov(y_m, y_2) & \cdots & CV_{0,m}^2 y_m^2 \end{bmatrix}$$

where $diag(x)$ indicates generating a diagonal matrix from the column vector $x$, and $cov(a, b)$ denotes the covariance between $a$ and $b$.

In some instances, the integrated circuit device is programmed to calculate the generalized least squares problem by multiplying by the inverse of the calculated covariance matrix. In some instances, the integrated circuit device is programmed to estimate data signal covariance based on fluorescence intensity across two or more photodetectors of the light detection system. In some instances, the integrated circuit device is programmed to estimate data signal covariance based on fluorescence intensity across each of the photodetection channels. In some embodiments, the integrated circuit device is programmed to calculate the generalized least squares problem according to:

$$\hat{f} = \arg\min_{f}(Xf - y)^T G(Xf - y)$$

where:

$G = (\Sigma^y)^{-1}$ is a weight matrix;
$\Sigma^y$ is a covariance matrix;
$X$ is a spectral matrix (spillover matrix);
$y$ is measured detector values from the plurality of photodetectors of the light detection system for each cell;
$f$ is true fluorophore abundances for each cell; and
$\hat{f}$ is estimated (unmixed) fluorophore abundances for each cell.

In some instances, the integrated circuit device is programmed to estimate a covariance matrix *a priori*. In certain instances, the integrated circuit device is programmed to apply the generalized least squares problem in real time (e.g., using an integrated circuit such as a field programmable gate array). In some instances, an *a priori* noise model is used to estimate each event covariance (e.g., calculate each event covariance matrix in real time). In certain instances, the *a priori* noise model uses only data collected for each specific event. In some instances, the covariance matrix includes estimations from an entire collected dataset. In some instances, the covariance matrix is generated through iterative optimization methods which empirically tune the covariance matrix to minimize the variance of the unmixed data.

In some embodiments, the integrated circuit device is programmed to determine data signal covariance by estimating by iterative optimization with a covariance matrix that minimizes variance of unmixed data signals. In certain instances, the integrated circuit device is programmed to calculate the generalized least squares problem by Cholesky decomposition of the covariance matrix. In certain instances, the generalized least squares problem is characterized by computing the Cholesky decomposition of the covariance matrix $\Sigma^y = CC^T$ and solving triangular systems to generate transformed inputs for an ordinary least squares algorithm according to:

Solving $C\overline{y} = y$ to generate $\overline{y}$, a decorrelated and whitened version of the data vector *y*.
Solving $C\overline{X} = X$ to generate $\overline{X}$, the corresponding transformed version of the spectral matrix *X*.
The transformed system $\overline{X}f = \overline{y}$ is solved by ordinary least squares, for example by solving the so-called normal equations $\overline{X}^T\overline{X}\hat{f} = \overline{X}^T\overline{y}$.

In some embodiments, the integrated circuit device is programmed to find the least-squares solution of the generalized least squares problem. In some instances, the integrated circuit device is programmed to find the least-squares solution of the generalized least squares problem by one or more of matrix decomposition, matrix factorization, QR factorization, Cholesky decomposition, singular value decomposition, LDL decomposition, and forward- and back-substitution. In some instances, the integrated circuit device is programmed to find the least-squares solution of the generalized least squares problem by solving the so-called normal equations via Cholesky or LDL decomposition, such as by Cholesky or LDL decomposition according to:

$$X^T G X \hat{f} = X^T G y$$

where *y* is measured detector values from the plurality of photodetectors of the light detection system for each cell; *X* is spillover; and

$$G \text{ is } \begin{bmatrix} G_{1,1} & G_{1,2} & \cdots & G_{1,m} \\ G_{2,1} & G_{2,2} & \cdots & G_{2,m} \\ \vdots & \vdots & \ddots & \vdots \\ G_{m,1} & G_{m,2} & \cdots & G_{m,m} \end{bmatrix}$$

In some instances, the integrated circuit device is programmed to find the least-squares solution of the generalized least squares problem by Cholesky or LDL decomposition according to:

$$X^T G X \hat{f} = X^T G y$$

$$A\hat{f} = B$$

(continued)

| | |
|---|---|
| $LDL^T\hat{f} = B$ | LDL decomposition |
| $Lz = B$ where $z = DL^T\hat{f}$ | Lower-triangular matrix solution |
| $Dx = z$ where $x = L^T\hat{f}$ | Diagonal matrix solution |
| $L^T\hat{f} = x$, solve for $\hat{f}$ | Upper-triangular matrix solution |

In some embodiments, the integrated circuit device is programmed to find the least-squares solution of the generalized least squares problem by matrix decomposition.

In some embodiments, the integrated circuit device is programmed to find the least-squares solution of the generalized least squares problem by matrix factorization. In some instances, the integrated circuit device is programmed to find the least-squares solution of the generalized least squares problem by QR factorization. In some instances, the integrated circuit device is programmed to calculate the generalized least squares problem using transformed $\overline{X}$ and $\overline{y}$ (as calculated via Cholesky decomposition of the covariance matrix described above) using QR factorization according to:

| | |
|---|---|
| $\overline{X}^T \overline{X}f = \overline{X}^T\overline{y}$ | Normal equations for transformed GLS problem |
| $QR = \overline{X}$ | QR decomposition of transformed $X$ |
| $(QR)^T QR\hat{f} = (QR)^T\overline{y}$ | Substitution |
| $R^T Q^T QR\hat{f} = R^T Q^T\overline{y}$ | Expand transpose of $QR$ |
| $R^T R\hat{f} = R^T Q^T\overline{y}Q^T Q$ | cancels due to orthogonality |
| $R\hat{f} = Q^T\overline{y}.$ | Triangular solve for $\hat{f}$ |

In some embodiments, the integrated circuit device is programmed to minimize the least-squares solution of the generalized least squares problem by singular value decomposition. In some instances, the singular value decomposition is the matrix that is the product $\overline{X} = U\Sigma V^T$ where $U$ and $V$ are orthogonal matrices and $\Sigma$ is a diagonal matrix containing singular values of $\overline{X}$. In some instances, the integrated circuit device is programmed to calculate the generalized least squares problem using singular value decomposition according to:

$$z = U^T\overline{y}$$

$$\Sigma W = z$$

$$\hat{f} = Vw,$$

where $U$ and $V$ are orthogonal matrices and $\Sigma$ is a diagonal matrix containing singular values of $\overline{X}$. In some embodiments, the integrated circuit device is programmed to find the least-squares solution of the generalized least squares problem by LDL decomposition. In certain embodiments, the integrated circuit device is programmed to find the least-squares solution of the generalized least squares problem by forward- and back-substitution.

In some embodiments, the integrated circuit devices are programmed to estimate an abundance of one or more of the fluorophores in the sample based on the calculated spectral unmixing matrix. In certain instances, the integrated circuit device is programmed to estimate the abundance of one or more fluorophores on a particle in the sample. In some embodiments, the integrated circuit devices are programmed to identify a particle in the sample based on the estimated abundance of each fluorophore on the particle. In certain instances, the integrated circuit devices are programmed to sort the identified particles in the sample.

**BRIEF DESCRIPTION OF THE FIGURES**

The invention may be best understood from the following detailed description when read in conjunction with the accompanying drawings. Included in the drawings are the following figures:

FIG. 1A depicts a flow diagram for spectrally resolving light using a generalized least squares problem according to certain embodiments.
FIG. 1B depicts the raw measurement variance from particles in a sample irradiated with light according to certain embodiments.
FIG. 1C depicts determining the quadratic noise coefficients in measurement covariance according to certain embodiments.

**FIG. 1D** depicts the estimation of unmixed spread in the absence of (i.e., zero) covariance.

**FIG. 2A** depicts a comparison of correlation and anti-correlation in quadratic noise according to certain embodiments.

**FIG. 2B** depicts a comparison of spectral unmixing using a generalized least squares problem according to certain embodiments with unmixing with ordinary least squares and weighted least squares.

**FIG. 3A** depicts an image-enabled particle sorter according to certain embodiments. **FIG. 3B** depicts image-enabled particle sorting data processing according to certain embodiments.

**FIG. 4A** depicts a functional block diagram of a particle analysis system according to certain embodiments. **FIG. 4B** depicts a flow cytometer according to certain embodiments.

**FIG.** 5 depicts a functional block diagram for one example of a particle analyzer control system according to certain embodiments.

**FIG. 6A** depicts a schematic drawing of a particle sorter system according to certain embodiments.

**FIG. 6B** depicts a schematic drawing of a particle sorter system according to certain embodiments.

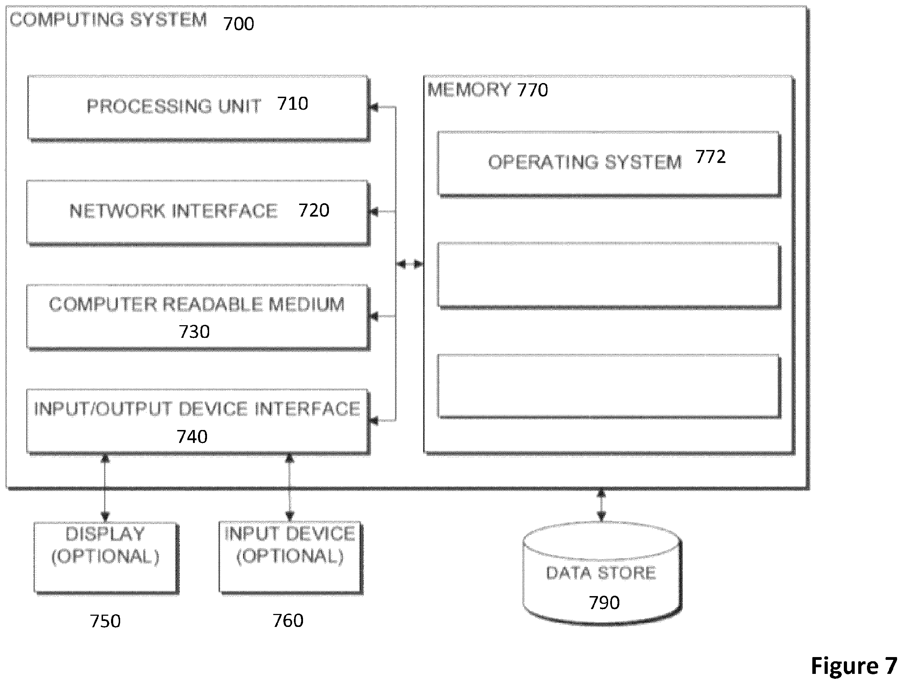

**FIG.** 7 depicts a block diagram of a computing system according to certain embodiments.

## DETAILED DESCRIPTION

Aspects of the present disclosure include methods for spectrally resolving light from fluorophores in a sample. Methods according to certain embodiments include detecting light with a light detection system from a sample having a plurality of fluorophores having overlapping fluorescence spectra and spectrally resolving light from each fluorophore in the sample with a generalized least squares problem. In some embodiments, methods include estimating the abundance of one or more of the fluorophores in the sample, such as on a particle. In certain instances, methods include identifying the particle in the sample based on the abundance of each fluorophore and sorting the particle. Methods according to some embodiments includes spectrally resolving the light from each fluorophore by calculating a spectral unmixing matrix for the fluorescence spectra of each fluorophore. Systems and integrated circuit devices (e.g., a field programmable gate array) for practicing the subject methods are also provided.

Before the present invention is described in greater detail, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible.

While the apparatus and method has or will be described for the sake of grammatical fluidity with functional explanations, it is to be expressly understood that the claims, unless expressly formulated under 35 U.S.C. §112, are not to be construed as necessarily limited in any way by the construction of "means" or "steps" limitations, but are to be accorded the full scope of the meaning and equivalents of the definition provided by the claims under the judicial doctrine of equivalents, and in the case where the claims are expressly formulated under 35 U.S.C. §112 are to be accorded full statutory equivalents under 35 U.S.C. §112.

As summarized above, the present disclosure provides methods for spectrally resolving fluorophores in a sample by a generalized least squares algorithm. In further describing embodiments of the disclosure, methods for spectrally resolving fluorophores in a sample, including estimating an abundance of each fluorophore in the sample (e.g., on a particle in the sample) as well as identifying and sorting particles based on the estimated abundance of each fluorophore are first described in greater detail. Next, systems and integrated circuited devices programmed to practice the subject methods using a generalized least squares algorithm, are described.

## METHODS FOR SPECTRALLY RESOLVING LIGHT WITH A GENERALIZED LEAST SQUARES ALGORITHM FROM FLUOROPHORES HAVING OVERLAPPING FLUORESCENCE SPECTRA IN A SAMPLE

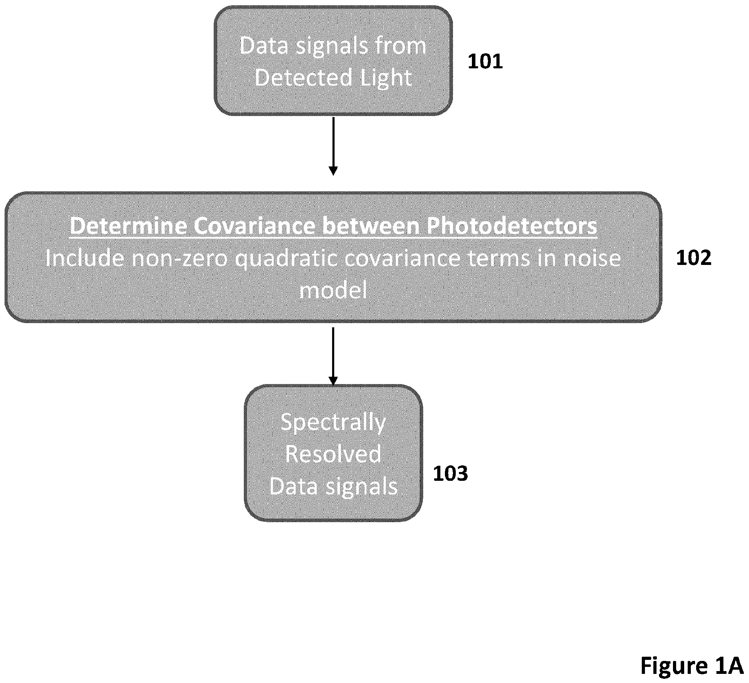

Aspects of the present disclosure include methods for spectrally resolving light from fluorophores, including light having overlapping fluorescence spectra, in a sample. In embodiments, light from each fluorophore is resolved (e.g., unmixed) using a generalized least squares algorithm. In some instances, spectral unmixing with light from fluorophores with the generalized least squares algorithm models covariance arising from measurement variation in a particle analyzer, such as a flow cytometer. A generalized least squares algorithm in certain embodiments provides for lower variance in unmixed data than spectrally resolving light from fluorophores using an ordinary least squares algorithm or a weighted least squares algorithm. In some instances, spectral unmixing is more precise as compared to weighted least squares algorithms when measurement noise is correlated across photodetectors in a light detection system. In some embodiments, the methods described herein provide for measurement data from a sample which accounts for heteroscedasticity and nonzero covariance in the measured data. In some instances, the generalized least squares algorithm described herein uses the inverse of an estimated covariance matrix for each event as a weight matrix which whitens and decorrelates the data, ensuring the optimality (minimum variance) of the least-squares solution. In certain instances, the subject methods estimate covariance arising from measurement variability alone, without the effect of intrinsic sample variability. Figure 1A depicts a flow diagram for spectrally resolving light using a generalized least squares algorithm according to certain embodiments. As discussed in greater detail below, data signals are generated from light detected from particles irradiated in a flow stream (101) and covariance between photodetectors is determined (102) on a per-event basis (i.e., a measurement variance is calculated for each particle). Data signals are spectrally resolved using the calculated measurement covariance (103) with the generalized least squares algorithm which applies the calculated measurement covariance.

The term "spectrally resolving" is used herein in its conventional sense to refer to spectrally distinguishing each fluorophore in the sample by assigning or attributing the overlapping wavelengths of light to each contributing fluorophore. In embodiments, the overlapping spectral component of fluorescence that is attributed to each fluorophore is determined by finding the solution to the generalized least squares problem. In certain instances, the generalized least squares algorithm (as described in greater detail below) includes calculating a spectral unmixing matrix. In some embodiments, samples of interest have a plurality of fluorophores where the fluorescence spectra of each fluorophore overlaps with the fluorescence spectra of at least one other fluorophore in the sample. In some instances, the fluorescence spectra of each fluorophore overlaps with the fluorescence spectra of at least one other fluorophore in the sample by 5 nm or more, such as by 10 nm or more, such as by 25 nm or more and including by 50 nm or more. In certain instances, the fluorescence spectra of one or more fluorophores in the sample overlaps with the fluorescence spectra of two or more different fluorophores in the sample, such as where each overlap in fluorescence spectra is by 5 nm or more, such as by 10 nm or more, such as by 25 nm or more and including by 50 nm or more. In other embodiments, samples of interest include a plurality of fluorophores having non-overlapping fluorescence spectra. In these embodiments, the fluorescence spectra of each fluorophore is adjacent to at least one other fluorophore within 10 nm or less, such as 9 nm or less, such as 8 nm or less, such as 7 nm or less, such as 6 nm or less, such as 5 nm or less, such as 4 nm or less, such as 3 nm or less, such as 2 nm or less and including 1 nm or less.

In practicing the subject methods, a sample is irradiated with a light source and light from the sample is detected with a light detection system having one or more photodetectors. In some instances, the light detection system includes a plurality of photodetectors. In some embodiments, the sample is a biological sample. The term "biological sample" is used in its conventional sense to refer to a whole organism, plant, fungi or a subset of animal tissues, cells or component parts which may in certain instances be found in blood, mucus, lymphatic fluid, synovial fluid, cerebrospinal fluid, saliva, bronchoalveolar lavage, amniotic fluid, amniotic cord blood, urine, vaginal fluid and semen. As such, a "biological sample" refers to both the native organism or a subset of its tissues as well as to a homogenate, lysate or extract prepared from the organism or a subset of its tissues, including but not limited to, for example, plasma, serum, spinal fluid, lymph fluid, sections of the skin, respiratory, gastrointestinal, cardiovascular, and genitourinary tracts, tears, saliva, milk, blood cells, tumors, organs. Biological samples may be any type of organismic tissue, including both healthy and diseased tissue (e.g., cancerous, malignant, necrotic, etc.). In certain embodiments, the biological sample is a liquid sample, such as blood or derivative thereof, e.g., plasma, tears, urine, semen, etc., where in some instances the sample is a blood sample, including whole blood, such as blood obtained from venipuncture or fingerstick (where the blood may or may not be combined with any reagents prior to assay, such as preservatives, anticoagulants, etc.).

In certain embodiments the source of the sample is a "mammal" or "mammalian", where these terms are used broadly to describe organisms which are within the class mammalia, including the orders carnivore (e.g., dogs and cats), rodentia (e.g., mice, guinea pigs, and rats), and primates (e.g., humans, chimpanzees, and monkeys). In some instances, the subjects are humans. The methods may be applied to samples obtained from human subjects of both genders and at any stage of development (i.e., neonates, infant, juvenile, adolescent, adult), where in certain embodiments the human subject is a juvenile, adolescent or adult. While the present invention may be applied to samples from a human subject, it is to be understood that the methods may also be carried-out on samples from other animal subjects (that is, in "non-human subjects") such as, but not limited to, birds, mice, rats, dogs, cats, livestock and horses.

In practicing the subject methods, a sample (e.g., in a flow stream of a flow cytometer) is irradiated with light from a light source. In some embodiments, the light source is a broadband light source, emitting light having a broad range of wavelengths, such as for example, spanning 50 nm or more, such as 100 nm or more, such as 150 nm or more, such as 200 nm or more, such as 250 nm or more, such as 300 nm or more, such as 350 nm or more, such as 400 nm or more and including spanning 500 nm or more. For example, one suitable broadband light source emits light having wavelengths from 200 nm to 1500 nm. Another example of a suitable broadband light source includes a light source that emits light having wavelengths from 400 nm to 1000 nm. Where methods include irradiating with a broadband light source, broadband light source protocols of interest may include, but are not limited to, a halogen lamp, deuterium arc lamp, xenon arc lamp, stabilized fiber-coupled broadband light source, a broadband LED with continuous spectrum, superluminescent emitting diode, semiconductor light emitting diode, wide spectrum LED white light source, an multi-LED integrated white light source, among other broadband light sources or any combination thereof.

In other embodiments, methods includes irradiating with a narrow band light source emitting a particular wavelength or a narrow range of wavelengths, such as for example with a light source which emits light in a narrow range of wavelengths like a range of 50 nm or less, such as 40 nm or less, such as 30 nm or less, such as 25 nm or less, such as 20 nm or less, such as 15 nm or less, such as 10 nm or less, such as 5 nm or less, such as 2 nm or less and including light sources which emit a specific wavelength of light (i.e., monochromatic light). Where methods include irradiating with a narrow band light source, narrow band light source protocols of interest may include, but are not limited to, a narrow wavelength LED, laser diode or a broadband light source coupled to one or more optical bandpass filters, diffraction gratings, monochromators or any combination thereof.

In certain embodiments, methods include irradiating the sample with one or more lasers. As discussed above, the type and number of lasers will vary depending on the sample as well as desired light collected and may be a gas laser, such as a helium-neon laser, argon laser, krypton laser, xenon laser, nitrogen laser, $CO_2$ laser, CO laser, argon-fluorine (ArF) excimer laser, krypton-fluorine (KrF) excimer laser, xenon chlorine (XeCl) excimer laser or xenon-fluorine (XeF) excimer laser or a combination thereof. In others instances, the methods include irradiating the flow stream with a dye laser, such as a stilbene, coumarin or rhodamine laser. In yet other instances, methods include irradiating the flow stream with a metal-vapor laser, such as a helium-cadmium (HeCd) laser, helium-mercury (HeHg) laser, helium-selenium (HeSe) laser, helium-silver (HeAg) laser, strontium laser, neon-copper (NeCu) laser, copper laser or gold laser and combinations thereof. In still other instances, methods include irradiating the flow stream with a solid-state laser, such as a ruby laser, an Nd:YAG laser, NdCrYAG laser, Er:YAG laser, Nd:YLF laser, $Nd:YVO_4$ laser, $Nd:YCa_4O(BO_3)_3$ laser, Nd:YCOB laser, titanium sapphire laser, thulim YAG laser, ytterbium YAG laser, ytterbium$_2$O$_3$ laser or cerium doped lasers and combinations thereof.

The sample may be irradiated with one or more of the above-mentioned light sources, such as 2 or more light sources, such as 3 or more light sources, such as 4 or more light sources, such as 5 or more light sources and including 10 or more light sources. The light source may include any combination of types of light sources. For example, in some embodiments, the methods include irradiating the sample in the flow stream with an array of lasers, such as an array having one or more gas lasers, one or more dye lasers and one or more solid-state lasers.

The sample may be irradiated with wavelengths ranging from 200 nm to 1500 nm, such as from 250 nm to 1250 nm, such as from 300 nm to 1000 nm, such as from 350 nm to 900 nm and including from 400 nm to 800 nm. For example, where the light source is a broadband light source, the sample may be irradiated with wavelengths from 200 nm to 900 nm. In other instances, where the light source includes a plurality of narrow band light sources, the sample may be irradiated with specific wavelengths in the range from 200 nm to 900 nm. For example, the light source may be plurality of narrow band LEDs (1 nm - 25 nm) each independently emitting light having a range of wavelengths between 200 nm to 900 nm. In other embodiments, the narrow band light source includes one or more lasers (such as a laser array) and the sample is irradiated with specific wavelengths ranging from 200 nm to 700 nm, such as with a laser array having gas lasers, excimer lasers, dye lasers, metal vapor lasers and solid-state laser as described above.

Where more than one light source is employed, the sample may be irradiated with the light sources simultaneously or sequentially, or a combination thereof. For example, the sample may be simultaneously irradiated with each of the light sources. In other embodiments, the flow stream is sequentially irradiated with each of the light sources. Where more than one light source is employed to irradiate the sample sequentially, the time each light source irradiates the sample may independently be 0.001 microseconds or more, such as 0.01 microseconds or more, such as 0.1 microseconds or more, such as 1 microsecond or more, such as 5 microseconds or more, such as 10 microseconds or more, such as 30 microseconds or more and including 60 microseconds or more. For example, methods may include irradiating the sample with the light source (e.g. laser) for a duration which ranges from 0.001 microseconds to 100 microseconds, such as from 0.01 microseconds to 75 microseconds, such as from 0.1 microseconds to 50 microseconds, such as from 1 microsecond to 25 microseconds and including from 5 microseconds to 10 microseconds. In embodiments where sample is sequentially irradiated with two or more light sources, the duration sample is irradiated by each light source may be the same or different.

The time period between irradiation by each light source may also vary, as desired, being separated independently by a delay of 0.001 microseconds or more, such as 0.01 microseconds or more, such as 0.1 microseconds or more, such as 1 microsecond or more, such as 5 microseconds or more, such as by 10 microseconds or more, such as by 15 microseconds or more, such as by 30 microseconds or more and including by 60 microseconds or more. For example, the time period between irradiation by each light source may range from 0.001 microseconds to 60 microseconds, such as from 0.01 microseconds to 50 microseconds, such as from 0.1 microseconds to 35 microseconds, such as from 1 microsecond to 25 microseconds and including from 5 microseconds to 10 microseconds. In certain embodiments, the time period between irradiation by each light source is 10 microseconds. In embodiments where sample is sequentially irradiated by more than two (i.e., 3 or more) light sources, the delay between irradiation by each light source may be the same or different.

The sample may be irradiated continuously or in discrete intervals. In some instances, methods include irradiating the sample in the sample with the light source continuously. In other instances, the sample in is irradiated with the light source in discrete intervals, such as irradiating every 0.001 millisecond, every 0.01 millisecond, every 0.1 millisecond, every 1 millisecond, every 10 milliseconds, every 100 milliseconds and including every 1000 milliseconds, or some other interval.

Depending on the light source, the sample may be irradiated from a distance which varies such as 0.01 mm or more, such as 0.05 mm or more, such as 0.1 mm or more, such as 0.5 mm or more, such as 1 mm or more, such as 2.5 mm or more, such as 5 mm or more, such as 10 mm or more, such as 15 mm or more, such as 25 mm or more and including 50 mm or more. Also, the angle or irradiation may also vary, ranging from 10° to 90°, such as from 15° to 85°, such as from 20° to 80°, such as from 25° to 75° and including from 30° to 60°, for example at a 90° angle.

In certain embodiments, methods include irradiating the sample with two or more beams of frequency shifted light. As described above, a light beam generator component may be employed having a laser and an acousto-optic device for frequency shifting the laser light. In these embodiments, methods include irradiating the acousto-optic device with the laser. Depending on the desired wavelengths of light produced in the output laser beam (e.g., for use in irradiating a sample in a flow stream), the laser may have a specific wavelength that varies from 200 nm to 1500 nm, such as from 250 nm to 1250 nm, such as from 300 nm to 1000 nm, such as from 350 nm to 900 nm and including from 400 nm to 800 nm. The acousto-optic device may be irradiated with one or more lasers, such as 2 or more lasers, such as 3 or more lasers, such as 4 or more lasers, such as 5 or more lasers and including 10 or more lasers. The lasers may include any combination of types of lasers. For example, in some embodiments, the methods include irradiating the acousto-optic device with an array of lasers, such as an array having one or more gas lasers, one or more dye lasers and one or more solid-state lasers.

Where more than one laser is employed, the acousto-optic device may be irradiated with the lasers simultaneously or sequentially, or a combination thereof. For example, the acousto-optic device may be simultaneously irradiated with each of the lasers. In other embodiments, the acousto-optic device is sequentially irradiated with each of the lasers. Where more than one laser is employed to irradiate the acousto-optic device sequentially, the time each laser irradiates the acousto-optic device may independently be 0.001 microseconds or more, such as 0.01 microseconds or more, such as 0.1 microseconds or more, such as 1 microsecond or more, such as 5 microseconds or more, such as 10 microseconds or more, such as 30 microseconds or more and including 60 microseconds or more. For example, methods may include irradiating the acousto-optic device with the laser for a duration which ranges from 0.001 microseconds to 100 microseconds, such as from 0.01 microseconds to 75 microseconds, such as from 0.1 microseconds to 50 microseconds, such as from 1 microsecond to 25 microseconds and including from 5 microseconds to 10 microseconds. In embodiments where the acousto-optic device is sequentially irradiated with two or more lasers, the duration the acousto-optic device is irradiated by each laser may be the same or different.

The time period between irradiation by each laser may also vary, as desired, being separated independently by a delay of 0.001 microseconds or more, such as 0.01 microseconds or more, such as 0.1 microseconds or more, such as 1 microsecond or more, such as 5 microseconds or more, such as by 10 microseconds or more, such as by 15 microseconds or more, such as by 30 microseconds or more and including by 60 microseconds or more. For example, the time period between irradiation by each light source may range from 0.001 microseconds to 60 microseconds, such as from 0.01 microseconds to 50 microseconds, such as from 0.1 microseconds to 35 microseconds, such as from 1 microsecond to 25 microseconds and including from 5 microseconds to 10 microseconds. In certain embodiments, the time period between irradiation by each laser is 10 microseconds. In embodiments where the acousto-optic device is sequentially irradiated by more than two (i.e., 3 or more) lasers, the delay between irradiation by each laser may be the same or different.

The acousto-optic device may be irradiated continuously or in discrete intervals. In some instances, methods include irradiating the acousto-optic device with the laser continuously. In other instances, the acousto-optic device is irradiated with the laser in discrete intervals, such as irradiating every 0.001 millisecond, every 0.01 millisecond, every 0.1 millisecond, every 1 millisecond, every 10 milliseconds, every 100 milliseconds and including every 1000 milliseconds, or some other interval.

Depending on the laser, the acousto-optic device may be irradiated from a distance which varies such as 0.01 mm or more, such as 0.05 mm or more, such as 0.1 mm or more, such as 0.5 mm or more, such as 1 mm or more, such as 2.5 mm or more, such as 5 mm or more, such as 10 mm or more, such as 15 mm or more, such as 25 mm or more and including 50 mm or more. Also, the angle or irradiation may also vary, ranging from 10° to 90°, such as from 15° to 85°, such as from 20° to 80°, such as from 25° to 75° and including from 30° to 60°, for example at a 90° angle.

In embodiments, methods include applying radiofrequency drive signals to the acousto-optic device to generate angularly deflected laser beams. Two or more radiofrequency drive signals may be applied to the acousto-optic device to generate an output laser beam with the desired number of angularly deflected laser beams, such as 3 or more radiofrequency drive signals, such as 4 or more radiofrequency drive signals, such as 5 or more radiofrequency drive signals, such as 6 or more radiofrequency drive signals, such as 7 or more radiofrequency drive signals, such as 8 or more radiofrequency drive signals, such as 9 or more radiofrequency drive signals, such as 10 or more radiofrequency drive signals, such as 15 or more radiofrequency drive signals, such as 25 or more radiofrequency drive signals, such as 50 or more radiofrequency drive signals and including 100 or more radiofrequency drive signals.

The angularly deflected laser beams produced by the radiofrequency drive signals each have an intensity based on the amplitude of the applied radiofrequency drive signal. In some embodiments, methods include applying radiofrequency drive signals having amplitudes sufficient to produce angularly deflected laser beams with a desired intensity. In some instances, each applied radiofrequency drive signal independently has an amplitude from about 0.001 V to about 500 V, such as from about 0.005 V to about 400 V, such as from about 0.01 V to about 300 V, such as from about 0.05 V to about 200 V, such as from about 0.1 V to about 100 V, such as from about 0.5 V to about 75 V, such as from about 1 V to 50 V, such as from about 2 V to 40 V, such as from 3 V to about 30 V and including from about 5 V to about 25 V. Each applied radiofrequency drive signal has, in some embodiments, a frequency of from about 0.001 MHz to about 500 MHz, such as from about 0.005 MHz to about 400 MHz, such as from about 0.01 MHz to about 300 MHz, such as from about 0.05 MHz to about 200 MHz, such as from about 0.1 MHz to about 100 MHz, such as from about 0.5 MHz to about 90 MHz, such as from about 1 MHz to about 75 MHz, such as from about 2 MHz to about 70 MHz, such as from about 3 MHz to about 65 MHz, such as from about 4 MHz to about 60 MHz and including from about 5 MHz to about 50 MHz.

In these embodiments, the angularly deflected laser beams in the output laser beam are spatially separated. Depending on the applied radiofrequency drive signals and desired irradiation profile of the output laser beam, the angularly deflected laser beams may be separated by 0.001 $\mu$m or more, such as by 0.005 $\mu$m or more, such as by 0.01 $\mu$m or more, such as by 0.05 $\mu$m or more, such as by 0.1 $\mu$m or more, such as by 0.5 $\mu$m or more, such as by 1 $\mu$m or more, such as by 5 $\mu$m or more, such as by 10 $\mu$m or more, such as by 100 $\mu$m or more, such as by 500 $\mu$m or more, such as by 1000 $\mu$m or more and including by 5000 $\mu$m or more. In some embodiments, the angularly deflected laser beams overlap, such as with an adjacent angularly deflected laser beam along a horizontal axis of the output laser beam. The overlap between adjacent angularly deflected laser beams (such as overlap of beam spots) may be an overlap of 0.001 $\mu$m or more, such as an overlap of 0.005 $\mu$m or more, such as an overlap of 0.01 $\mu$m or more, such as an overlap of 0.05 $\mu$m or more, such as an overlap of 0.1 $\mu$m or more, such as an overlap of 0.5 $\mu$m or more, such as an overlap of 1 $\mu$m or more, such as an overlap of 5 $\mu$m or more, such as an overlap of 10 $\mu$m or more and including an overlap of 100 $\mu$m or more.

In certain instances, the flow stream is irradiated with a plurality of beams of frequency-shifted light and a cell in the flow stream is imaged by fluorescence imaging using radiofrequency tagged emission (FIRE) to generate a frequency-encoded image, such as those described in Diebold, et al. Nature Photonics Vol. 7(10); 806-810 (2013), as well as described in U.S. Patent Nos. 9,423,353; 9,784,661; 9,983,132; 10,006,852; 10,078,045; 10,036,699; 10,222,316; 10,288,546; 10,324,019; 10,408,758; 10,451,538; 10,620,111; and U.S. Patent Publication Nos. 2017/0133857; 2017/0328826; 2017/0350803; 2018/0275042; 2019/0376895 and 2019/0376894 the disclosures of which are herein incorporated by reference.

As discussed above, in embodiments light from the irradiated sample is conveyed to a light detection system as described in greater detail below and measured by the plurality of photodetectors. In some embodiments, methods include measuring the collected light over a range of wavelengths (e.g., 200 nm - 1000 nm). For example, methods may include collecting spectra of light over one or more of the wavelength ranges of 200 nm - 1000 nm. In yet other embodiments, methods include measuring collected light at one or more specific wavelengths. For example, the collected light may be measured at one or more of 450 nm, 518 nm, 519 nm, 561 nm, 578 nm, 605 nm, 607 nm, 625 nm, 650 nm, 660 nm, 667 nm, 670 nm, 668 nm, 695 nm, 710 nm, 723 nm, 780 nm, 785 nm, 647 nm, 617 nm and any combinations thereof. In certain embodiments, methods including measuring wavelengths of light which correspond to the fluorescence peak wavelength of fluorophores. In some embodiments, methods include measuring collected light across the entire fluorescence spectrum of each fluorophore in the sample.

The collected light may be measured continuously or in discrete intervals. In some instances, methods include taking measurements of the light continuously. In other instances, the light is measured in discrete intervals, such as measuring light every 0.001 millisecond, every 0.01 millisecond, every 0.1 millisecond, every 1 millisecond, every 10 milliseconds, every 100 milliseconds and including every 1000 milliseconds, or some other interval.

Measurements of the collected light may be taken one or more times during the subject methods, such as 2 or more times, such as 3 or more times, such as 5 or more times and including 10 or more times. In certain embodiments, the light propagation is measured 2 or more times, with the data in certain instances being averaged.

Light from the sample may be measured at one or more wavelengths of, such as at 5 or more different wavelengths, such as at 10 or more different wavelengths, such as at 25 or more different wavelengths, such as at 50 or more different wavelengths, such as at 100 or more different wavelengths, such as at 200 or more different wavelengths, such as at 300 or more different wavelengths and including measuring the collected light at 400 or more different wavelengths.

In embodiments, methods include spectrally resolving the light from each fluorophore in the sample using a generalized least squares algorithm. In some embodiments, the overlap between each different fluorophore is determined and the contribution of each fluorophore to the overlapping fluorescence is calculated. In some embodiments, spectrally resolving light with the generalized least squares algorithm includes calculating a spectral unmixing matrix for the fluorescence spectra for each of the plurality of fluorophores having overlapping fluorescence in the sample detected by the light detection system. For example, spectrally resolving light according to the methods described herein may include a Moore-Penrose inverse or pseudoinverse of the spectral matrix. As described in greater detail below, spectrally resolving the light from each fluorophore using the generalized least squares algorithm (e.g., calculating a spectral unmixing matrix for each fluorophore) may be used to estimate the abundance of each fluorophore in the sample. In certain embodiments, the abundance of each fluorophore associated with a target particle may be determined. The abundance of each fluorophore associated with a target particle may be used in identifying and classifying a particle. In some instances, identified or classified particles may be used to sort target particles (e.g., cells) in the sample. In certain embodiments, spectrally resolving fluorophores in the sample, such as by calculating spectral unmixing, is conducted so that sorting is sufficiently fast to sort particles in real time after detection by the light detection system.

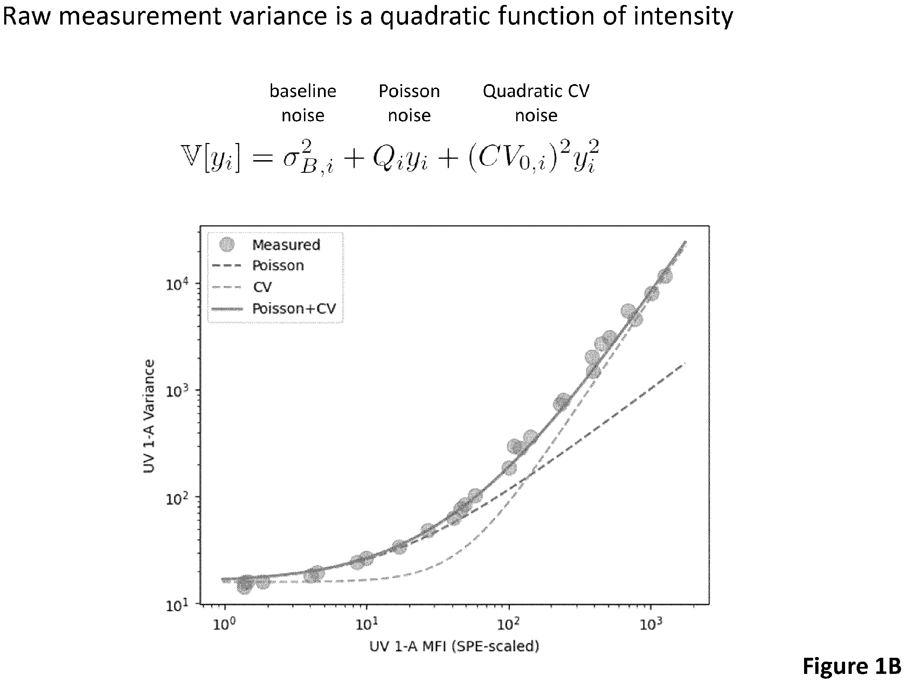

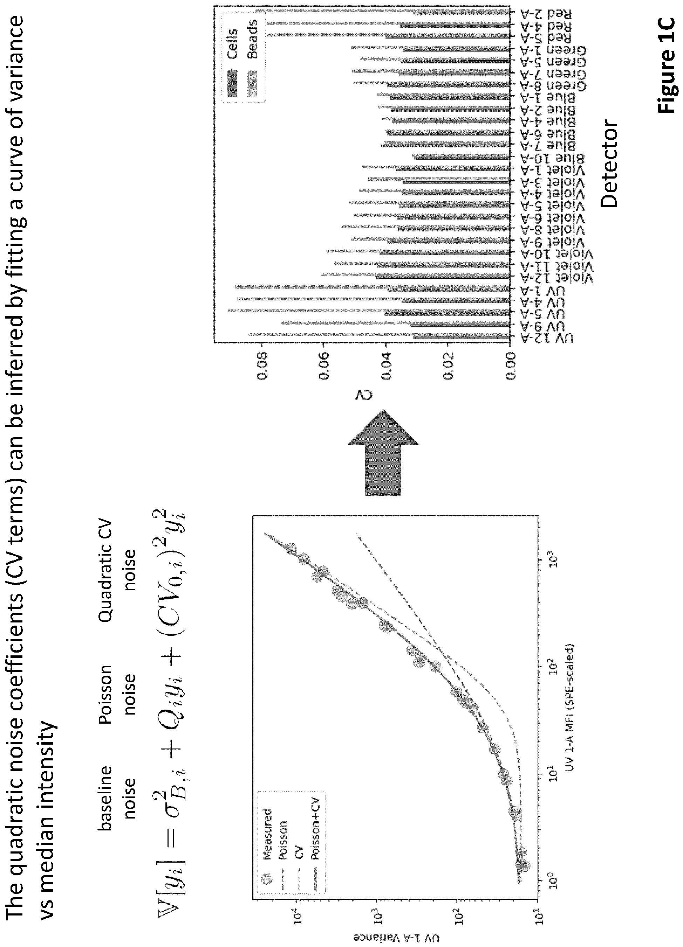

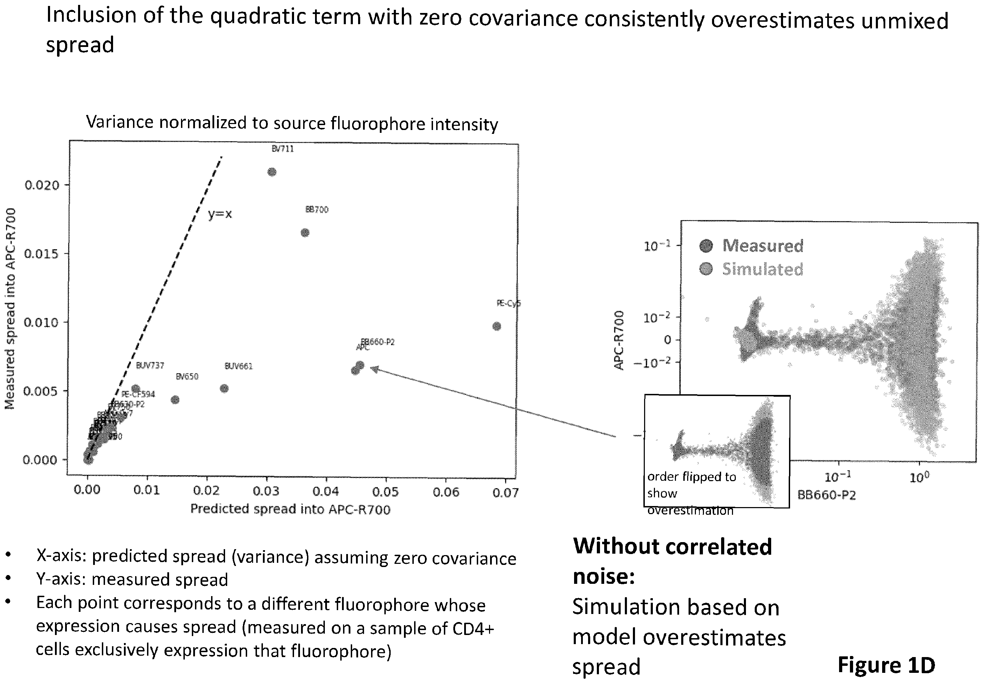

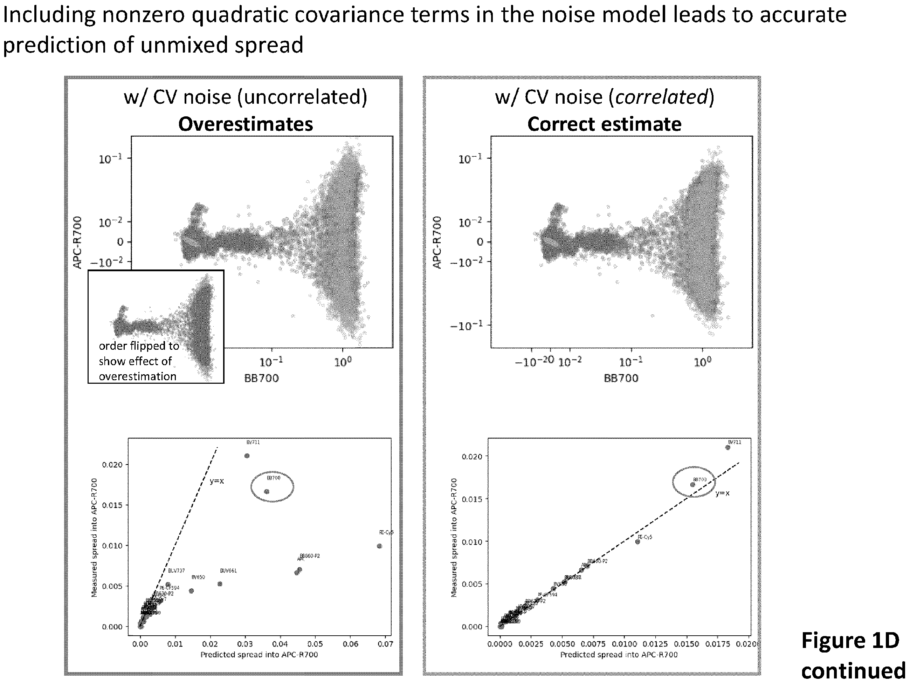

In some embodiments, methods include determining data signal covariance in one or more photodetector channels. In certain instances, data signal covariance is determined in each photodetector channel. The term "data signal covariance" is used herein in its conventional sense to refer to the differential measurement by each photodetector channel. For example, different measurements (from particle to particle, and from detector to detector) in embodiments will have different noise levels, due both to signal-independent constant sources of noise (such as baseline electronic noise from amplifiers and analog-to-digital converters, and baseline shot noise from constant optical sources such as ambient illumination or scattered laser light) and to signal-dependent sources of noise that vary linearly with signal (e.g., shot noise arising from the fact that photon measurements follow Poisson statistics) or quadratically with signal (e.g., multiplicative noise arising from random fluctuations in fluidics and illumination intensity as particles flow through the system). Figure 1B depicts the raw measurement variance from particles in a sample irradiated with light according to certain embodiments. The variance is a function of baseline noise, Poisson noise and quadratic covariance (CV) noise. Figure 1C depicts determining the quadratic noise coefficients in measurement covariance. In some instances, the quadratic noise coefficients are inferred by fitting a curve of variance versus the median intensity of light detected in each photodetector channel. In certain instances, the per-detector quadratic covariance coefficient is inferred from raw measurement data using the baseline noise per detector and the linear Poisson coefficients. Figure 1D depicts the estimation of unmixed spread in the absence of (i.e., zero) covariance. As shown in Figure 1D, inclusion of the quadratic term with zero covariance will overestimate unmixed spread. Measured spread versus a simulation based on models with no covariance demonstrates this overestimate in the spread. On the other hand, including nonzero quadratic covariance terms in the noise model leads to accurate prediction of unmixed spread.

In some embodiments, the subject methods account for non-zero covariance in measurement by different photodetector channels. In some instances, spectrally resolving light with the generalized least squares algorithm is weighted by the inverse of the variance between photodetector channels. In some instances, measurements in different photodetector channels are decorrelated by multiplication by the inverse of a measurement covariance matrix (as described below). In some instances, the data signal covariance in each photodetector channel includes one or more of an intrinsic sample variability component and a measurement variability component. In some instances, the data signal covariance includes electronic noise in each photodetector channel. In some instances, the data signal covariance includes shot noise in each photodetector channel. In some instances, the data signal covariance varies linearly with the generated data signals. In some instances, the data signal covariance varies quadratically with the generated data signals. In some instances, the data signal covariance is correlated across two or more of the plurality of photodetector channels. In some instances, the data signal covariance is calculated according to:

$$V[y_i] = \sigma_{B,i}^2 + Q_i y_i + (CV_{0,i})^2 y_i^2$$

wherein:

$y_i$ is a measured detector signal;

$\sigma_{B,i}^2$ is a baseline noise component;

$Q_i y_i$ is a Poisson noise component;

$Q_i$ is a Poisson noise coefficient;

$(CV_{0,i})^2 y_i^2$ is a quadratic noise component; and

$CV_{0,i}$ is a quadratic noise coefficient.

In some embodiments, the data signal covariance is calculated using a covariance matrix. In some instances, the covariance matrix contains non-zero diagonal values. In certain instances, the covariance is calculated with a covariance matrix according to:

$$V[y] = \Sigma^y = \begin{bmatrix} V[y_1] & cov(y_1, y_2) & \cdots & cov(y_1, y_m) \\ cov(y_2, y_1) & V[y_2] & \cdots & cov(y_2, y_m) \\ \vdots & \vdots & \ddots & \vdots \\ cov(y_m, y_1) & cov(y_m, y_2) & \cdots & V[y_m] \end{bmatrix}$$

$$= diag(\sigma_B^2) + diag(Q)diag(y) + \begin{bmatrix} CV_{0,1}^2 y_1^2 & cov(y_1, y_2) & \cdots & cov(y_1, y_m) \\ cov(y_2, y_1) & CV_{0,2}^2 y_2^2 & \cdots & cov(y_2, y_m) \\ \vdots & \vdots & \ddots & \vdots \\ cov(y_m, y_1) & cov(y_m, y_2) & \cdots & CV_{0,m}^2 y_m^2 \end{bmatrix}$$

wherein *diag(x)* indicates generating a diagonal matrix from the column vector *x,* and *cov(a, b)* denotes the covariance between *a* and *b*.

In some instances, the solution to the generalized least squares problem is calculated by multiplying by the inverse of the calculated covariance matrix. In some instances, methods include estimating data signal covariance based on fluorescence intensity across two or more photodetectors of the light detection system. In some instances, methods include estimating data signal covariance based on fluorescence intensity across each of the photodetection channels. In some embodiments, the generalized least squares problem is calculated according to:

$$\hat{f} = \arg\min_f (Xf - y)^T G(Xf - y)$$

wherein:

$G = (\Sigma^y)^{-1}$ is a weight matrix;

$\Sigma^y$ is a covariance matrix;

$X$ is a spectral matrix (spillover matrix);

$y$ is measured detector values from the plurality of photodetectors of the light detection system for each cell;

$f$ is true fluorophore abundances for each cell; and

$\hat{f}$ is estimated (unmixed) fluorophore abundances for each cell.

In some instances, the method further includes generating *a priori* an estimated covariance matrix. In certain instances, the generalized least squares algorithm is applied in real time (e.g., using an integrated circuit such as a field programmable gate array). In some instances, an *a priori* noise model is used to estimate each event covariance (e.g., calculate each event covariance matrix in real time). In certain instances, the *a priori* noise model uses only data collected for each specific event. In some instances, the covariance matrix includes estimations from an entire collected dataset. In some instances, the covariance matrix is generated through iterative optimization methods which empirically tune the covariance matrix to minimize the variance of the unmixed data.

In some embodiments, data signal covariance is determined by estimating by iterative optimization with a covariance matrix that minimizes variance of unmixed data signals. In certain instances, the finding the solution to the generalized least squares problem is characterized by Cholesky decomposition of the covariance matrix. In certain instances, finding the solution to the generalized least squares problem is characterized by computing the Cholesky decomposition of the covariance matrix $\Sigma^y = CC^T$ and solving triangular systems to generate transformed inputs for an ordinary least squares algorithm according to:

Solving $C\overline{y} = y$ to generate $\overline{y}$, a decorrelated and whitened version of the data vector y.
Solving $C\overline{X} = X$ to generate $\overline{X}$, the corresponding transformed version of the spectral matrix X.
The transformed system $\overline{X}f = \overline{y}$ is solved by ordinary least squares, for example by solving the so-called normal equations $\overline{X}^T\overline{X}f = \overline{X}^T\overline{y}$.

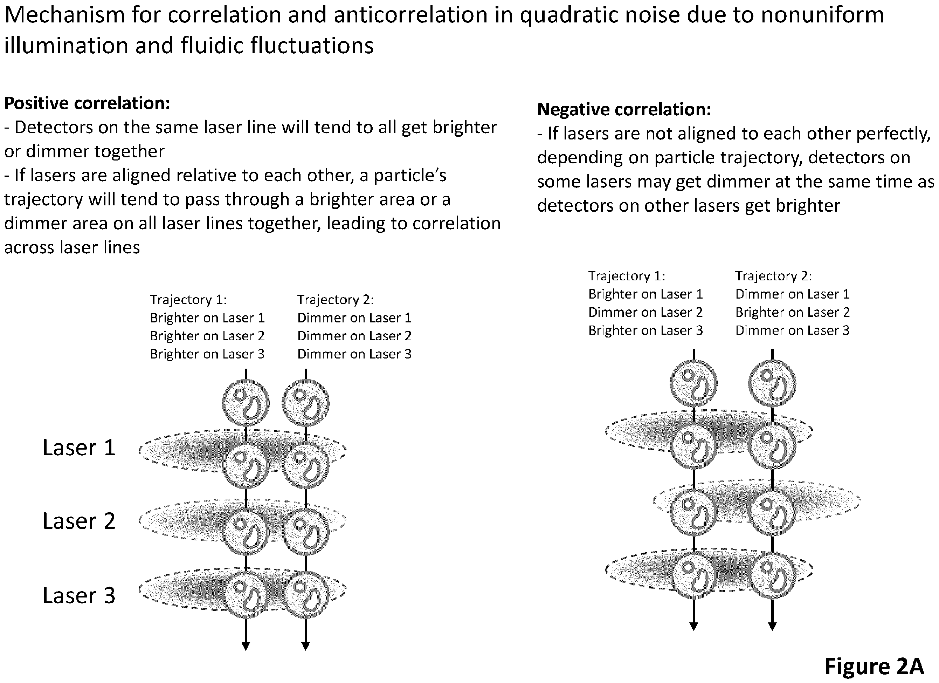

Figure 2A depicts a comparison of correlation and anti-correlation in quadratic noise according to certain embodiments. In some instances, random fluctuations in measured signal arising from system perturbations may be correlated across multiple detectors. A flow stream in a flow cytometer flow cell is depicted in Figure 2A, where illumination laser beams with a gaussian beam intensity profile are aligned so that they are brightest at the center of the core stream and dimmer toward the edges. While an individual particle typically follows a straight trajectory within the core stream (i.e., in the axial direction of the cylindrical core stream), its radial position is essentially random upon entering the core stream. A particle passing through the center of the core stream in a well-aligned flow cytometer will experience a higher degree of illumination by all lasers than an identical particle passing through the core stream closer to the outer edge, which will pass through a lower-intensity region of each beam. This would result in a dimmer recorded signal across all detectors for the second particle compared to the first particle; in other words, although the fluctuation itself is random, the fluctuation goes "in the same direction" for all measurement channels. Imperfections in alignment and differences in collection efficiency at different wavelengths mean that such fluctuations will not be perfectly correlated across detectors. In some instances, these fluctuations are anticorrelated (i.e., certain detectors receive a brighter signal while other detectors receive a dimmer signal). In some instances, the structure of the quadratic noise is correlated (i.e., has a covariance matrix with nonzero diagonal elements).

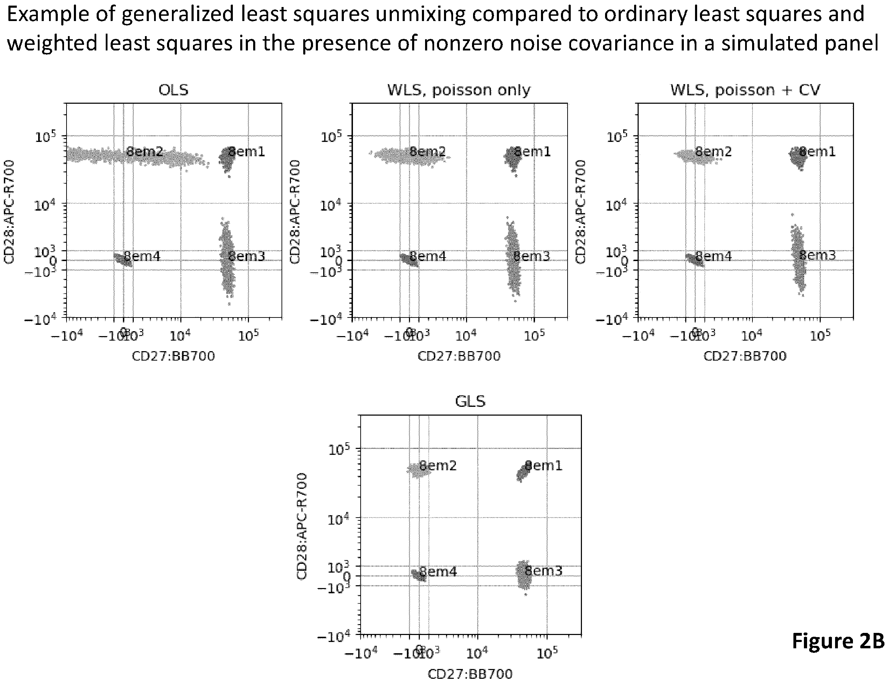

Figure 2B depicts a comparison of spectral unmixing using a generalized least squares algorithm according to certain embodiments with unmixing with ordinary least squares and weighted least squares. As shown in the example in Figure 2B, simulation results show that in the presence of correlated, heteroscedastic measurement noise, generalized least squares can provide a substantial improvement (lower unmixed data variance) over both ordinary least squares and weighted least squares for spectral measurements with a light detection system having a plurality of photodetectors.

In some embodiments, methods include finding the least-squares solution of the generalized least squares problem. In some instances, the least-squares solution of the generalized least squares problem is found by one or more of matrix decomposition, matrix factorization, QR factorization, Cholesky decomposition, singular value decomposition, LDL decomposition, and forward- and back-substitution. In some instances, methods include finding the least-squares solution of the generalized least squares problem by solving the so-called normal equations via Cholesky or LDL decomposition, such as by Cholesky or LDL decomposition according to:

$$X^TGX\hat{f} = X^TGy$$

Where y is measured detector values from the plurality of photodetectors of the light detection system for each cell; X is spectral spillover; and

$$G \text{ is } \begin{bmatrix} G_{1,1} & G_{1,2} & \cdots & G_{1,m} \\ G_{2,1} & G_{2,2} & \cdots & G_{2,m} \\ \vdots & \vdots & \ddots & \vdots \\ G_{m,1} & G_{m,2} & \cdots & G_{m,m} \end{bmatrix}$$

In some instances, methods include finding the least-squares solution of the generalized least squares problem by Cholesky or LDL decomposition according to:

$$X^TGX\hat{f} = X^TGy$$

$A\hat{f} = B$
$LDL^T\hat{f} = B$         LDL decomposition
$Lz = B$ where $z = DL^T\hat{f}$     Lower-triangular matrix solution
$Dx = z$ where $x = L^T\hat{f}$     Diagonal matrix solution
$L^T\hat{f} = x$, solve for $\hat{f}$     Upper-triangular matrix solution

In some embodiments, methods include finding the least-squares solution of the generalized least squares problem by matrix decomposition.

In some embodiments, methods include finding the least-squares solution of the generalized least squares problem by matrix factorization. In some instances, the least-squares solution of the generalized least squares problem is found by QR factorization. In some instances, the generalized least squares problem is calculated using transformed $\overline{X}$ and $\overline{y}$ (as calculated via Cholesky decomposition of the covariance matrix described above) using QR factorization according to:

$\overline{X}^T \overline{X}f = \overline{X}^T\overline{y}$      Normal equations for transformed GLS problem
$QR = \overline{X}$      QR decomposition of transformed $X$
$(QR)^TQR\hat{f} = (QR)^T\overline{y}$      Substitution
$R^TQ^TQR\hat{f} = R^TQ^T\overline{y}$      Expand transpose of $QR$
$R^TR\hat{f} = R^TQ^T\overline{y}Q^TQ$      cancels due to orthogonality
$R\hat{f} = Q^T\overline{y}$.      Triangular solve for $\hat{f}$

In some embodiments, methods include finding the least-squares solution of the generalized least squares problem by singular value decomposition. In some instances, the singular value decomposition is the matrix that is the product $\overline{X} = U\Sigma V^T$ where $U$ and $V$ are orthogonal matrices and $\Sigma$ is a diagonal matrix containing singular values of $\overline{X}$. In some instances, the generalized least squares problem is calculated using singular value decomposition according to:

$$z = U^T\overline{y}$$

$$\Sigma w = z$$

$$\hat{f} = Vw,$$

where $U$ and $V$ are orthogonal matrices and $\Sigma$ is a diagonal matrix containing singular values of $\overline{X}$. In some embodiments, methods include finding the least-squares solution of the generalized least squares problem by LDL decomposition. In certain embodiments, methods include minimizing the least-squares solution of the generalized least squares problem by forward- and back-substitution.

In some embodiments, methods include estimating covariance arising from measurement variability alone, without the effect of intrinsic sample variability. In certain instances, an analytical covariance model is used that predicts the measurement covariance of one or more particles (e.g., each particle in the sample) as a function of that particle's intensity profile across all photodetectors. In some instances, the measurement covariance includes one or more parameters such as: (1) baseline noise per photodetector; (2) linear Poisson coefficients (photoelectron scaling factors) per photodetector; (3) per photodetector quadratic CV coefficients (which can be in certain instances estimated from raw data directly); and (4) quadratic CV correlation coefficients describing the extent to which quadratic noise is correlated between each pair of photodetectors. In some instances, baseline noise per photodetector and the linear Poisson coefficients are determined during instrument calibration. In some instances, the per photodetector quadratic CV coefficients for each detector can be estimated from measured raw data. In some instances, the quadratic CV correlation is determined through a model-fitting process using specific classes of measured calibration data such as cells or particles stained with individual fluorochromes and subsequently spectrally unmixed.

In certain embodiments, intrinsic variability of a single spectral source of signal (e.g., a single fluorophore) in an otherwise homogeneous population of particles is correlated across all photodetectors receiving signal from that fluorophore. In some instances, this can lead to high covariance in raw data space. In certain instances, when the sample is spectrally unmixed, all of the intrinsic variation of fluorophore expression is contained in the variance of the unmixed signal for each fluorophore. In certain instances, any other dimensions (fluorophores) present in the unmixing matrix will have zero variance arising from the single source fluorophore.

In some instances, where measurement noise has nonzero covariance and is at least partially uncorrelated, covarying noise sources contribute to variance in the unmixed dimensions not corresponding to the expressed fluorophore. In some instances, methods include measuring the unmixed variance in non-expressed fluorophore channels in measured data to determine ground truth unmixed variance. In some instances, methods include applying the noise model which includes constant, linear, and quadratic coefficients with predetermined estimates for the quadratic noise correlation coefficients and adjusting the correlation coefficients (e.g., through a range of values) until the unmixed variance estimated by the model for the ground truth samples' median intensity levels matches the measured unmixed variance. In some instances, the noise model is then defined and used for event-by-event calculation of the generalized least squares problem.

In some embodiments, methods include calculating the abundance of one or more fluorophores in the sample from the spectrally resolved light from each fluorophore. In certain instances, the abundance of fluorophores associated with (e.g., chemically associated (i.e., covalently, ionically) or physically associated) a target particle is calculated from the spectrally resolved light from each fluorophore associated with the particle. For instance, in one example the relative abundance of each fluorophore associated with a target particle is calculated from the spectrally resolved light from each fluorophore. In another example, the absolute abundance of each fluorophore associated with the target particle is calculated from the spectrally resolved light from each fluorophore. In certain embodiments, a particle may be identified or classified based on the relative abundance of each fluorophore determined to be associated with the particle. In these embodiments, the particle may be identified or classified by any convenient protocol such as by: comparing the relative or absolute abundance of each fluorophore associated with a particle with a control sample having particles of known identity; or by conducting spectroscopic or other assay analysis of a population of particles (e.g., cells) having the calculated relative or absolute abundance of associated fluorophores.

In certain embodiments, methods include sorting one or more of the particles (e.g., cells) of the sample that are identified based on the estimated abundance of the fluorophores associated with the particle. The term "sorting" is used herein in its conventional sense to refer to separating components (e.g., droplets containing cells, droplets containing non-cellular particles such as biological macromolecules) of a sample and in some instances, delivering the separated components to one or more sample collection containers. For example, methods may include sorting 2 or more components of the sample, such as 3 or more components, such as 4 or more components, such as 5 or more components, such as 10 or more components, such as 15 or more components and including sorting 25 or more components of the sample.

In sorting particles identified based on the abundance of fluorophores associated with the particle, methods include data acquisition, analysis and recording, such as with a computer, where multiple data channels record data from each detector used in obtaining the overlapping spectra of the plurality of fluorophores associated with the particle. In these embodiments, analysis includes spectrally resolving light (e.g., by calculating the spectral unmixing matrix) from the plurality of fluorophores having overlapping spectra that are associated with the particle and identifying the particle based on the estimated abundance of each fluorophore associated with the particle. This analysis may be conveyed to a sorting system which is configured to generate a set of digitized parameters based on the particle classification.

In some embodiments, methods for sorting components of sample include sorting particles (e.g., cells in a biological sample) with a particle sorting module having deflector plates, such as described in U.S. Patent Publication No. 2017/0299493, filed on March 28, 2017, the disclosure of which is incorporated herein by reference. In certain embodiments, cells of the sample are sorted using a sort decision module having a plurality of sort decision units, such as those described in U.S. Provisional Patent Application No. 62/803,264, filed on February 8, 2019, the disclosure of which is incorporated herein by reference.

## SYSTEMS FOR SPECTRALLY RESOLVING LIGHT WITH A GENERALIZED LEAST SQUARES ALGORITHM FROM FLUOROPHORES HAVING OVERLAPPING FLUORESCENCE SPECTRA IN A SAMPLE

As summarized above, aspects of the present disclosure include a system for spectrally resolving light from fluorophores having overlapping fluorescence spectra in a sample. As described above, the term "spectrally resolving" is used herein in its conventional sense to refer to spectrally distinguishing each fluorophore in the sample by assigning or attributing the overlapping wavelengths of light to each contributing fluorophore. In embodiments, the overlapping spectral component of fluorescence that is attributed to each fluorophore is determined by calculating a spectral unmixing matrix. In embodiments, the subject systems are used to characterize samples having a plurality of fluorophores where the fluorescence spectra of each fluorophore overlaps with the fluorescence spectra of at least one other fluorophore in the sample. In some instances, the fluorescence spectra of each fluorophore overlaps with the fluorescence spectra of at least one other fluorophore in the sample by 5 nm or more, such as by 10 nm or more, such as by 25 nm or more and including by 50 nm or more. In certain instances, the fluorescence spectra of one or more fluorophores in the sample overlaps with the fluorescence spectra of two or more different fluorophores in the sample, such as where each overlap in fluorescence spectra is by 5 nm or more, such as by 10 nm or more, such as by 25 nm or more and including by 50 nm or more.

In embodiments, systems include a light source configured to irradiate a sample having a plurality of fluorophores where the fluorescence spectra of each fluorophore overlaps with the fluorescence spectra of at least one other fluorophore in the sample. In embodiments, the light source may be any suitable broadband or narrow band source of light. Depending on the components in the sample (e.g., cells, beads, non-cellular particles, etc.), the light source may be configured to emit wavelengths of light that vary, ranging from 200 nm to 1500 nm, such as from 250 nm to 1250 nm, such as from 300 nm to 1000 nm, such as from 350 nm to 900 nm and including from 400 nm to 800 nm. For example, the light source may include a broadband light source emitting light having wavelengths from 200 nm to 900 nm. In other instances, the light source includes a narrow band light source emitting a wavelength ranging from 200 nm to 900 nm. For example, the light source may be a narrow band LED (1 nm - 25 nm) emitting light having a wavelength ranging between 200 nm to 900 nm.

In certain embodiments, the light source is a laser. In some instances, the subject systems include a gas laser, such as a helium-neon laser, argon laser, krypton laser, xenon laser, nitrogen laser, $CO_2$ laser, CO laser, argon-fluorine (ArF) excimer laser, krypton-fluorine (KrF) excimer laser, xenon chlorine (XeCl) excimer laser or xenon-fluorine (XeF) excimer laser or a combination thereof. In others instances, the subject systems include a dye laser, such as a stilbene, coumarin or rhodamine laser. In yet other instances, lasers of interest include a metal-vapor laser, such as a helium-cadmium (HeCd) laser, helium-mercury (HeHg) laser, helium-selenium (HeSe) laser, helium-silver (HeAg) laser, strontium laser, neon-copper (NeCu) laser, copper laser or gold laser and combinations thereof. In still other instances, the subject systems include a solid-state laser, such as a ruby laser, an Nd:YAG laser, NdCrYAG laser, Er:YAG laser, Nd:YLF laser, Nd:YVO$_4$ laser, Nd:YCa$_4$O(BO$_3$)$_3$ laser, Nd:YCOB laser, titanium sapphire laser, thulim YAG laser, ytterbium YAG laser, ytterbium$_2$O$_3$ laser or cerium doped lasers and combinations thereof.

In other embodiments, the light source is a non-laser light source, such as a lamp, including but not limited to a halogen lamp, deuterium arc lamp, xenon arc lamp, a light-emitting diode, such as a broadband LED with continuous spectrum, superluminescent emitting diode, semiconductor light emitting diode, wide spectrum LED white light source, an multi-LED integrated. In some instances, the non-laser light source is a stabilized fiber-coupled broadband light source, white light source, among other light sources or any combination thereof.

The light source may be positioned any suitable distance from the sample (e.g., the flow stream in a flow cytometer), such as at a distance of 0.001 mm or more from the flow stream, such as 0.005 mm or more, such as 0.01 mm or more, such as 0.05 mm or more, such as 0.1 mm or more, such as 0.5 mm or more, such as 1 mm or more, such as 5 mm or more, such as 10 mm or more, such as 25 mm or more and including at a distance of 100 mm or more. In addition, the light source irradiate the sample at any suitable angle (e.g., relative the vertical axis of the flow stream), such as at an angle ranging from 10° to 90°, such as from 15° to 85°, such as from 20° to 80°, such as from 25° to 75° and including from 30° to 60°, for example at a 90° angle.

The light source may be configured to irradiate the sample continuously or in discrete intervals. In some instances, systems include a light source that is configured to irradiate the sample continuously, such as with a continuous wave laser that continuously irradiates the flow stream at the interrogation point in a flow cytometer. In other instances, systems of interest include a light source that is configured to irradiate the sample at discrete intervals, such as every 0.001 milliseconds, every 0.01 milliseconds, every 0.1 milliseconds, every 1 millisecond, every 10 milliseconds, every 100 milliseconds and including every 1000 milliseconds, or some other interval. Where the light source is configured to irradiate the sample at discrete intervals, systems may include one or more additional components to provide for intermittent irradiation of the sample with the light source. For example, the subject systems in these embodiments may include one or more laser beam choppers, manually or computer controlled beam stops for blocking and exposing the sample to the light source.

In some embodiments, the light source is a laser. Lasers of interest may include pulsed lasers or continuous wave lasers. For example, the laser may be a gas laser, such as a helium-neon laser, argon laser, krypton laser, xenon laser, nitrogen laser, $CO_2$ laser, CO laser, argon-fluorine (ArF) excimer laser, krypton-fluorine (KrF) excimer laser, xenon chlorine (XeCl) excimer laser or xenon-fluorine (XeF) excimer laser or a combination thereof; a dye laser, such as a stilbene, coumarin or rhodamine laser; a metal-vapor laser, such as a helium-cadmium (HeCd) laser, helium-mercury (HeHg) laser, helium-selenium (HeSe) laser, helium-silver (HeAg) laser, strontium laser, neon-copper (NeCu) laser, copper laser or gold laser and combinations thereof; a solid-state laser, such as a ruby laser, an Nd:YAG laser, NdCrYAG laser, Er:YAG laser, Nd:YLF laser, Nd:YVO$_4$ laser, Nd:YCa$_4$O(BO$_3$)$_3$ laser, Nd:YCOB laser, titanium sapphire laser, thulim YAG laser, ytterbium YAG laser, ytterbium$_2$O$_3$ laser or cerium doped lasers and combinations thereof; a semiconductor diode laser, optically pumped semiconductor laser (OPSL), or a frequency doubled- or frequency tripled implementation of any of the above mentioned lasers.

In certain embodiments, the light source is a light beam generator that is configured to generate two or more beams of frequency shifted light. In some instances, the light beam generator includes a laser, a radiofrequency generator configured to apply radiofrequency drive signals to an acousto-optic device to generate two or more angularly deflected laser beams. In these embodiments, the laser may be a pulsed lasers or continuous wave laser. For example lasers in light beam generators of interest may be a gas laser, such as a helium-neon laser, argon laser, krypton laser, xenon laser, nitrogen laser, CO2 laser, CO laser, argon-fluorine (ArF) excimer laser, krypton-fluorine (KrF) excimer laser, xenon chlorine (XeCI) excimer laser or xenon-fluorine (XeF) excimer laser or a combination thereof; a dye laser, such as a stilbene, coumarin or rhodamine laser; a metal-vapor laser, such as a helium-cadmium (HeCd) laser, helium-mercury (HeHg) laser, helium-selenium (HeSe) laser, helium-silver (HeAg) laser, strontium laser, neon-copper (NeCu) laser, copper laser or gold laser and combinations thereof; a solid-state laser, such as a ruby laser, an Nd:YAG laser, NdCrYAG laser, Er:YAG laser, Nd:YLF laser, Nd:YVO4 laser, Nd:YCa$_4$O(BO$_3$)$_3$ laser, Nd:YCOB laser, titanium sapphire laser, thulim YAG laser, ytterbium YAG laser, ytterbium$_2$O$_3$ laser or cerium doped lasers and combinations thereof.

The acousto-optic device may be any convenient acousto-optic protocol configured to frequency shift laser light using applied acoustic waves. In certain embodiments, the acousto-optic device is an acousto-optic deflector. The acousto-optic device in the subject system is configured to generate angularly deflected laser beams from the light from the laser and the applied radiofrequency drive signals. The radiofrequency drive signals may be applied to the acousto-optic device with any suitable radiofrequency drive signal source, such as a direct digital synthesizer (DDS), arbitrary waveform generator (AWG), or electrical pulse generator.

In embodiments, a controller is configured to apply radiofrequency drive signals to the acousto-optic device to produce the desired number of angularly deflected laser beams in the output laser beam, such as being configured to apply 3 or more radiofrequency drive signals, such as 4 or more radiofrequency drive signals, such as 5 or more radiofrequency drive signals, such as 6 or more radiofrequency drive signals, such as 7 or more radiofrequency drive signals, such as 8 or more radiofrequency drive signals, such as 9 or more radiofrequency drive signals, such as 10 or more radiofrequency drive signals, such as 15 or more radiofrequency drive signals, such as 25 or more radiofrequency drive signals, such as 50 or more radiofrequency drive signals and including being configured to apply 100 or more radiofrequency drive signals.

In some instances, to produce an intensity profile of the angularly deflected laser beams in the output laser beam, the controller is configured to apply radiofrequency drive signals having an amplitude that varies such as from about 0.001 V to about 500 V, such as from about 0.005 V to about 400 V, such as from about 0.01 V to about 300 V, such as from about 0.05 V to about 200 V, such as from about 0.1 V to about 100 V, such as from about 0.5 V to about 75 V, such as from about 1 V to 50 V, such as from about 2 V to 40 V, such as from 3 V to about 30 V and including from about 5 V to about 25 V. Each applied radiofrequency drive signal has, in some embodiments, a frequency of from about 0.001 MHz to about 500 MHz, such as from about 0.005 MHz to about 400 MHz, such as from about 0.01 MHz to about 300 MHz, such as from about 0.05 MHz to about 200 MHz, such as from about 0.1 MHz to about 100 MHz, such as from about 0.5 MHz to about 90 MHz, such as from about 1 MHz to about 75 MHz, such as from about 2 MHz to about 70 MHz, such as from about 3 MHz to about 65 MHz, such as from about 4 MHz to about 60 MHz and including from about 5 MHz to about 50 MHz.

In certain embodiments, the controller has a processor having memory operably coupled to the processor such that the memory includes instructions stored thereon, which when executed by the processor, cause the processor to produce an output laser beam with angularly deflected laser beams having a desired intensity profile. For example, the memory may include instructions to produce two or more angularly deflected laser beams with the same intensities, such as 3 or more, such as 4 or more, such as 5 or more, such as 10 or more, such as 25 or more, such as 50 or more and including memory may include instructions to produce 100 or more angularly deflected laser beams with the same intensities. In other embodiments, the may include instructions to produce two or more angularly deflected laser beams with different intensities, such as 3 or more, such as 4 or more, such as 5 or more, such as 10 or more, such as 25 or more, such as 50 or more and including memory may include instructions to produce 100 or more angularly deflected laser beams with different intensities.

In certain embodiments, the controller has a processor having memory operably coupled to the processor such that the memory includes instructions stored thereon, which when executed by the processor, cause the processor to produce an output laser beam having increasing intensity from the edges to the center of the output laser beam along the horizontal axis. In these instances, the intensity of the angularly deflected laser beam at the center of the output beam may range from 0.1% to about 99% of the intensity of the angularly deflected laser beams at the edge of the output laser beam along the horizontal axis, such as from 0.5% to about 95%, such as from 1% to about 90%, such as from about 2% to about 85%, such as from about 3% to about 80%, such as from about 4% to about 75%, such as from about 5% to about 70%, such as from about 6% to about 65%, such as from about 7% to about 60%, such as from about 8% to about 55% and including from about 10% to about 50% of the intensity of the angularly deflected laser beams at the edge of the output laser beam along the horizontal axis. In other embodiments, the controller has a processor having memory operably coupled to the processor such that the memory includes instructions stored thereon, which when executed by the processor, cause the processor to produce an output laser beam having an increasing intensity from the edges to the center of the output laser beam along the horizontal axis. In these instances, the intensity of the angularly deflected laser beam at the edges of the output beam may range from 0.1% to about 99% of the intensity of the angularly deflected laser beams at the center of the output laser beam along the horizontal axis, such as from 0.5% to about 95%, such as from 1% to about 90%, such as from about 2% to about 85%, such as from about 3% to about 80%, such as from about 4% to about 75%, such as from about 5% to about 70%, such as from about 6% to about 65%, such as from about 7% to about 60%, such as from about 8% to about 55% and including from about 10% to about 50% of the intensity of the angularly deflected laser beams at the center of the output laser beam along the horizontal axis. In yet other embodiments, the controller has a processor having memory operably coupled to the processor such that the memory includes instructions stored thereon, which when executed by the processor, cause the processor to produce an output laser beam having an intensity profile with a Gaussian distribution along the horizontal axis. In still other embodiments, the controller has a processor having memory operably coupled to the processor such that the memory includes instructions stored thereon, which when executed by the processor, cause the processor to produce an output laser beam having a top hat intensity profile along the horizontal axis.

In embodiments, light beam generators of interest may be configured to produce angularly deflected laser beams in the output laser beam that are spatially separated. Depending on the applied radiofrequency drive signals and desired irradiation profile of the output laser beam, the angularly deflected laser beams may be separated by 0.001 $\mu$m or more, such as by 0.005 $\mu$m or more, such as by 0.01 $\mu$m or more, such as by 0.05 $\mu$m or more, such as by 0.1 $\mu$m or more, such as by 0.5 $\mu$m or more, such as by 1 $\mu$m or more, such as by 5 $\mu$m or more, such as by 10 $\mu$m or more, such as by 100 $\mu$m or more, such as by 500 $\mu$m or more, such as by 1000 $\mu$m or more and including by 5000 $\mu$m or more. In some embodiments, systems are configured to produce angularly deflected laser beams in the output laser beam that overlap, such as with an adjacent angularly deflected laser beam along a horizontal axis of the output laser beam. The overlap between adjacent angularly deflected laser beams (such as overlap of beam spots) may be an overlap of 0.001 $\mu$m or more, such as an overlap of 0.005 $\mu$m or more, such as an overlap of 0.01 $\mu$m or more, such as an overlap of 0.05 $\mu$m or more, such as an overlap of 0.1 $\mu$m or more, such as an overlap of 0.5 $\mu$m or more, such as an overlap of 1 $\mu$m or more, such as an overlap of 5 $\mu$m or more, such as an overlap of 10 $\mu$m or more and including an overlap of 100 $\mu$m or more.

In certain instances, light beam generators configured to generate two or more beams of frequency shifted light include laser excitation modules as described in U.S. Patent Nos. 9,423,353; 9,784,661; 9,983,132; 10,006,852; 10,078,045; 10,036,699; 10,222,316; 10,288,546; 10,324,019; 10,408,758; 10,451,538; 10,620,111; and U.S. Patent Publication Nos. 2017/0133857; 2017/0328826; 2017/0350803; 2018/0275042; 2019/0376895 and 2019/0376894 the disclosures of which are herein incorporated by reference.

In embodiments, systems include a light detection system having a plurality of photodetectors. Photodetectors of interest may include, but are not limited to optical sensors, such as active-pixel sensors (APSs), avalanche photodiodes, image sensors, charge-coupled devices (CCDs), intensified charge-coupled devices (ICCDs), light emitting diodes, photon counters, bolometers, pyroelectric detectors, photoresistors, photovoltaic cells, photodiodes, photomultiplier tubes, phototransistors, quantum dot photoconductors or photodiodes and combinations thereof, among other photodetectors. In certain embodiments, light from a sample is measured with a charge-coupled device (CCD), semiconductor charge-coupled devices (CCD), active pixel sensors (APS), complementary metal-oxide semiconductor (CMOS) image sensors or N-type metal-oxide semiconductor (NMOS) image sensors.

In some embodiments, light detection systems of interest include a plurality of photodetectors. In some instances, the light detection system includes a plurality of solid-state detectors such as photodiodes. In certain instances, the light detection system includes a photodetector array, such as an array of photodiodes. In these embodiments, the photodetector array may include 4 or more photodetectors, such as 10 or more photodetectors, such as 25 or more photodetectors, such as 50 or more photodetectors, such as 100 or more photodetectors, such as 250 or more photodetectors, such as 500 or more photodetectors, such as 750 or more photodetectors and including 1000 or more photodetectors. For example, the detector may be a photodiode array having 4 or more photodiodes, such as 10 or more photodiodes, such as 25 or more photodiodes, such as 50 or more photodiodes, such as 100 or more photodiodes, such as 250 or more photodiodes, such as 500 or more photodiodes, such as 750 or more photodiodes and including 1000 or more photodiodes.

The photodetectors may be arranged in any geometric configuration as desired, where arrangements of interest include, but are not limited to a square configuration, rectangular configuration, trapezoidal configuration, triangular configuration, hexagonal configuration, heptagonal configuration, octagonal configuration, nonagonal configuration, decagonal configuration, dodecagonal configuration, circular configuration, oval configuration as well as irregular patterned configurations. The photodetectors in the photodetector array may be oriented with respect to the other (as referenced in an X-Z plane) at an angle ranging from 10° to 180°, such as from 15° to 170°, such as from 20° to 160°, such as from 25° to 150°, such as from 30° to 120° and including from 45° to 90°. The photodetector array may be any suitable shape and may be a rectilinear shape, e.g., squares, rectangles, trapezoids, triangles, hexagons, etc., curvilinear shapes, e.g., circles, ovals, as well as irregular shapes, e.g., a parabolic bottom portion coupled to a planar top portion. In certain embodiments, the photodetector array has a rectangular-shaped active surface.

Each photodetector (e.g., photodiode) in the array may have an active surface with a width that ranges from 5 $\mu$m to 250 $\mu$m, such as from 10 $\mu$m to 225 $\mu$m, such as from 15 $\mu$m to 200 $\mu$m, such as from 20 $\mu$m to 175 $\mu$m, such as from 25 $\mu$m to 150 $\mu$m, such as from 30 $\mu$m to 125 $\mu$m and including from 50 $\mu$m to 100 $\mu$m and a length that ranges from 5 $\mu$m to

250 $\mu$m, such as from 10 $\mu$m to 225 $\mu$m, such as from 15 $\mu$m to 200 $\mu$m, such as from 20 $\mu$m to 175 $\mu$m, such as from 25 $\mu$m to 150 $\mu$m, such as from 30 $\mu$m to 125 $\mu$m and including from 50 $\mu$m to 100 $\mu$m, where the surface area of each photodetector (e.g., photodiode) in the array ranges from 25 to $\mu m^2$ to 10000 $\mu m^2$, such as from 50 to $\mu m^2$ to 9000 $\mu m^2$, such as from 75 to $\mu m^2$ to 8000 $\mu m^2$, such as from 100 to $\mu m^2$ to 7000 $\mu m^2$, such as from 150 to $\mu m^2$ to 6000 $\mu m^2$ and including from 200 to $\mu m^2$ to 5000 $\mu m^2$.

The size of the photodetector array may vary depending on the amount and intensity of the light, the number of photodetectors and the desired sensitivity and may have a length that ranges from 0.01 mm to 100 mm, such as from 0.05 mm to 90 mm, such as from 0.1 mm to 80 mm, such as from 0.5 mm to 70 mm, such as from 1 mm to 60 mm, such as from 2 mm to 50 mm, such as from 3 mm to 40 mm, such as from 4 mm to 30 mm and including from 5 mm to 25 mm. The width of the photodetector array may also vary, ranging from 0.01 mm to 100 mm, such as from 0.05 mm to 90 mm, such as from 0.1 mm to 80 mm, such as from 0.5 mm to 70 mm, such as from 1 mm to 60 mm, such as from 2 mm to 50 mm, such as from 3 mm to 40 mm, such as from 4 mm to 30 mm and including from 5 mm to 25 mm. As such, the active surface of the photodetector array may range from 0.1 $mm^2$ to 10000 $mm^2$, such as from 0.5 $mm^2$ to 5000 $mm^2$, such as from 1 $mm^2$ to 1000 $mm^2$, such as from 5 $mm^2$ to 500 $mm^2$, and including from 10 $mm^2$ to 100 $mm^2$.

Photodetectors of interest are configured to measure collected light at one or more wavelengths, such as at 2 or more wavelengths, such as at 5 or more different wavelengths, such as at 10 or more different wavelengths, such as at 25 or more different wavelengths, such as at 50 or more different wavelengths, such as at 100 or more different wavelengths, such as at 200 or more different wavelengths, such as at 300 or more different wavelengths and including measuring light emitted by a sample in the flow stream at 400 or more different wavelengths.

In some embodiments, photodetectors are configured to measure collected light over a range of wavelengths (e.g., 200 nm - 1000 nm). In certain embodiments, photodetectors of interest are configured to collect spectra of light over a range of wavelengths. For example, systems may include one or more detectors configured to collect spectra of light over one or more of the wavelength ranges of 200 nm - 1000 nm. In yet other embodiments, detectors of interest are configured to measure light from the sample in the flow stream at one or more specific wavelengths. For example, systems may include one or more detectors configured to measure light at one or more of 450 nm, 518 nm, 519 nm, 561 nm, 578 nm, 605 nm, 607 nm, 625 nm, 650 nm, 660 nm, 667 nm, 670 nm, 668 nm, 695 nm, 710 nm, 723 nm, 780 nm, 785 nm, 647 nm, 617 nm and any combinations thereof. In certain embodiments, photodetectors may be configured to be paired with specific fluorophores, such as those used with the sample in a fluorescence assay. In some embodiments, photodetectors are configured to measure collected light across the entire fluorescence spectrum of each fluorophore in the sample.

The light detection system is configured to measure light continuously or in discrete intervals. In some instances, photodetectors of interest are configured to take measurements of the collected light continuously. In other instances, the light detection system is configured to take measurements in discrete intervals, such as measuring light every 0.001 millisecond, every 0.01 millisecond, every 0.1 millisecond, every 1 millisecond, every 10 milliseconds, every 100 milliseconds and including every 1000 milliseconds, or some other interval.

In embodiments, systems are configured to analyze light from the irradiated sample and spectrally resolve light from each fluorophore in the sample using a generalized least squares algorithm. In some embodiments, systems include memory having instructions stored thereon for determining the overlap between each different fluorophore in the sample and calculating the contribution of each fluorophore to the overlapping fluorescence. In certain embodiments, systems are configured to calculate a spectral unmixing matrix for the fluorescence spectra of the plurality of fluorophores having overlapping fluorescence in the sample detected by the light detection system. As described in greater detail below, systems may also be configured to estimate the abundance of each fluorophore in the sample . In certain embodiments, the abundance of each fluorophore associated with a target particle may be determined. The system may be configured to identify and classify a target particle based on the abundance of each fluorophore associated with the target particle. In some instances, systems are configured to sort the identified or classified particles. In these embodiments, systems may include computer controlled systems where the systems further include one or more computers for complete automation or partial automation of a system for practicing methods described herein.

In some embodiments, systems include a computer having a computer readable storage medium with a computer program stored thereon, where the computer program when loaded on the computer includes instructions for irradiating a flow cell having a sample in a flow stream with a light source and detecting light from the flow cell with a light detection system having a plurality of photodetectors, spectrally resolving the light from each fluorophore in the sample using a generalized least squares algorithm, estimating the abundance of each fluorophore; and sorting particles in the sample based on the estimated fluorophore abundance.

In some embodiments, the memory includes instructions stored thereon, which when executed by the processor, cause the processor to determine data signal covariance in each photodetector channel. In some instances, the data signal covariance in each photodetector channel includes an intrinsic sample variability component and a measurement variability component. In some instances, the data signal covariance includes electronic noise in each photodetector channel. In some instances, the data signal covariance includes shot noise in each photodetector channel. In some instances, the data signal covariance varies linearly with the generated data signals. In some instances, the data signal covariance varies quadratically with the generated data signals. In some instances, the data signal covariance is correlated across two or more of the plurality of photodetector channels. In some instances, the memory includes instructions to calculate the data signal covariance according to:

$$V[y_i] = \sigma_{B,i}^2 + Q_i y_i + (CV_{0,i})^2 y_i^2$$

where:

$y_i$ is a measured detector signal;

$\sigma_{B,i}^2$ is a baseline noise component;

$Q_i y_i$ is a Poisson noise component;

$Q_i$ is a Poisson noise coefficient;

$(CV_{0,i})^2 y_i^2$ is a quadratic noise component; and

$CV_{0,i}$ is a quadratic noise coefficient.

In some instances, the memory includes instructions to calculate the data signal covariance using a covariance matrix. In some instances, the covariance matrix contains non-zero diagonal values. In some instances, the memory includes instructions to calculate the covariance with a covariance matrix according to:

$$V[y] = \Sigma^y = \begin{bmatrix} V[y_1] & cov(y_1, y_2) & \cdots & cov(y_1, y_m) \\ cov(y_2, y_1) & V[y_2] & \cdots & cov(y_2, y_m) \\ \vdots & \vdots & \ddots & \vdots \\ cov(y_m, y_1) & cov(y_m, y_2) & \cdots & V[y_m] \end{bmatrix}$$

$$= diag(\sigma_B^2) + diag(Q)diag(y) + \begin{bmatrix} CV_{0,1}^2 y_1^2 & cov(y_1, y_2) & \cdots & cov(y_1, y_m) \\ cov(y_2, y_1) & CV_{0,2}^2 y_2^2 & \cdots & cov(y_2, y_m) \\ \vdots & \vdots & \ddots & \vdots \\ cov(y_m, y_1) & cov(y_m, y_2) & \cdots & CV_{0,m}^2 y_m^2 \end{bmatrix}$$

wherein *diag(x)* indicates generating a diagonal matrix from the column vector *x,* and *cov(a, b)* denotes the covariance between *a* and *b*.

In some instances, the memory includes instructions to calculate the generalized least squares problem by multiplying by the inverse of the calculated covariance matrix. In some instances, the memory includes instructions to estimate data signal covariance based on fluorescence intensity across two or more photodetectors of the light detection system. In some instances, the memory includes instructions to estimate data signal covariance based on fluorescence intensity across each of the photodetection channels. In some embodiments, the memory includes instructions to calculate the generalized least squares problem according to:

$$\hat{f} = \arg\min_f (Xf - y)^T G (Xf - y)$$

wherein:

$G = (\Sigma^y)^{-1}$ is a weight matrix;

$\Sigma^y$ is a covariance matrix;

$X$ is a spectral matrix (spillover matrix);

$y$ is measured detector values from the plurality of photodetectors of the light detection system for each cell;

$f$ is true fluorophore abundances for each cell; and

$\hat{f}$ is estimated (unmixed) fluorophore abundances for each cell.

In some instances, the memory includes instructions to estimate a covariance matrix *a priori.* In certain instances, the memory includes instructions to apply the generalized least squares problem in real time (e.g., using an integrated circuit such as a field programmable gate array). In some instances, an *a priori* noise model is used to estimate each event covariance (e.g., calculate each event covariance matrix in real time). In certain instances, the *a priori* noise model uses only data collected for each specific event. In some instances, the covariance matrix includes estimations from an entire collected dataset.

In some embodiments, the memory includes instructions to determine data signal covariance by estimating by iterative optimization with a covariance matrix that minimizes variance of unmixed data signals. In certain instances, the memory includes instructions to calculate the generalized least squares problem by Cholesky decomposition of the covariance matrix. In certain instances, the generalized least squares problem is characterized by computing the Cholesky decomposition of the covariance matrix $\Sigma^y = CC^T$ and solving triangular systems to generate transformed inputs for an ordinary least squares algorithm according to:

Solving $C\bar{y} = y$ to generate $\bar{y}$, a decorrelated and whitened version of the data vector $y$.
Solving $C\bar{X} = X$ to generate $\bar{X}$, the corresponding transformed version of the spectral matrix $X$.
The transformed system $\bar{X}f = \bar{y}$ is solved by ordinary least squares, for example by solving the so-called normal equations $\bar{X}^T\bar{X}f = \bar{X}^T\bar{y}$.

In some embodiments, the memory includes instructions to find the least-squares solution of the generalized least squares problem. In some instances, the memory includes instructions to find the least-squares solution of the generalized least squares problem by one or more of matrix decomposition, matrix factorization, QR factorization, Cholesky decomposition, singular value decomposition, LDL decomposition, and forward- and back-substitution. In some instances, the memory includes instructions to find the least-squares solution of the generalized least squares problem by solving the so-called normal equations via Cholesky or LDL decomposition, such as by Cholesky or LDL decomposition according to:

$$X^TGX\hat{f} = X^TGy$$

where $y$ is measured detector values from the plurality of photodetectors of the light detection system for each cell; $X$ is spillover; and

$$G \text{ is } \begin{bmatrix} G_{1,1} & G_{1,2} & \cdots & G_{1,m} \\ G_{2,1} & G_{2,2} & \cdots & G_{2,m} \\ \vdots & \vdots & \ddots & \vdots \\ G_{m,1} & G_{m,2} & \cdots & G_{m,m} \end{bmatrix}$$

In some instances, the memory includes instructions to find the least-squares solution of the generalized least squares problem by Cholesky or LDL decomposition according to:

$$X^TGX\hat{f} = X^TGy$$

$A\hat{f} = B$
$LDL^T\hat{f} = B$            LDL decomposition
$Lz = B$ where $z = DL^T\hat{f}$      Lower-triangular matrix solution
$Dx = z$ where $x = L^T\hat{f}$      Diagonal matrix solution
$L^T\hat{f} = x$, solve for $\hat{f}$      Upper-triangular matrix solution

In some embodiments, the memory includes instructions to find the least-squares solution of the generalized least squares problem by matrix decomposition.

In some embodiments, the memory includes instructions to find the least-squares solution of the generalized least squares problem by matrix factorization. In some instances, the memory includes instructions to find the least-squares solution of the generalized least squares problem by QR factorization. In some instances, the memory includes instructions to calculate the generalized least squares problem using transformed $\bar{X}$ and $\bar{y}$ (as calculated via Cholesky decomposition of the covariance matrix described above) using QR factorization according to:

$\bar{X}^T\bar{X}f = \bar{X}^T\bar{y}$      Normal equations for transformed GLS problem
$QR = \bar{X}$      QR decomposition of transformed $X$
$(QR)^TQR\hat{f} = (QR)^T\bar{y}$      Substitution

(continued)

| | |
|---|---|
| $R^TQ^TQR\hat{f} = R^TQ^T\overline{y}$ | Expand transpose of $QR$ |
| $R^TR\hat{f} = R^TQ^T\overline{y}Q^TQ$ | cancels due to orthogonality |
| $R\hat{f} = Q^T\overline{y}.$ | Triangular solve for $\hat{f}$ |

In some embodiments, the memory includes instructions to find the least-squares solution of the generalized least squares problem by singular value decomposition. In some instances, the singular value decomposition is the matrix that is the product $\overline{X} = U\Sigma V^T$ where $U$ and $V$ are orthogonal matrices and $\Sigma$ is a diagonal matrix containing singular values of $\overline{X}$. In some instances, the singular value decomposition is the matrix that is the product $\overline{X} = U\Sigma V^T$ where $U$ and $V$ are orthogonal matrices and $\Sigma$ is a diagonal matrix containing singular values of $\overline{X}$. In some instances, the memory includes instructions to calculate the generalized least squares problem using singular value decomposition according to:

$$z = U^T\overline{y}$$

$$\Sigma w = z$$

$$\hat{f} = Vw,$$

where $U$ and $V$ are orthogonal matrices and $\Sigma$ is a diagonal matrix containing singular values of $\overline{X}$.

In some embodiments, the memory includes instructions to find the least-squares solution of the generalized least squares problem by LDL decomposition. In certain embodiments, the memory includes instructions to find the least-squares solution of the generalized least squares problem by forward- and back-substitution.

In certain embodiments, the subject systems include a field programmable gate array and the spectral unmixing algorithm is calculated for each cell in real time on a field programmable gated array.

In some embodiments, systems include a computer having a computer readable storage medium with a computer program stored thereon, where the computer program when loaded on the computer further includes instructions for calculating the abundance of one or more fluorophores in the sample from the spectrally resolved light from each fluorophore. In certain instances, the abundance of fluorophores associated with (e.g., chemically associated (i.e., covalently, ionically) or physically associated) a target particle is calculated from the spectrally resolved light from each fluorophore associated with the particle. For instance, in one example the relative abundance of each fluorophore associated with a target particle is calculated from the spectrally resolved light from each fluorophore. In another example, the absolute abundance of each fluorophore associated with the target particle is calculated from the spectrally resolved light from each fluorophore.

In certain embodiments, systems are configured to identify or classify a particle based on the relative abundance of each fluorophore determined to be associated with the particle. In these embodiments, the subject systems may be configured to identify or classify the particle by any convenient protocol such as by: comparing the relative or absolute abundance of each fluorophore associated with a particle with a control sample having particles of known identity; or by conducting spectroscopic or other assay analysis of a population of particles (e.g., cells) having the calculated relative or absolute abundance of associated fluorophores.

Systems according to some embodiments, may include a display and operator input device. Operator input devices may, for example, be a keyboard, mouse, or the like. The processing module includes a processor which has access to a memory having instructions stored thereon for performing the steps of the subject methods. The processing module may include an operating system, a graphical user interface (GUI) controller, a system memory, memory storage devices, and input-output controllers, cache memory, a data backup unit, and many other devices. The processor may be a commercially available processor or it may be one of other processors that are or will become available. The processor executes the operating system and the operating system interfaces with firmware and hardware in a well-known manner, and facilitates the processor in coordinating and executing the functions of various computer programs that may be written in a variety of programming languages, such as Java, Perl, C++, other high level or low-level languages, as well as combinations thereof, as is known in the art. The operating system, typically in cooperation with the processor, coordinates and executes functions of the other components of the computer. The operating system also provides scheduling, input-output control, file and data management, memory management, and communication control and related services, all in accordance with known techniques. The processor may be any suitable analog or digital system. In some embodiments, the processor includes analog electronics which provide feedback control, such as for example negative feedback control.

The system memory may be any of a variety of known or future memory storage devices. Examples include any commonly available random-access memory (RAM), magnetic medium such as a resident hard disk or tape, an optical medium such as a read and write compact disc, flash memory devices, or other memory storage device. The memory storage device may be any of a variety of known or future devices, including a compact disk drive, a tape drive, a removable hard disk drive, or a diskette drive. Such types of memory storage devices typically read from, and/or write to, a program storage medium (not shown) such as, respectively, a compact disk, magnetic tape, removable hard disk, or floppy diskette. Any of these program storage media, or others now in use or that may later be developed, may be considered a computer program product. As will be appreciated, these program storage media typically store a computer software program and/or data. Computer software programs, also called computer control logic, typically are stored in system memory and/or the program storage device used in conjunction with the memory storage device.

In some embodiments, a computer program product is described comprising a computer usable medium having control logic (computer software program, including program code) stored therein. The control logic, when executed by the processor the computer, causes the processor to perform functions described herein. In other embodiments, some functions are implemented primarily in hardware using, for example, a hardware state machine. Implementation of the hardware state machine so as to perform the functions described herein will be apparent to those skilled in the relevant arts.

Memory may be any suitable device in which the processor can store and retrieve data, such as magnetic, optical, or solid-state storage devices (including magnetic or optical disks or tape or RAM, or any other suitable device, either fixed or portable). The processor may include a general-purpose digital microprocessor suitably programmed from a computer readable medium carrying necessary program code. Programming can be provided remotely to processor through a communication channel, or previously saved in a computer program product such as memory or some other portable or fixed computer readable storage medium using any of those devices in connection with memory. For example, a magnetic or optical disk may carry the programming, and can be read by a disk writer/reader. Systems of the invention also include programming, e.g., in the form of computer program products, algorithms for use in practicing the methods as described above. Programming according to the present invention can be recorded on computer readable media, e.g., any medium that can be read and accessed directly by a computer. Such media include, but are not limited to: magnetic storage media, such as floppy discs, hard disc storage medium, and magnetic tape; optical storage media such as CD-ROM; electrical storage media such as RAM and ROM; portable flash drive; and hybrids of these categories such as magnetic/optical storage media.

The processor may also have access to a communication channel to communicate with a user at a remote location. By remote location is meant the user is not directly in contact with the system and relays input information to an input manager from an external device, such as a a computer connected to a Wide Area Network ("WAN"), telephone network, satellite network, or any other suitable communication channel, including a mobile telephone (i.e., smartphone).

In some embodiments, systems according to the present disclosure may be configured to include a communication interface. In some embodiments, the communication interface includes a receiver and/or transmitter for communicating with a network and/or another device. The communication interface can be configured for wired or wireless communication, including, but not limited to, radio frequency (RF) communication (e.g., Radio-Frequency Identification (RFID), Zigbee communication protocols, WiFi, infrared, wireless Universal Serial Bus (USB), Ultra-Wide Band (UWB), Bluetooth® communication protocols, and cellular communication, such as code division multiple access (CDMA) or Global System for Mobile communications (GSM).

In one embodiment, the communication interface is configured to include one or more communication ports, e.g., physical ports or interfaces such as a USB port, an RS-232 port, or any other suitable electrical connection port to allow data communication between the subject systems and other external devices such as a computer terminal (for example, at a physician's office or in hospital environment) that is configured for similar complementary data communication.

In one embodiment, the communication interface is configured for infrared communication, Bluetooth® communication, or any other suitable wireless communication protocol to enable the subject systems to communicate with other devices such as computer terminals and/or networks, communication enabled mobile telephones, personal digital assistants, or any other communication devices which the user may use in conjunction.

In one embodiment, the communication interface is configured to provide a connection for data transfer utilizing Internet Protocol (IP) through a cell phone network, Short Message Service (SMS), wireless connection to a personal computer (PC) on a Local Area Network (LAN) which is connected to the internet, or WiFi connection to the internet at a WiFi hotspot.

In one embodiment, the subject systems are configured to wirelessly communicate with a server device via the communication interface, e.g., using a common standard such as 802.11 or Bluetooth® RF protocol, or an IrDA infrared protocol. The server device may be another portable device, such as a smart phone, Personal Digital Assistant (PDA) or notebook computer; or a larger device such as a desktop computer, appliance, etc. In some embodiments, the server device has a display, such as a liquid crystal display (LCD), as well as an input device, such as buttons, a keyboard, mouse or touch-screen.

In some embodiments, the communication interface is configured to automatically or semi-automatically communicate data stored in the subject systems, e.g., in an optional data storage unit, with a network or server device using one or more of the communication protocols and/or mechanisms described above.

Output controllers may include controllers for any of a variety of known display devices for presenting information to a user, whether a human or a machine, whether local or remote. If one of the display devices provides visual information, this information typically may be logically and/or physically organized as an array of picture elements. A graphical user interface (GUI) controller may include any of a variety of known or future software programs for providing graphical input and output interfaces between the system and a user, and for processing user inputs. The functional elements of the computer may communicate with each other via system bus. Some of these communications may be accomplished in alternative embodiments using network or other types of remote communications. The output manager may also provide information generated by the processing module to a user at a remote location, e.g., over the Internet, phone or satellite network, in accordance with known techniques. The presentation of data by the output manager may be implemented in accordance with a variety of known techniques. As some examples, data may include SQL, HTML or XML documents, email or other files, or data in other forms. The data may include Internet URL addresses so that a user may retrieve additional SQL, HTML, XML, or other documents or data from remote sources. The one or more platforms present in the subject systems may be any type of known computer platform or a type to be developed in the future, although they typically will be of a class of computer commonly referred to as servers. However, they may also be a main-frame computer, a work station, or other computer type. They may be connected via any known or future type of cabling or other communication system including wireless systems, either networked or otherwise. They may be co-located or they may be physically separated. Various operating systems may be employed on any of the computer platforms, possibly depending on the type and/or make of computer platform chosen. Appropriate operating systems include Windows 10, Windows NT®, Windows XP, Windows 7, Windows 8, iOS, Sun Solaris, Linux, OS/400, Compaq Tru64 Unix, SGI IRIX, Siemens Reliant Unix, Ubuntu, Zorin OS and others.

In certain embodiments, the subject systems include one or more optical adjustment components for adjusting the light such as light irradiated onto the sample (e.g., from a laser) or light collected from the sample (e.g., scattered, fluorescence). For example, the optical adjustment may be to increase the dimensions of the light, the focus of the light or to collimate the light. In some instances, optical adjustment is a magnification protocol so as to increase the dimensions of the light (e.g., beam spot), such as increasing the dimensions by 5% or more, such as by 10% or more, such as by 25% or more, such as by 50% or more and including increasing the dimensions by 75% or more. In other embodiments, optical adjustment includes focusing the light so as to reduce the light dimensions, such as by 5% or greater, such as by 10% or greater, such as by 25% or greater, such as by 50% or greater and including reducing the dimensions of the beam spot by 75% or greater. In certain embodiments, optical adjustment includes collimating the light. The term "collimate" is used in its conventional sense to refer to the optically adjusting the collinearity of light propagation or reducing divergence by the light of from a common axis of propagation. In some instances, collimating includes narrowing the spatial cross section of a light beam (e.g., reducing the beam profile of a laser)

In some embodiments, the optical adjustment component is a focusing lens having a magnification ratio of from 0.1 to 0.95, such as a magnification ratio of from 0.2 to 0.9, such as a magnification ratio of from 0.3 to 0.85, such as a magnification ratio of from 0.35 to 0.8, such as a magnification ratio of from 0.5 to 0.75 and including a magnification ratio of from 0.55 to 0.7, for example a magnification ratio of 0.6. For example, the focusing lens is, in certain instances, a double achromatic de-magnifying lens having a magnification ratio of about 0.6. The focal length of the focusing lens may vary, ranging from 5 mm to 20 mm, such as from 6 mm to 19 mm, such as from 7 mm to 18 mm, such as from 8 mm to 17 mm, such as from 9 mm to 16 and including a focal length ranging from 10 mm to 15 mm. In certain embodiments, the focusing lens has a focal length of about 13 mm.

In other embodiments, the optical adjustment component is a collimator. The collimator may be any convenient collimating protocol, such as one or more mirrors or curved lenses or a combination thereof. For example, the collimator is in certain instances a single collimating lens. In other instances, the collimator is a collimating mirror. In yet other instances, the collimator includes two lenses. In still other instances, the collimator includes a mirror and a lens. Where the collimator includes one or more lenses, the focal length of the collimating lens may vary, ranging from 5 mm to 40 mm, such as from 6 mm to 37.5 mm, such as from 7 mm to 35 mm, such as from 8 mm to 32.5 mm, such as from 9 mm to 30 mm, such as from 10 mm to 27.5 mm, such as from 12.5 mm to 25 mm and including a focal length ranging from 15 mm to 20 mm.

In some embodiments, the subject systems include a flow cell nozzle having a nozzle orifice configured to flow a flow stream through the flow cell nozzle. The subject flow cell nozzle has an orifice which propagates a fluidic sample to a sample interrogation region, where in some embodiments, the flow cell nozzle includes a proximal cylindrical portion defining a longitudinal axis and a distal frustoconical portion which terminates in a flat surface having the nozzle orifice that is transverse to the longitudinal axis. The length of the proximal cylindrical portion (as measured along the longitudinal axis) may vary ranging from 1 mm to 15 mm, such as from 1.5 mm to 12.5 mm, such as from 2 mm to 10 mm, such as from 3 mm to 9 mm and including from 4 mm to 8 mm. The length of the distal frustoconical portion (as measured along the longitudinal axis) may also vary, ranging from 1 mm to 10 mm, such as from 2 mm to 9 mm, such as from 3 mm to 8 mm and including from 4 mm to 7 mm. The diameter of the of the flow cell nozzle chamber may vary, in some embodiments, ranging from 1 mm to 10 mm, such as from 2 mm to 9 mm, such as from 3 mm to 8 mm and including from 4 mm to 7 mm.

In certain instances, the nozzle chamber does not include a cylindrical portion and the entire flow cell nozzle chamber is frustoconically shaped. In these embodiments, the length of the frustoconical nozzle chamber (as measured along the longitudinal axis transverse to the nozzle orifice), may range from 1 mm to 15 mm, such as from 1.5 mm to 12.5 mm, such as from 2 mm to 10 mm, such as from 3 mm to 9 mm and including from 4 mm to 8 mm. The diameter of the proximal portion of the frustoconical nozzle chamber may range from 1 mm to 10 mm, such as from 2 mm to 9 mm, such as from 3 mm to 8 mm and including from 4 mm to 7 mm.

In embodiments, the sample flow stream emanates from an orifice at the distal end of the flow cell nozzle. Depending on the desired characteristics of the flow stream, the flow cell nozzle orifice may be any suitable shape where cross-sectional shapes of interest include, but are not limited to: rectilinear cross sectional shapes, e.g., squares, rectangles, trapezoids, triangles, hexagons, etc., curvilinear cross-sectional shapes, e.g., circles, ovals, as well as irregular shapes, e.g., a parabolic bottom portion coupled to a planar top portion. In certain embodiments, flow cell nozzle of interest has a circular orifice. The size of the nozzle orifice may vary, in some embodiments ranging from 1 $\mu$m to 20000 $\mu$m, such as from 2 $\mu$m to 17500 $\mu$m, such as from 5 $\mu$m to 15000 $\mu$m, such as from 10 $\mu$m to 12500 $\mu$m, such as from 15 $\mu$m to 10000 $\mu$m, such as from 25 $\mu$m to 7500 $\mu$m, such as from 50 $\mu$m to 5000 $\mu$m, such as from 75 $\mu$m to 1000 $\mu$m, such as from 100 $\mu$m to 750 $\mu$m and including from 150 $\mu$m to 500 $\mu$m. In certain embodiments, the nozzle orifice is 100 $\mu$m.

In some embodiments, the flow cell nozzle includes a sample injection port configured to provide a sample to the flow cell nozzle. In embodiments, the sample injection system is configured to provide suitable flow of sample to the flow cell nozzle chamber. Depending on the desired characteristics of the flow stream, the rate of sample conveyed to the flow cell nozzle chamber by the sample injection port may be 1 $\mu$L/sec or more, such as 2 $\mu$L/sec or more, such as 3 $\mu$L/sec or more, such as 5 $\mu$L/sec or more, such as 10 $\mu$L/sec or more, such as 15 $\mu$L/sec or more, such as 25 $\mu$L/sec or more, such as 50 $\mu$L/sec or more, such as 100 $\mu$L/sec or more, such as 150 $\mu$L/sec or more , such as 200 $\mu$L/sec or more, such as 250 $\mu$L/sec or more, such as 300 $\mu$L/sec or more, such as 350 $\mu$L/sec or more, such as 400 $\mu$L/sec or more, such as 450 $\mu$L/sec or more and including 500 $\mu$L/sec or more. For example, the sample flow rate may range from 1 $\mu$L/sec to about 500 $\mu$L/sec, such as from 2 $\mu$L/sec to about 450 $\mu$L/sec, such as from 3 $\mu$L/sec to about 400 $\mu$L/sec, such as from 4 $\mu$L/sec to about 350 $\mu$L/sec, such as from 5 $\mu$L/sec to about 300 $\mu$L/sec, such as from 6 $\mu$L/sec to about 250 $\mu$L/sec, such as from 7 $\mu$L/sec to about 200 $\mu$L/sec, such as from 8 $\mu$L/sec to about 150 $\mu$L/sec, such as from 9 $\mu$L/sec to about 125 $\mu$L/sec and including from 10 $\mu$L/sec to about 100 $\mu$L/sec.

The sample injection port may be an orifice positioned in a wall of the nozzle chamber or may be a conduit positioned at the proximal end of the nozzle chamber. Where the sample injection port is an orifice positioned in a wall of the nozzle chamber, the sample injection port orifice may be any suitable shape where cross-sectional shapes of interest include, but are not limited to: rectilinear cross sectional shapes, e.g., squares, rectangles, trapezoids, triangles, hexagons, etc., curvilinear cross-sectional shapes, e.g., circles, ovals, etc., as well as irregular shapes, e.g., a parabolic bottom portion coupled to a planar top portion. In certain embodiments, the sample injection port has a circular orifice. The size of the sample injection port orifice may vary depending on shape, in certain instances, having an opening ranging from 0.1 mm to 5.0 mm, e.g., 0.2 to 3.0 mm, e.g., 0.5 mm to 2.5 mm, such as from 0.75 mm to 2.25 mm, such as from 1 mm to 2 mm and including from 1.25 mm to 1.75 mm, for example 1.5 mm.

In certain instances, the sample injection port is a conduit positioned at a proximal end of the flow cell nozzle chamber. For example, the sample injection port may be a conduit positioned to have the orifice of the sample injection port in line with the flow cell nozzle orifice. Where the sample injection port is a conduit positioned in line with the flow cell nozzle orifice, the cross-sectional shape of the sample injection tube may be any suitable shape where cross-sectional shapes of interest include, but are not limited to: rectilinear cross sectional shapes, e.g., squares, rectangles, trapezoids, triangles, hexagons, etc., curvilinear cross-sectional shapes, e.g., circles, ovals, as well as irregular shapes, e.g., a parabolic bottom portion coupled to a planar top portion. The orifice of the conduit may vary depending on shape, in certain instances, having an opening ranging from 0.1 mm to 5.0 mm, e.g., 0.2 to 3.0 mm, e.g., 0.5 mm to 2.5 mm, such as from 0.75 mm to 2.25 mm, such as from 1 mm to 2 mm and including from 1.25 mm to 1.75 mm, for example 1.5 mm. The shape of the tip of the sample injection port may be the same or different from the cross-section shape of the sample injection tube. For example, the orifice of the sample injection port may include a beveled tip having a bevel angle ranging from 1° to 10°, such as from 2° to 9°, such as from 3° to 8°, such as from 4° to 7° and including a bevel angle of 5°.

In some embodiments, the flow cell nozzle also includes a sheath fluid injection port configured to provide a sheath fluid to the flow cell nozzle. In embodiments, the sheath fluid injection system is configured to provide a flow of sheath fluid to the flow cell nozzle chamber, for example in conjunction with the sample to produce a laminated flow stream of sheath fluid surrounding the sample flow stream. Depending on the desired characteristics of the flow stream, the rate of sheath fluid conveyed to the flow cell nozzle chamber by the may be 25 $\mu$L/sec or more, such as 50 $\mu$L/sec or more, such as 75 $\mu$L/sec or more, such as 100 $\mu$L/sec or more, such as 250 $\mu$L/sec or more, such as 500 $\mu$L/sec or more, such as 750 $\mu$L/sec or more, such as 1000 $\mu$L/sec or more and including 2500 $\mu$L/sec or more. For example, the sheath fluid flow rate may range from 1 $\mu$L/sec to about 500 $\mu$L/sec, such as from 2 $\mu$L/sec to about 450 $\mu$L/sec, such as from 3 $\mu$L/sec to about 400 $\mu$L/sec, such as from 4 $\mu$L/sec to about 350 $\mu$L/sec, such as from 5 $\mu$L/sec to about 300 $\mu$L/sec, such as from 6 $\mu$L/sec to about 250 $\mu$L/sec, such as from 7 $\mu$L/sec to about 200 $\mu$L/sec, such as from 8 $\mu$L/sec to about 150 $\mu$L/sec, such as from 9

μL/sec to about 125 μL/sec and including from 10 μL/sec to about 100 μL/sec.

In some embodiments, the sheath fluid injection port is an orifice positioned in a wall of the nozzle chamber. The sheath fluid injection port orifice may be any suitable shape where cross-sectional shapes of interest include, but are not limited to: rectilinear cross sectional shapes, e.g., squares, rectangles, trapezoids, triangles, hexagons, etc., curvilinear cross-sectional shapes, e.g., circles, ovals, as well as irregular shapes, e.g., a parabolic bottom portion coupled to a planar top portion. The size of the sample injection port orifice may vary depending on shape, in certain instances, having an opening ranging from 0.1 mm to 5.0 mm, e.g., 0.2 to 3.0 mm, e.g., 0.5 mm to 2.5 mm, such as from 0.75 mm to 2.25 mm, such as from 1 mm to 2 mm and including from 1.25 mm to 1.75 mm, for example 1.5 mm.

The subject systems, in certain instances, include a sample interrogation region in fluid communication with the flow cell nozzle orifice. In these instances, a sample flow stream emanates from an orifice at the distal end of the flow cell nozzle and particles in the flow stream may be irradiated with a light source at the sample interrogation region. The size of the interrogation region may vary depending on the properties of the flow nozzle, such as the size of the nozzle orifice and sample injection port size. In embodiments, the interrogation region may have a width that is 0.01 mm or more, such as 0.05 mm or more, such as 0.1 mm or more, such as 0.5 mm or more, such as 1 mm or more, such as 2 mm or more, such as 3 mm or more, such as 5 mm or more and including 10 mm or more. The length of the interrogation region may also vary, ranging in some instances along 0.01 mm or more, such as 0.1 mm or more, such as 0.5 mm or more, such as 1 mm or more, such as 1.5 mm or more, such as 2 mm or more, such as 3 mm or more, such as 5 mm or more, such as 10 or more, such as 15 mm or more, such as 20 mm or more, such as 25 mm or more and including 50 mm or more.

The interrogation region may be configured to facilitate irradiation of a planar cross-section of an emanating flow stream or may be configured to facilitate irradiation of a diffuse field (e.g., with a diffuse laser or lamp) of a predetermined length. In some embodiments, the interrogation region includes a transparent window that facilitates irradiation of a predetermined length of an emanating flow stream, such as 1 mm or more, such as 2 mm or more, such as 3 mm or more, such as 4 mm or more, such as 5 mm or more and including 10 mm or more. Depending on the light source used to irradiate the emanating flow stream (as described below), the interrogation region may be configured to pass light that ranges from 100 nm to 1500 nm, such as from 150 nm to 1400 nm, such as from 200 nm to 1300 nm, such as from 250 nm to 1200 nm, such as from 300 nm to 1100 nm, such as from 350 nm to 1000 nm, such as from 400 nm to 900 nm and including from 500 nm to 800 nm. As such, the interrogation region may be formed from any transparent material which passes the desired range of wavelength, including but not limited to optical glass, borosilicate glass, Pyrex glass, ultraviolet quartz, infrared quartz, sapphire as well as plastic, such as polycarbonates, polyvinyl chloride (PVC), polyurethanes, polyethers, polyamides, polyimides, or copolymers of these thermoplastics, such as PETG (glycol-modified polyethylene terephthalate), among other polymeric plastic materials, including polyester, where polyesters of interest may include, but are not limited to poly(alkylene terephthalates) such as polyethylene terephthalate) (PET), bottle-grade PET (a copolymer made based on monoethylene glycol, terephthalic acid, and other comonomers such as isophthalic acid, cyclohexene dimethanol, etc.), poly(butylene terephthalate) (PBT), and poly(hexamethylene terephthalate); poly(alkylene adipates) such as polyethylene adipate), poly(1,4-butylene adipate), and poly(hexamethylene adipate); poly(alkylene suberates) such as polyethylene suberate); poly(alkylene sebacates) such as polyethylene sebacate); poly(ε-caprolactone) and poly(β-propiolactone); poly(alkylene isophthalates) such as polyethylene isophthalate); poly(alkylene 2,6-naphthalene-dicarboxylates) such as polyethylene 2,6-naphthalene-dicarboxylate); poly(alkylene sulfonyl-4,4'-dibenzoates) such as polyethylene sulfonyl-4,4'-dibenzoate); poly(p-phenylene alkylene dicarboxylates) such as poly(p-phenylene ethylene dicarboxylates); poly(trans-1,4-cyclohexanediyl alkylene dicarboxylates) such as poly(trans-1,4-cyclohexanediyl ethylene dicarboxylate); poly(1,4-cyclohexane-dimethylene alkylene dicarboxylates) such as poly(1,4-cyclohexane-dimethylene ethylene dicarboxylate); poly([2.2.2]-bicyclooctane-1,4-dimethylene alkylene dicarboxylates) such as poly([2.2.2]-bicyclooctane-1,4-dimethylene ethylene dicarboxylate); lactic acid polymers and copolymers such as (S)-polylactide, (R,S)-polylactide, poly(tetramethylglycolide), and poly(lactide-co-glycolide); and polycarbonates of bisphenol A, 3,3'-dimethylbisphenol A, 3,3',5,5'-tetrachlorobisphenol A, 3,3',5,5'-tetramethylbisphenol A; polyamides such as poly(p-phenylene terephthalamide); polyesters, e.g., polyethylene terephthalates, e.g., Mylar™ polyethylene terephthalate; etc. In some embodiments, the subject systems include a cuvette positioned in the sample interrogation region. In embodiments, the cuvette may pass light that ranges from 100 nm to 1500 nm, such as from 150 nm to 1400 nm, such as from 200 nm to 1300 nm, such as from 250 nm to 1200 nm, such as from 300 nm to 1100 nm, such as from 350 nm to 1000 nm, such as from 400 nm to 900 nm and including from 500 nm to 800 nm.

In certain embodiments, light detection systems having the plurality of photodetectors as described above are part of or positioned in a particle analyzer, such as a particle sorter. In certain embodiments, the subject systems are flow cytometric systems that includes the photodiode and amplifier component as part of a light detection system for detecting light emitted by a sample in a flow stream. Suitable flow cytometry systems may include, but are not limited to, those described in Ormerod (ed.), Flow Cytometry: A Practical Approach, Oxford Univ. Press (1997); Jaroszeski et al. (eds.), Flow Cytometry Protocols, Methods in Molecular Biology No. 91, Humana Press (1997); Practical Flow Cytometry, 3rd ed., Wiley-Liss (1995); Virgo, et al. (2012) Ann Clin Biochem. Jan;49(pt 1):17-28; Linden, et. al., Semin Throm Hemost. 2004 Oct;30(5):502-11; Alison, et al. J Pathol, 2010 Dec; 222(4):335-344; and Herbig, *et al.*

(2007) Crit Rev Ther Drug Carrier Syst. 24(3):203-255; the disclosures of which are incorporated herein by reference. In certain instances, flow cytometry systems of interest include BD Biosciences FACSCanto™ flow cytometer, BD Biosciences FACSCanto™ II flow cytometer, BD Accuri™ flow cytometer, BD Accuri™ C6 Plus flow cytometer, BD Biosciences FACSCelesta™ flow cytometer, BD Biosciences FACSLyric™ flow cytometer, BD Biosciences FACSVerse™ flow cytometer, BD Biosciences FACSymphony™ flow cytometer, BD Biosciences LSRFortessa™ flow cytometer, BD Biosciences LSRFortessa™ X-20 flow cytometer, BD Biosciences FACSPresto™ flow cytometer, BD Biosciences FACSVia™ flow cytometer and BD Biosciences FACSCalibur™ cell sorter, a BD Biosciences FACSCount™ cell sorter, BD Biosciences FACSLyric™ cell sorter, BD Biosciences Via™ cell sorter, BD Biosciences Influx™ cell sorter, BD Biosciences Jazz™ cell sorter, BD Biosciences Aria™ cell sorter, BD Biosciences FACSAria™ II cell sorter, BD Biosciences FACSAria™ III cell sorter, BD Biosciences FACSAria™ Fusion cell sorter and BD Biosciences FACSMelody™ cell sorter, BD Biosciences FACSymphony™ S6 cell sorter or the like.

In some embodiments, the subject systems are flow cytometric systems, such those described in U.S. Patent Nos. 10,663,476; 10,620,111; 10,613,017; 10,605,713; 10,585,031; 10,578,542; 10,578,469; 10,481,074; 10,302,545; 10,145,793; 10,113,967; 10,006,852; 9,952,076; 9,933,341; 9,726,527; 9,453,789; 9,200,334; 9,097,640; 9,095,494; 9,092,034; 8,975,595; 8,753,573; 8,233,146; 8,140,300; 7,544,326; 7,201,875; 7,129,505; 6,821,740; 6,813,017; 6,809,804; 6,372,506; 5,700,692; 5,643,796; 5,627,040; 5,620,842; 5,602,039; 4,987,086; 4,498,766; the disclosures of which are herein incorporated by reference in their entirety.

In some embodiments, the subject systems are particle sorting systems that are configured to sort particles with an enclosed particle sorting module, such as those described in U.S. Patent Publication No. 2017/0299493, the disclosure of which is incorporated herein by reference. In certain embodiments, particles (e.g., cells) of the sample are sorted using a sort decision module having a plurality of sort decision units, such as those described in U.S. Patent Publication No. 2020/0256781, the disclosure of which is incorporated herein by reference. In some embodiments, the subject systems include a particle sorting module having deflector plates, such as described in U.S. Patent Publication No. 2017/0299493, filed on March 28, 2017, the disclosure of which is incorporated herein by reference.

In certain instances, flow cytometry systems of the invention are configured for imaging particles in a flow stream by fluorescence imaging using radiofrequency tagged emission (FIRE), such as those described in Diebold, et al. Nature Photonics Vol. 7(10); 806-810 (2013) as well as described in U.S. Patent Nos. 9,423,353; 9,784,661; 9,983,132; 10,006,852; 10,078,045; 10,036,699; 10,222,316; 10,288,546; 10,324,019; 10,408,758; 10,451,538; 10,620,111; and U.S. Patent Publication Nos. 2017/0133857; 2017/0328826; 2017/0350803; 2018/0275042; 2019/0376895 and 2019/0376894 the disclosures of which are herein incorporated by reference.

In certain embodiments, the subject systems are configured to sort one or more of the particles (e.g., cells) of the sample that are identified based on the estimated abundance of the fluorophores associated with the particle as described above. The term "sorting" is used herein in its conventional sense to refer to separating components (e.g., cells, non-cellular particles such as biological macromolecules) of the sample and in some instances delivering the separated components to one or more sample collection containers. For example, the subject systems may be configured for sorting samples having 2 or more components, such as 3 or more components, such as 4 or more components, such as 5 or more components, such as 10 or more components, such as 15 or more components and including soring a sample having 25 or more components. One or more of the sample components may be separated from the sample and delivered to a sample collection container, such as 2 or more sample components, such as 3 or more sample components, such as 4 or more sample components, such as 5 or more sample components, such as 10 or more sample components and including 15 or more sample components may be separated from the sample and delivered to a sample collection container.

In some embodiments, particle sorting systems of interest are configured to sort particles with an enclosed particle sorting module, such as those described in U.S. Patent Publication No. 2017/0299493, filed on March 28, 2017, the disclosure of which is incorporated herein by reference. In certain embodiments, particles (e.g., cells) of the sample are sorted using a sort decision module having a plurality of sort decision units, such as those described in U.S. Patent Publication No. 2020/0256781, the disclosure of which is incorporated herein by reference. In some embodiments, the subject systems include a particle sorting module having deflector plates, such as described in U.S. Patent Publication No. 2017/0299493, filed on March 28, 2017, the disclosure of which is incorporated herein by reference.

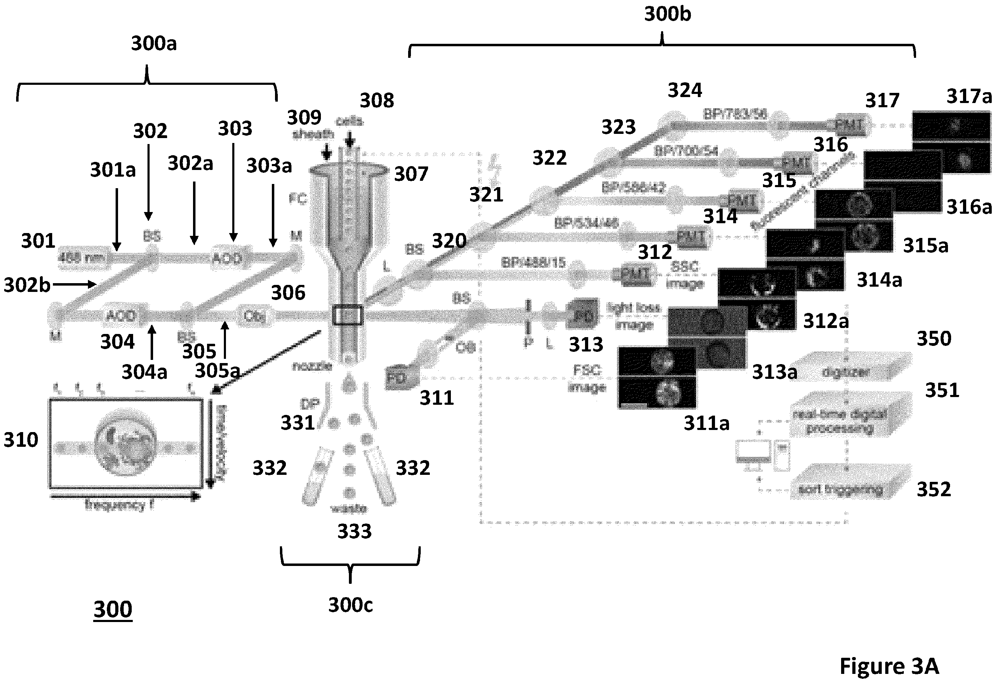

In certain embodiments, systems are a fluorescence imaging using radiofrequency tagged emission image-enabled particle sorter, such as depicted in FIG. 3A. Particle sorter 300 includes a light irradiation component 300a which includes light source 301 (e.g., 488 nm laser) which generates output beam of light 301a that is split with beamsplitter 302 into beams 302a and 302b. Light beam 302a is propagated through acousto-optic device (e.g., an acousto-optic deflector, AOD) 303 to generate an output beam 303a having one or more angularly deflected beams of light. In some instances, output beam 303a generated from acousto-optic device 303 includes a local oscillator beam and a plurality of radiofrequency comb beams. Light beam 302b is propagated through acousto-optic device (e.g., an acousto-optic deflector, AOD) 304 to generate an output beam 304a having one or more angularly deflected beams of light. In some instances, output beam 304a generated from acousto-optic device 304 includes a local oscillator beam and a plurality of radiofrequency comb beams. Output beams 303a and 304a generated from acousto-optic devices 303 and 304, respectively are combined with beamsplitter 305 to generate output beam 305a which is conveyed through an optical component 306 (e.g., an objective lens) to irradiate particles in flow cell 307. In certain embodiments, acousto-optic device 303 (AOD) splits a single laser beam into an array of beamlets, each having different optical frequency and angle. Second AOD 304 tunes the optical frequency of a reference beam, which is then overlapped with the array of beamlets at beam combiner 305. In certain embodiments, the light irradiation system having a light source and acousto-optic device can also include those described in Schraivogel, et al. ("High-speed fluorescence image-enabled cell sorting" Science (2022), 375 (6578): 315-320) and United States Patent Publication No. 2021/0404943, the disclosure of which is herein incorporated by reference.

Output beam 305a irradiates sample particles 308 propagating through flow cell 307 (e.g., with sheath fluid 309) at irradiation region 310. As shown in irradiation region 310, a plurality of beams (e.g., angularly deflected radiofrequency shifted beams of light depicted as dots across irradiation region 310) overlaps with a reference local oscillator beam (depicted as the shaded line across irradiation region 310). Due to their differing optical frequencies, the overlapping beams exhibit a beating behavior, which causes each beamlet to carry a sinusoidal modulation at a distinct frequency $f_{1-n}$.

Light from the irradiated sample is conveyed to light detection system 300b that includes a plurality of photodetectors. Light detection system 300b includes forward scattered light photodetector 311 for generating forward scatter images 311a and a side scattered light photodetector 312 for generating side scatter images 312a. Light detection system 300b also includes brightfield photodetector 313 for generating light loss images 313a. In some embodiments, forward scatter detector 311 and side scatter detector 312 are photodiodes (e.g., avalanche photodiodes, APDs). In some instances, brightfield photodetector 313 is a photomultiplier tube (PMT). Fluorescence from the irradiated sample is also detected with fluorescence photodetectors 314-317. In some instances, photodetectors 314-317 are photomultiplier tubes. Light from the irradiated sample is directed to the side scatter detection channel 312 and fluorescence detection channels 314-317 through beamsplitter 320. Light detection system 300b includes bandpass optical components 321, 322, 323 and 324 (e.g., dichroic mirrors) for propagating predetermined wavelength of light to photodetectors 314-317. In some instances, optical component 321 is a 534 nm/40 nm bandpass. In some instances, optical component 322 is a 586 nm/42 nm bandpass. In some instances, optical component 323 is a 700 nm/54 nm bandpass. In some instances, optical component 324 is a 783 nm/56 nm bandpass. The first number represents the center of a spectral band. The second number provides a range of the spectral band. Thus, a 510/20 filter extends 10 nm on each side of the center of the spectral band, or from 500 nm to 520 nm.

Data signals generated in response to light detected in scattered light detection channels 311 and 312, brightfield light detection channel 313 and fluorescence detection channels 314-317 are processed by real-time digital processing with processors 350 and 351. Images 311a-317a can be generated in each light detection channel based on the data signals generated in processors 350 and 351. Image-enabled sorting is performed in response to a sort signal generated in sort trigger 352. Sorting component 300c includes deflection plates 331 for deflecting particles into sample containers 332 or to waste stream 333. In some instances, sort component 300c is configured to sort particles with an enclosed particle sorting module, such as those described in U.S. Patent Publication No. 2017/0299493, filed on March 28, 2017, the disclosure of which is incorporated herein by reference. In certain embodiments, sorting component 300c includes a sort decision module having a plurality of sort decision units, such as those described in U.S. Patent Publication No. 2020/0256781, the disclosure of which is incorporated herein by reference.

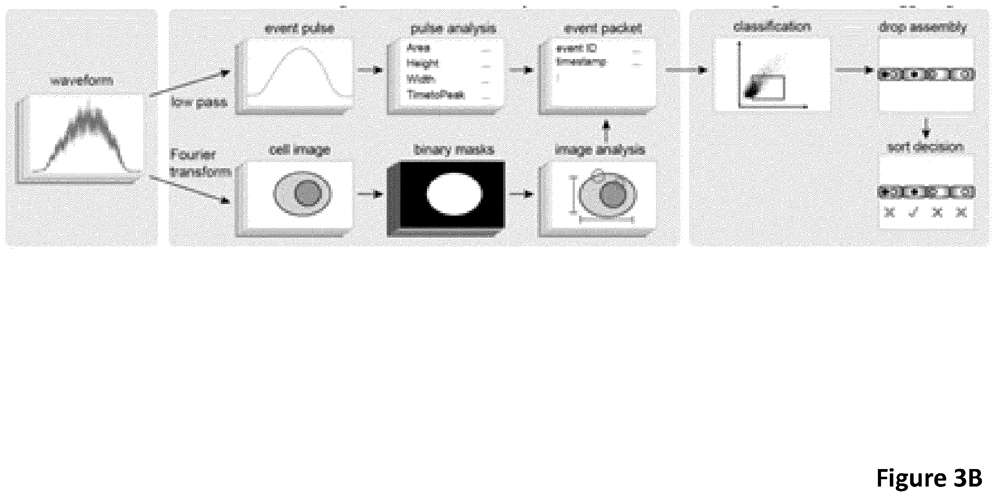

Figure 3B depicts image-enabled particle sorting data processing according to certain embodiments. In some instances, image-enabled particle sorting data processing is a low-latency data processing pipeline. Each photodetector produces a pulse with high-frequency modulations encoding the image (waveform). Fourier analysis is performed to reconstruct the image from the modulated pulse. An image processing pipeline produces a set of image features (image analysis), which are combined with features derived from a pulse processing pipeline (event packet). Real-time sort classification electronics then classify the particle based on image features, producing a sort decision that is used to selectively charge the droplets.

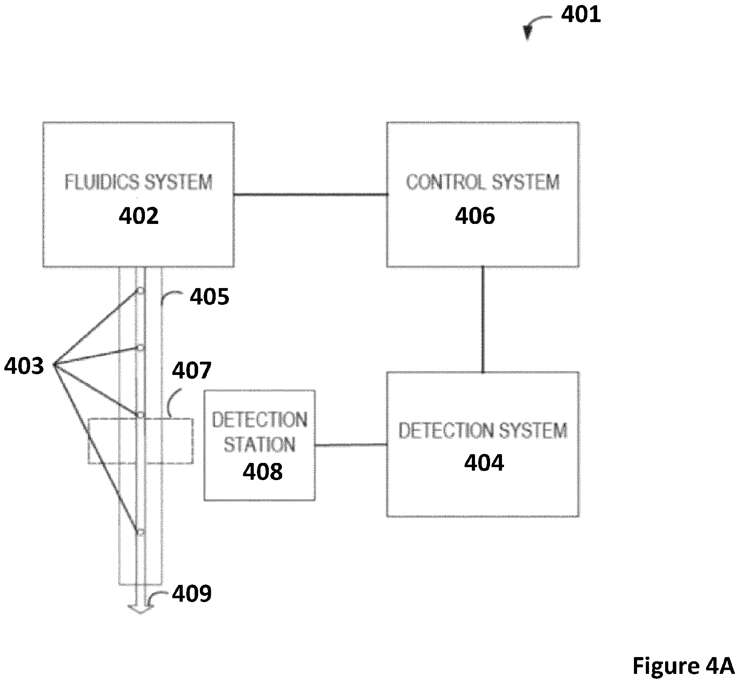

In some embodiments, systems are particle analyzers where the particle analysis system 401 (FIG. 4A) can be used to analyze and characterize particles, with or without physically sorting the particles into collection vessels. FIG. 4A shows a functional block diagram of a particle analysis system for computational based sample analysis and particle characterization. In some embodiments, the particle analysis system 401 is a flow system. The particle analysis system 401 shown in FIG. 4A can be configured to perform, in whole or in part, the methods described herein such as. The particle analysis system 401 includes a fluidics system 402. The fluidics system 402 can include or be coupled with a sample tube 405 and a moving fluid column within the sample tube in which particles 403 (e.g. cells) of a sample move along a common sample path 409.

The particle analysis system 401 includes a detection system 404 configured to collect a signal from each particle as it passes one or more detection stations along the common sample path. A detection station 408 generally refers to a monitored area 407 of the common sample path. Detection can, in some implementations, include detecting light or one or more other properties of the particles 403 as they pass through a monitored area 407. In FIG. 4A, one detection station 408 with one monitored area 407 is shown. Some implementations of the particle analysis system 401 can include multiple detection stations. Furthermore, some detection stations can monitor more than one area.

Each signal is assigned a signal value to form a data point for each particle. As described above, this data can be referred to as event data. The data point can be a multidimensional data point including values for respective properties measured for a particle. The detection system 404 is configured to collect a succession of such data points in a first-time interval.

The particle analysis system 401 can also include a control system 306. The control system 406 can include one or more processors, an amplitude control circuit and/or a frequency control circuit. The control system shown can be operationally associated with the fluidics system 402. The control system can be configured to generate a calculated signal frequency for at least a portion of the first-time interval based on a Poisson distribution and the number of data points collected by the detection system 404 during the first time interval. The control system 406 can be further configured to generate an experimental signal frequency based on the number of data points in the portion of the first time interval. The control system 406 can additionally compare the experimental signal frequency with that of a calculated signal frequency or a predetermined signal frequency.

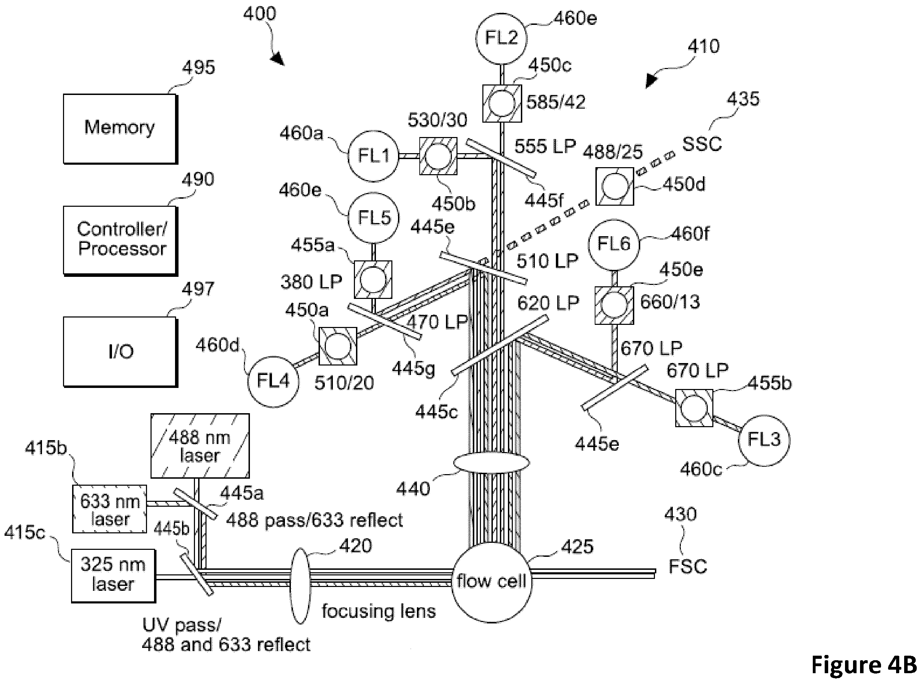

FIG. 4B shows a system 400 for flow cytometry in accordance with an illustrative embodiment of the present invention. The system 400 includes a flow cytometer 410, a controller/processor 490 and a memory 495. The flow cytometer 410 includes one or more excitation lasers 415a-415c, a focusing lens 420, a flow chamber 425, a forward scatter detector 430, a side scatter detector 435, a fluorescence collection lens 440, one or more beam splitters 445a-445g, one or more bandpass filters 450a-450e, one or more longpass ("LP") filters 455a-455b, and one or more fluorescent detectors 460a-460f.

The excitation lasers 115a-c emit light in the form of a laser beam. The wavelengths of the laser beams emitted from excitation lasers 415a-415c are 488 nm, 633 nm, and 325 nm, respectively, in the example system of FIG. 4B. The laser beams are first directed through one or more of beam splitters 445a and 445b. Beam splitter 445a transmits light at 488 nm and reflects light at 633 nm. Beam splitter 445b transmits UV light (light with a wavelength in the range of 10 to 400 nm) and reflects light at 488 nm and 633 nm.

The laser beams are then directed to a focusing lens 420, which focuses the beams onto the portion of a fluid stream where particles of a sample are located, within the flow chamber 425. The flow chamber is part of a fluidics system which directs particles, typically one at a time, in a stream to the focused laser beam for interrogation. The flow chamber can comprise a flow cell in a benchtop cytometer or a nozzle tip in a stream-in-air cytometer.

The light from the laser beam(s) interacts with the particles in the sample by diffraction, refraction, reflection, scattering, and absorption with re-emission at various different wavelengths depending on the characteristics of the particle such as its size, internal structure, and the presence of one or more fluorescent molecules attached to or naturally present on or in the particle. The fluorescence emissions as well as the diffracted light, refracted light, reflected light, and scattered light may be routed to one or more of the forward scatter detector 430, the side scatter detector 435, and the one or more fluorescent detectors 460a-460f through one or more of the beam splitters 445a-445g, the bandpass filters 450a-450e, the longpass filters 455a-455b, and the fluorescence collection lens 440.

The fluorescence collection lens 440 collects light emitted from the particle- laser beam interaction and routes that light towards one or more beam splitters and filters. Bandpass filters, such as bandpass filters 450a-450e, allow a narrow range of wavelengths to pass through the filter. For example, bandpass filter 450a is a 510/20 filter. The first number represents the center of a spectral band. The second number provides a range of the spectral band. Thus, a 510/20 filter extends 10 nm on each side of the center of the spectral band, or from 500 nm to 520 nm. Shortpass filters transmit wavelengths of light equal to or shorter than a specified wavelength. Longpass filters, such as longpass filters 455a-455b, transmit wavelengths of light equal to or longer than a specified wavelength of light. For example, longpass filter 455a, which is a 670 nm longpass filter, transmits light equal to or longer than 670 nm. Filters are often selected to optimize the specificity of a detector for a particular fluorescent dye. The filters can be configured so that the spectral band of light transmitted to the detector is close to the emission peak of a fluorescent dye.

Beam splitters direct light of different wavelengths in different directions. Beam splitters can be characterized by filter properties such as shortpass and longpass. For example, beam splitter 445g is a 620 SP beam splitter, meaning that the beam splitter 445g transmits wavelengths of light that are 620 nm or shorter and reflects wavelengths of light that are longer than 620 nm in a different direction. In one embodiment, the beam splitters 445a-445g can comprise optical mirrors, such as dichroic mirrors.

The forward scatter detector 430 is positioned slightly off axis from the direct beam through the flow cell and is configured to detect diffracted light, the excitation light that travels through or around the particle in mostly a forward direction. The intensity of the light detected by the forward scatter detector is dependent on the overall size of the particle. The forward scatter detector can include a photodiode. The side scatter detector 435 is configured to detect refracted and reflected light from the surfaces and internal structures of the particle, and tends to increase with increasing particle complexity of structure. The fluorescence emissions from fluorescent molecules associated with the particle can be detected by the one or more fluorescent detectors 460a-460f. The side scatter detector 435 and fluorescent detectors can include photomultiplier tubes. The signals detected at the forward scatter detector 430, the side scatter detector 435 and the fluorescent detectors can be converted to electronic signals (voltages) by the detectors. This data can provide information about the sample.

One of skill in the art will recognize that a flow cytometer in accordance with an embodiment of the present invention is not limited to the flow cytometer depicted in FIG. 4B, but can include any flow cytometer known in the art. For example, a flow cytometer may have any number of lasers, beam splitters, filters, and detectors at various wavelengths and in various different configurations.

In operation, cytometer operation is controlled by a controller/processor 490, and the measurement data from the detectors can be stored in the memory 495 and processed by the controller/processor 490. Although not shown explicitly, the controller/processor 190 is coupled to the detectors to receive the output signals therefrom, and may also be coupled to electrical and electromechanical components of the flow cytometer 400 to control the lasers, fluid flow parameters, and the like. Input/output (I/O) capabilities 497 may be provided also in the system. The memory 495, controller/processor 490, and I/O 497 may be entirely provided as an integral part of the flow cytometer 410. In such an embodiment, a display may also form part of the I/O capabilities 497 for presenting experimental data to users of the cytometer 400. Alternatively, some or all of the memory 495 and controller/processor 490 and I/O capabilities may be part of one or more external devices such as a general purpose computer. In some embodiments, some or all of the memory 495 and controller/processor 490 can be in wireless or wired communication with the cytometer 410. The controller/processor 490 in conjunction with the memory 495 and the I/O 497 can be configured to perform various functions related to the preparation and analysis of a flow cytometer experiment.

The system illustrated in FIG. 4B includes six different detectors that detect fluorescent light in six different wavelength bands (which may be referred to herein as a "filter window" for a given detector) as defined by the configuration of filters and/or splitters in the beam path from the flow cell 425 to each detector. Different fluorescent molecules used for a flow cytometer experiment will emit light in their own characteristic wavelength bands. The particular fluorescent labels used for an experiment and their associated fluorescent emission bands may be selected to generally coincide with the filter windows of the detectors. However, as more detectors are provided, and more labels are utilized, perfect correspondence between filter windows and fluorescent emission spectra is not possible. It is generally true that although the peak of the emission spectra of a particular fluorescent molecule may lie within the filter window of one particular detector, some of the emission spectra of that label will also overlap the filter windows of one or more other detectors. This may be referred to as spillover. The I/O 497 can be configured to receive data regarding a flow cytometer experiment having a panel of fluorescent labels and a plurality of cell populations having a plurality of markers, each cell population having a subset of the plurality of markers. The I/O 497 can also be configured to receive biological data assigning one or more markers to one or more cell populations, marker density data, emission spectrum data, data assigning labels to one or more markers, and cytometer configuration data. Flow cytometer experiment data, such as label spectral characteristics and flow cytometer configuration data can also be stored in the memory 495. The controller/processor 490 can be configured to evaluate one or more assignments of labels to markers.

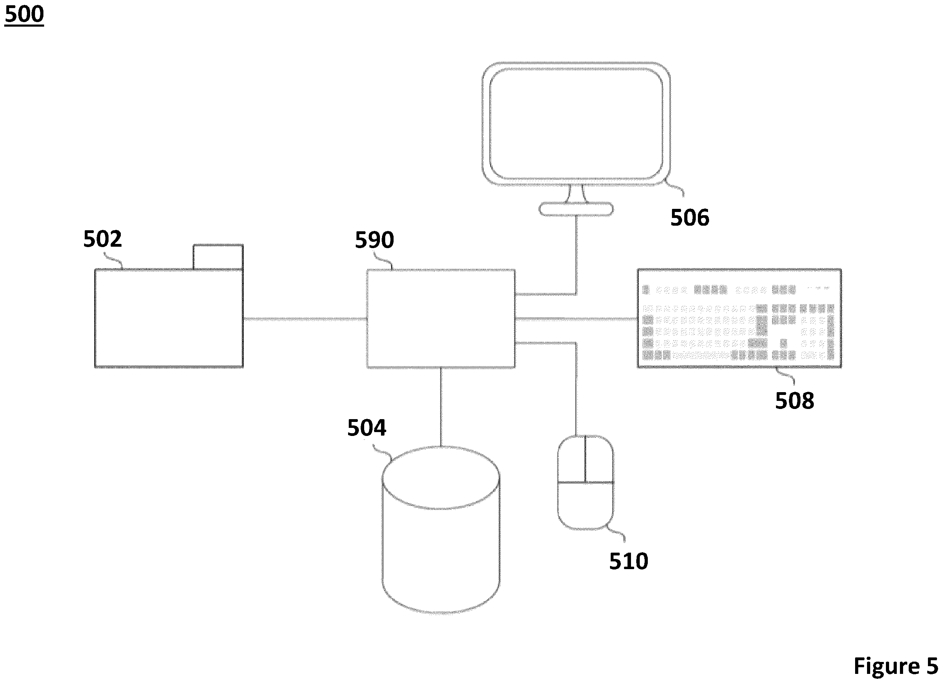

FIG. 5 shows a functional block diagram for one example of a particle analyzer control system, such as an analytics controller 500, for analyzing and displaying biological events. An analytics controller 500 can be configured to implement a variety of processes for controlling graphic display of biological events.

A particle analyzer or sorting system 502 can be configured to acquire biological event data. For example, a flow cytometer can generate flow cytometric event data. The particle analyzer 502 can be configured to provide biological event data to the analytics controller 500. A data communication channel can be included between the particle analyzer or sorting system 502 and the analytics controller 500. The biological event data can be provided to the analytics controller 500 via the data communication channel.

The analytics controller 500 can be configured to receive biological event data from the particle analyzer or sorting system 502. The biological event data received from the particle analyzer or sorting system 502 can include flow cytometric event data. The analytics controller 500 can be configured to provide a graphical display including a first plot of biological event data to a display device 506. The analytics controller 500 can be further configured to render a region of interest as a gate around a population of biological event data shown by the display device 506, overlaid upon the first plot, for example. In some embodiments, the gate can be a logical combination of one or more graphical regions of interest drawn upon a single parameter histogram or bivariate plot. In some embodiments, the display can be used to display particle parameters or saturated detector data.

The analytics controller 500 can be further configured to display the biological event data on the display device 506 within the gate differently from other events in the biological event data outside of the gate. For example, the analytics controller 500 can be configured to render the color of biological event data contained within the gate to be distinct from the color of biological event data outside of the gate. The display device 506 can be implemented as a monitor, a tablet computer, a smartphone, or other electronic device configured to present graphical interfaces.

The analytics controller 500 can be configured to receive a gate selection signal identifying the gate from a first input device. For example, the first input device can be implemented as a mouse 510. The mouse 510 can initiate a gate selection signal to the analytics controller 500 identifying the gate to be displayed on or manipulated via the display device 506 (e.g., by clicking on or in the desired gate when the cursor is positioned there). In some implementations, the first device can be implemented as the keyboard 508 or other means for providing an input signal to the analytics controller 500 such as a touchscreen, a stylus, an optical detector, or a voice recognition system. Some input devices can include multiple inputting functions. In such implementations, the inputting functions can each be considered an input device. For example, as shown in FIG. 5, the mouse 510 can include a right mouse button and a left mouse button, each of which can generate a triggering event.

The triggering event can cause the analytics controller 500 to alter the manner in which the data is displayed, which portions of the data is actually displayed on the display device 506, and/or provide input to further processing such as selection of a population of interest for particle sorting.

In some embodiments, the analytics controller 500 can be configured to detect when gate selection is initiated by the mouse 510. The analytics controller 500 can be further configured to automatically modify plot visualization to facilitate the gating process. The modification can be based on the specific distribution of biological event data received by the analytics controller 500.

The analytics controller 500 can be connected to a storage device 504. The storage device 504 can be configured to receive and store biological event data from the analytics controller 500. The storage device 504 can also be configured to receive and store flow cytometric event data from the analytics controller 500. The storage device 504 can be further configured to allow retrieval of biological event data, such as flow cytometric event data, by the analytics controller 500.

A display device 506 can be configured to receive display data from the analytics controller 500. The display data can comprise plots of biological event data and gates outlining sections of the plots. The display device 506 can be further configured to alter the information presented according to input received from the analytics controller 500 in conjunction with input from the particle analyzer 502, the storage device 504, the keyboard 508, and/or the mouse 510.

In some implementations, the analytics controller 500 can generate a user interface to receive example events for sorting. For example, the user interface can include a control for receiving example events or example images. The example events or images or an example gate can be provided prior to collection of event data for a sample, or based on an initial set of events for a portion of the sample.

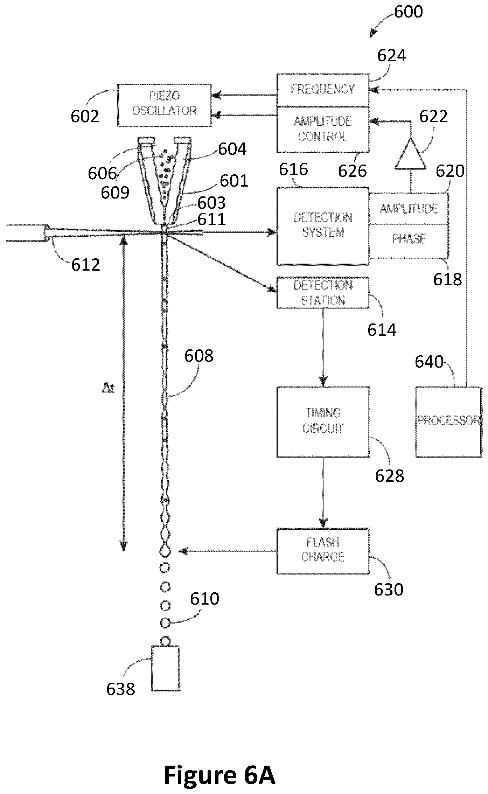

FIG. 6A is a schematic drawing of a particle sorter system 600 (e.g., the particle analyzer or sorting system 502) in accordance with one embodiment presented herein. In some embodiments, the particle sorter system 600 is a cell sorter system. As shown in FIG. 6A, a drop formation transducer 602 (e.g., piezo-oscillator) is coupled to a fluid conduit 601, which can be coupled to, can include, or can be, a nozzle 603. Within the fluid conduit 601, sheath fluid 604 hydrodynamically focuses a sample fluid 606 comprising particles 609 into a moving fluid column 608 (e.g., a stream). Within the moving fluid column 608, particles 609 (e.g., cells) are lined up in single file to cross a monitored area 611 (e.g., where laser-stream intersect), irradiated by an irradiation source 612 (e.g., a laser). Vibration of the drop formation transducer 602 causes moving fluid column 608 to break into a plurality of drops 610, some of which contain particles 609.

In operation, a detection station 614 (e.g., an event detector) identifies when a particle of interest (or cell of interest) crosses the monitored area 611. Detection station 614 feeds into a timing circuit 628, which in turn feeds into a flash charge circuit 630. At a drop break off point, informed by a timed drop delay ($\Delta t$), a flash charge can be applied to the moving fluid column 608 such that a drop of interest carries a charge. The drop of interest can include one or more particles or cells to be sorted. The charged drop can then be sorted by activating deflection plates (not shown) to deflect the drop into a vessel such as a collection tube or a multi-well or microwell sample plate where a well or microwell can be associated with drops of particular interest. As shown in FIG. 6A, the drops can be collected in a drain receptacle 638.

A detection system 616 (e.g., a drop boundary detector) serves to automatically determine the phase of a drop drive signal when a particle of interest passes the monitored area 611. An exemplary drop boundary detector is described in U.S. Pat. No. 7,679,039, which is incorporated herein by reference in its entirety. The detection system 616 allows the instrument to accurately calculate the place of each detected particle in a drop. The detection system 616 can feed into an amplitude signal 620 and/or phase 618 signal, which in turn feeds (via amplifier 622) into an amplitude control circuit 626 and/or frequency control circuit 624. The amplitude control circuit 626 and/or frequency control circuit 624, in turn, controls the drop formation transducer 602. The amplitude control circuit 626 and/or frequency control circuit 624 can be included in a control system.

In some implementations, sort electronics (e.g., the detection system 616, the detection station 614 and a processor 640) can be coupled with a memory configured to store the detected events and a sort decision based thereon. The sort decision can be included in the event data for a particle. In some implementations, the detection system 616 and the detection station 614 can be implemented as a single detection unit or communicatively coupled such that an event measurement can be collected by one of the detection system 616 or the detection station 614 and provided to the non-collecting element.

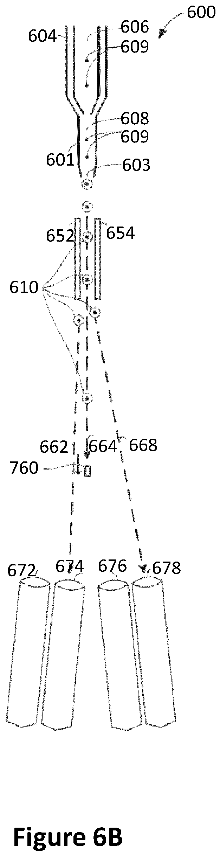

FIG. 6B is a schematic drawing of a particle sorter system, in accordance with one embodiment presented herein. The particle sorter system 600 shown in FIG. 6B, includes deflection plates 652 and 654. A charge can be applied via a stream-charging wire in a barb. This creates a stream of droplets 610 containing particles 610 for analysis. The particles can be illuminated with one or more light sources (e.g., lasers) to generate light scatter and fluorescence information. The information for a particle is analyzed such as by sorting electronics or other detection system (not shown in FIG. 6B). The deflection plates 652 and 654 can be independently controlled to attract or repel the charged droplet to guide the droplet toward a destination collection receptacle (e.g., one of 672, 674, 676, or 678). As shown in FIG. 6B, the deflection plates 652 and 654 can be controlled to direct a particle along a first path 662 toward the receptacle 674 or along a second path 668 toward the receptacle 678. If the particle is not of interest (e.g., does not exhibit scatter or illumination information within a specified sort range), deflection plates may allow the particle to continue along a flow path 664. Such uncharged droplets may pass into a waste receptacle such as via aspirator 670.

The sorting electronics can be included to initiate collection of measurements, receive fluorescence signals for particles, and determine how to adjust the deflection plates to cause sorting of the particles. Example implementations of the embodiment shown in FIG. 6B include the BD FACSAria™ line of flow cytometers commercially provided by Becton, Dickinson and Company (Franklin Lakes, NJ).

## INTEGRATED CIRCUIT DEVICES

Aspects of the present disclosure also include integrated circuit devices programmed to spectrally resolve light from each fluorophore in the sample using a generalized least squares problem as described in the methods detailed above. In some embodiments, integrated circuit devices of interest include a field programmable gate array (FPGA). In other embodiments, integrated circuit devices include an application specific integrated circuit (ASIC). In yet other embodiments, integrated circuit devices include a complex programmable logic device (CPLD). In some embodiments, the subject integrated circuit devices are programmed to determine the overlap between each different fluorophore in the sample and calculate the contribution of each fluorophore to the overlapping fluorescence. In certain embodiments, the integrated circuit is programmed to calculate a spectral unmixing matrix for fluorescence spectra of a plurality of fluorophores having overlapping fluorescence in a sample detected by a light detection system having a plurality of photodetectors. In certain embodiments, integrated circuit devices according to certain embodiments are programmed to calculate a spectral unmixing matrix for fluorescence spectra of a plurality of fluorophores for each cell in a sample. As described in greater detail below, integrated circuit devices may be programmed to estimate the abundance of each fluorophore in the sample. In certain embodiments, the abundance of each fluorophore associated with a target particle may be determined. The integrated circuit may be programmed to identify and classify a target particle based on the abundance of each fluorophore associated with the target particle may. In some instances, integrated circuits are configured to sort the identified or classified particles.

In some embodiments, the integrated circuit device is programmed to determine data signal covariance in each photodetector channel. In some instances, the data signal covariance in each photodetector channel includes an intrinsic sample variability component and a measurement variability component. In some instances, the data signal covariance includes electronic noise in each photodetector channel. In some instances, the data signal covariance includes shot noise in each photodetector channel. In some instances, the data signal covariance varies linearly with the generated data signals. In some instances, the data signal covariance varies quadratically with the generated data signals. In some instances, the data signal covariance is correlated across two or more of the plurality of photodetector channels. In some instances, the integrated circuit device is programmed to calculate the data signal covariance according to:

$$V[y_i] = \sigma_{B,i}^2 + Q_i y_i + (CV_{0,i})^2 y_i^2$$

wherein:

$y_i$ is a measured detector signal;

$\sigma_{B,i}^2$ is a baseline noise component;
$Q_i y_i$ is a Poisson noise component;
$Q_i$ is a Poisson noise coefficient;

$(CV_{0,i})^2 y_i^2$ is a quadratic noise component; and
$CV_{0,i}$ is a quadratic noise coefficient.

In some instances, the integrated circuit device is programmed to calculate the data signal covariance using a covariance matrix. In some instances, the covariance matrix contains non-zero diagonal values. In certain instances, the integrated circuit device is programmed to calculate the covariance with a covariance matrix according to:

$$V[y] = \Sigma^y = \begin{bmatrix} V[y_1] & cov(y_1, y_2) & \cdots & cov(y_1, y_m) \\ cov(y_2, y_1) & V[y_2] & \cdots & cov(y_2, y_m) \\ \vdots & \vdots & \ddots & \vdots \\ cov(y_m, y_1) & cov(y_m, y_2) & \cdots & V[y_m] \end{bmatrix}$$

$$= diag(\sigma_B^2) + diag(Q)diag(y) + \begin{bmatrix} CV_{0,1}^2 y_1^2 & cov(y_1, y_2) & \cdots & cov(y_1, y_m) \\ cov(y_2, y_1) & CV_{0,2}^2 y_2^2 & \cdots & cov(y_2, y_m) \\ \vdots & \vdots & \ddots & \vdots \\ cov(y_m, y_1) & cov(y_m, y_2) & \cdots & CV_{0,m}^2 y_m^2 \end{bmatrix}$$

wherein *diag(x)* indicates generating a diagonal matrix from the column vector *x,* and *cov(a, b)* denotes the covariance between *a* and *b*.

In some instances, the integrated circuit device is programmed to calculate the generalized least squares problem by multiplying by the inverse of the calculated covariance matrix. In some instances, the integrated circuit device is programmed to estimate data signal covariance based on fluorescence intensity across two or more photodetectors of the light detection system. In some instances, the integrated circuit device is programmed to estimate data signal covariance based on fluorescence intensity across each of the photodetection channels. In some embodiments, the integrated circuit device is programmed to calculate the generalized least squares problem according to:

$$\hat{f} = \arg\min_{f}(Xf - y)^T G(Xf - y)$$

wherein:

$G = (\Sigma^y)^{-1}$ is a weight matrix;
$\Sigma^y$ is a covariance matrix;
$X$ is a spectral matrix (spillover matrix);
$y$ is measured detector values from the plurality of photodetectors of the light detection system for each cell;
$f$ is true fluorophore abundances for each cell; and
$\hat{f}$ is estimated (unmixed) fluorophore abundances for each cell.

In some instances, the integrated circuit device is programmed to estimate a covariance matrix a *priori.* In certain instances, the integrated circuit device is programmed to apply the generalized least squares problem in real time (e.g., using an integrated circuit such as a field programmable gate array). In some instances, an a *priori* noise model is used to estimate each event covariance (e.g., calculate each event covariance matrix in real time). In certain instances, the a *priori* noise model uses only data collected for each specific event. In some instances, the covariance matrix includes estimations from an entire collected dataset. In some instances, the covariance matrix is generated through iterative optimization methods which empirically tune the covariance matrix to minimize the variance of the unmixed data.

In some embodiments, the integrated circuit device is programmed to determine data signal covariance by estimating by iterative optimization with a covariance matrix that minimizes variance of unmixed data signals. In certain instances, the integrated circuit device is programmed to calculate the generalized least squares problem by Cholesky decomposition of the covariance matrix. In certain instances, the generalized least squares problem is characterized by computing the Cholesky decomposition of the covariance matrix $\Sigma^y = CC^T$ and solving triangular systems to generate transformed inputs for an ordinary least squares algorithm according to:

Solving $C\bar{y} = y$ to generate $\bar{y}$, a decorrelated and whitened version of the data vector *y*.
Solving $C\bar{X} = X$ to generate $\bar{X}$, the corresponding transformed version of the spectral matrix *X*.
The transformed system $\bar{X}f = \bar{y}$ is solved by ordinary least squares, for example by solving the so-called normal equations $\bar{X}^T\bar{X}\hat{f} = \bar{X}^T\bar{y}$.

In some embodiments, the integrated circuit device is programmed to find the least-squares solution of the generalized least squares problem. In some instances, the integrated circuit device is programmed to find the least-squares solution of the generalized least squares problem by one or more of matrix decomposition, matrix factorization, QR factorization, Cholesky decomposition, singular value decomposition, LDL decomposition, and forward- and back-substitution. In some instances, the integrated circuit device is programmed to find the least-squares solution of the generalized least squares problem by solving the so-called normal equations via Cholesky or LDL decomposition, such as by Cholesky or LDL decomposition according to:

$$X^TGX\hat{f} = X^TGy$$

where $y$ is measured detector values from the plurality of photodetectors of the light detection system for each cell; $X$ is spillover; and

$$G \text{ is } \begin{bmatrix} G_{1,1} & G_{1,2} & \cdots & G_{1,m} \\ G_{2,1} & G_{2,2} & \cdots & G_{2,m} \\ \vdots & \vdots & \ddots & \vdots \\ G_{m,1} & G_{m,2} & \cdots & G_{m,m} \end{bmatrix}$$

In some instances, the integrated circuit device is programmed to find the least-squares solution of the generalized least squares problem by Cholesky or LDL decomposition according to:

$$X^TGX\hat{f} = X^TGy$$

$A\hat{f} = B$
$LDL^T\hat{f} = B$      LDL decomposition
$Lz = B \text{ where } z = DL^T\hat{f}$      Lower-triangular matrix solution
$Dx = z \text{ where } x = L_T\hat{f}$      Diagonal matrix solution
$L_T\hat{f} = x, \text{ solve for } \hat{f}$      Upper-triangular matrix solution

In some embodiments, the integrated circuit device is programmed to find the least-squares solution of the generalized least squares problem by matrix decomposition. In some embodiments, the integrated circuit device is programmed to find the least-squares solution of the generalized least squares problem by matrix factorization. In some instances, the integrated circuit device is programmed to minimize the least-squares solution of the generalized least squares problem by QR factorization. In some instances, the integrated circuit device is programmed to calculate the generalized least squares problem using transformed $\overline{X}$ and $\overline{y}$ (as calculated via Cholesky decomposition of the covariance matrix described above) using QR factorization according to:

$\overline{X}^TXf = \overline{X}^Ty$      Normal equations for transformed GLS problem
$QR = \overline{X}$      QR decomposition of transformed $X$
$(QR)^TQR\hat{f} = (QR)^T\overline{y}$      Substitution
$R^TQ^TQR\hat{f} = R^TQ^T\overline{y}$      Expand transpose of $QR$
$R^TR\hat{f} = R^TQ^T\overline{y}Q^TQ$      cancels due to orthogonality
$R\hat{f} = Q^T\overline{y}.$      Triangular solve for $\hat{f}$

In some embodiments, the integrated circuit device is programmed to minimize the least-squares solution of the generalized least squares problem by singular value decomposition. In some instances, the singular value decomposition is the matrix that is the product $\overline{X} = U\Sigma V^T$ where $U$ and $V$ are orthogonal matrices and $\Sigma$ is a diagonal matrix containing singular values of $\overline{X}$. In some instances, the integrated circuit device is programmed to calculate the generalized least squares problem using singular value decomposition according to:

$$z = U^T\overline{y}$$

$$\Sigma W = z$$

$$\hat{f} = Vw,$$

where $U$ and $V$ are orthogonal matrices and $\Sigma$ is a diagonal matrix containing singular values of $\overline{X}$. In some embodiments, the integrated circuit device is programmed to find the least-squares solution of the generalized least squares problem by LDL decomposition. In certain embodiments, the integrated circuit device is programmed to find the least-squares solution of the generalized least squares problem by forward- and back-substitution.

In some embodiments, the integrated circuit devices are programmed to estimate an abundance of one or more of the fluorophores in the sample based on the calculated spectral unmixing matrix. In certain instances, the integrated circuit device is programmed to estimate the abundance of one or more fluorophores on a particle in the sample. In some embodiments, the integrated circuit devices are programmed to identify a particle in the sample based on the estimated abundance of each fluorophore on the particle. In certain instances, the integrated circuit devices are programmed to sort the identified particles in the sample.

In some embodiments, integrated circuits of interest are programmed to calculate the abundance of one or more fluorophores in the sample from the spectrally resolved light from each fluorophore. In certain instances, the abundance of fluorophores associated with (e.g., chemically associated (i.e., covalently, ionically) or physically associated) a target particle is calculated from the spectrally resolved light from each fluorophore associated with the particle. For instance, in one example the integrated circuit is programmed to calculate the relative abundance of each fluorophore associated with a target particle from the spectrally resolved light from each fluorophore. In another example, the integrated circuit is programmed to calculate the absolute abundance of each fluorophore associated with the target particle from the spectrally resolved light from each fluorophore.

In certain embodiments, the integrated circuit is programmed to identify or classify a particle based on the relative abundance of each fluorophore determined to be associated with the particle. In these embodiments, the integrated circuits may be programmed to identify or classify the particle by any convenient protocol such as by: comparing the relative or absolute abundance of each fluorophore associated with a particle with a control sample having particles of known identity; or by conducting spectroscopic or other assay analysis of a population of particles (e.g., cells) having the calculated relative or absolute abundance of associated fluorophores.

## KITS

Aspects of the present disclosure further include kits, where kits include one or more of the integrated circuits described herein. In some embodiments, kits may further include programming for the subject systems, such as in the form of a computer readable medium (e.g., flash drive, USB storage, compact disk, DVD, Blu-ray disk, etc.) or instructions for downloading the programming from an internet web protocol or cloud server. Kits may further include instructions for practicing the subject methods. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, and the like. Yet another form of these instructions is a computer readable medium, e.g., diskette, compact disk (CD), portable flash drive, and the like, on which the information has been recorded. Yet another form of these instructions that may be present is a website address which may be used via the internet to access the information at a removed site.

## UTILITY

The subject systems, methods and computer systems find use in a variety of applications where it is desirable to analyze and sort particle components in a sample in a fluid medium, such as a biological sample. In some embodiments, the systems and methods described herein find use in flow cytometry characterization of biological samples labelled with fluorescent tags. In other embodiments, the systems and methods find use in spectroscopy of emitted light. In addition, the subject systems and methods find use in increasing the obtainable signal from light collected from a sample (e.g., in a flow stream). In certain instances, the present disclosure finds use in enhancing measurement of light collected from a sample that is irradiated in a flow stream in a flow cytometer. Embodiments of the present disclosure find use where it is desirable to provide a flow cytometer with improved cell sorting accuracy, enhanced particle collection, particle charging efficiency, more accurate particle charging and enhanced particle deflection during cell sorting.

Embodiments of the present disclosure also find use in applications where cells prepared from a biological sample may be desired for research, laboratory testing or for use in therapy. In some embodiments, the subject methods and devices may facilitate obtaining individual cells prepared from a target fluidic or tissue biological sample. For example, the subject methods and systems facilitate obtaining cells from fluidic or tissue samples to be used as a research or diagnostic specimen for diseases such as cancer. Likewise, the subject methods and systems may facilitate obtaining cells from fluidic or tissue samples to be used in therapy. Methods and devices of the present disclosure allow for separating and collecting cells from a biological sample (e.g., organ, tissue, tissue fragment, fluid) with enhanced efficiency and low cost as compared to traditional flow cytometry systems.

**EXPERIMENTAL**

A description of spectral unmixing and covariance modeling according to certain embodiments of the present disclosure is set forth below. The scope of the present disclosure is not intended to be limited to the exemplary embodiments shown and described below.

*1 Definitions and Terminology*

1.1 Variables

- $m$: Number of detectors

- $n$: Number of fluorophores

- $y$: Vector [$m \times 1$] of measured intensity per detector

- $f$: Vector [$n \times 1$] of fluorophore abundances (unmixed)

- $X$: Spectral spillover matrix [$m \times n$]

- $x_k$: $k$-th column of $X$ (i.e., the SOVs corresponding to fluorophore $k$)

- $X^\dagger$: Pseudoinverse of spillover matrix (unmixing matrix) [$n \times m$]

- $x_k^\dagger$ : $k$-th row of $X^\dagger$ (i.e., the unmixing coefficiencts corresponding to fluorophore $k$)

- $\varepsilon_y$: Vector [$m \times 1$] of noise (error) per detector

- $W$: Weight matrix for WLS (diagonal)

- $G$: Weight matrix for GLS

GLS

We use a linear mixture model to describe measured signals in a flow cytometer:

$$y = Xf + \epsilon_y$$

where $y$ is the vector of measured detector values, $X$ is the spectral ("spillover") matrix that describes how signals map from fluorophores to detectors, $f$ is the vector of fluorophore abundances, and $\varepsilon_y$ is the noise vector representing zero-mean random error for each observation (detector). We know $y$ and $X$ and want to solve for $f$.

Our objective in spectral unmixing is to estimate fluorophore abundances $f$ given observed signals y and spectral matrix $X$. The least-squares family of solutions treat this as an optimization problem, where the best estimate of $f$ (denoted $\hat{f}$) is the one that minimizes some objective function that quantifies the euclidean distance between the observed values of $y$ and the predicted values calculated from the linear mixture model. In the case of ordinary least squares (OLS), this difference is defined as the 2-norm between the measured and predicted values of the dependent variable:

$$\underset{\hat{f}}{\operatorname{argmin}} \parallel \left( X\hat{f} - y \right) \parallel^2$$

There are a number of mathematically equivalent solutions for this problem, with varying tradeoffs in terms of computational complexity and numerical stability. A common approach is to solve the so-called "normal equations":

$$X^T X \hat{f} = X^T y$$

The Gauss-Markov Theorem states that this OLS solution is the best linear unbiased estimator (BLUE) if the following assumptions are true: 1. The error vector $\varepsilon$ is zero-mean, 2. The error is homoscedastic (i.e., all detectors have the same variance), and 3. The errors are uncorrelated between detectors (i.e., the detector covariance matrix is diagonal).

In the case of fluorescence measurements in a flow cytometer, measured data satisfies the first requirement (due largely to the use of baseline restoration in signal processing) but does not necessarily satisfy the second and third requirements. Weighted least squares (WLS) allows us to unmix optimally even in the presence of heteroscedasticity, and generalized least squares (GLS) allows us to unmix optimally in the presence of both heteroscedasticity and correlated noise (nonzero detector covariance). Both WLS and GLS can be thought of as transformations that modify the data to satisfy the three assumptions of the Gauss-Markov theorem so that OLS can be applied to the transformed problem.

Generalized least squares takes a similar form to the WLS problem, but instead of using a diagonal weight matrix, the inverse of the full covariance matrix of the raw detector signal $\Sigma^y$ is used. This allows for accounting for nonzero detector covariances arising from correlated noise sources.

$$\underset{\hat{f}}{\mathrm{argmin}} \left(X\hat{f} - y\right)^T G\left(X\hat{f} - y\right)$$

where $G = (\Sigma^y)^{-1}$. The solution takes the same general approach as in WLS, but now with a more complex non-diagonal weight matrix $G$:

$$X^T G X \hat{f} = X^T G y$$

Note that this is equivalent to "whitening" and "decorrelating" the noise and reframing the GLS problem as the OLS problem. We can go even further and pre-whiten $X$ and $y$ with $G^{1/2}$ with $\overline{X} = G^{1/2}X$ and $\overline{y} = G^{1/2}y$, leaving us with our original OLS problem on the transformed matrices: $\overline{X}^T \overline{X} \hat{f} = \overline{X}^T \overline{y}$.

This may be solved using the following matrix decomposition approach. The Cholesky decomposition (technically, the LDL decomposition, a special case of Cholesky) allows us to express $X^T G X$ as $LDL^T$, where $L$ is unit lower-triangular (meaning it has ones on the diagonal) and $D$ is diagonal. Letting $A = X^T G X$ and $B = X^T G y$, we can rewrite the WLS problem as $A\hat{f} = B$ and proceed as follows:

| | |
|---|---|
| Transform LHS | $A = X^T G X$ |
| Transform RHS | $B = X^T G y$ |
| Normal equation | $A\hat{f} = B$ |
| Cholesky decomp | $LDL^T\hat{f} = B$ |
| Lower-triangular solve | $Lz = B$ for $z$ |
| Diagonal solve | $Du = z$ for $u$ |
| Upper-triangular solve | $L^T\hat{f} = u$ for $\hat{f}$ |

The final equation will provide us with our GLS solution. Note that we use two intermediate vectors $z$ and $u$ along the way to reduce the problem to entirely triangular and diagonal solves.

Note that this technique includes calculating $(\Sigma^y)^{-1}$. To avoid this, we can instead do the following:

| | |
|---|---|
| Cholesky decomp | $\Sigma^y = CC^T$ |
| Lower-triangular solve | $CX = X$ for $\overline{X}$ |
| Lower-triangular solve | $C\overline{y} = y$ for $\overline{y}$ |
| Solve transformed system | $\overline{X}^T \overline{X} \hat{f} = \overline{X}^T \overline{y}$ for $\hat{f}$ |

Here, lower-triangular solves are used to form the transformed (whitened and decorrelated) $\overline{X}$ and $\overline{y}$, and can now solve the transformed OLS problem using any approach.

Noise Model

Definitions

- $\Sigma^y$: Variance-covariance matrix of raw detector intensity
- $\Sigma^{y0}$: Variance-covariance matrix of raw detector intensity in the absence of biological spread (biological "point source")
- $V_i^y$ : Raw/detector-space variance of detector $i$
- $\Sigma^f$: Variance-covariance matrix of unmixed fluorophore abundance
- $V_k^f$ : Unmixed/marker-space variance of fluorophore $k$
- Baseline noise:
    - $\Sigma_B^y$ : Matrix [$m \times m$] of baseline variance (constant with intensity, diagonal/uncorrelated)
    - $B$: Vector [$m \times 1$] of detector baseline variance $\sigma_{Bi}^2$
- Poisson noise:
    - $\Sigma_Q^y$ : Matrix [$m \times m$] of Poisson/photoelectron variance (linear with intensity, diagonal/uncorrelated)
    - $Q$: Vector [$m \times 1$] of statistical photoelectron scaling factors per channel
- CV noise:
    - $\Sigma_{CV}^y$ : Matrix [$m \times m$] of multiplicative measurement/system variance (quadratic with intensity, correlated)
    - $CV$: Vector [$m \times 1$] of CV per channel
    - Corr: Correlation matrix [$m \times m$] of detector CV noise
    - $\rho_{ij}$: Correlation coefficient between channels $i$ and $j$
- Biological (intrinsic) spread:
    - $\Sigma_{\text{bio}}^y$ : Matrix [$m \times m$] of raw variance/covariance arising from underlying biological spread
    - $\mathbb{V}[f_k]$ : True variance of the underlying expression of fluor $k$.

Single-Detector Noise Model

Variance in flow cytometry intensity signals is described with the following noise model (shown here for a single detector $i$):

$$\mathbb{V}[y_i] = \sigma_{B,i}^2 + Q_i y_i + \left(CV_{0,i}\right)^2 y_i^2$$

This expression contains a constant, a linear, and a quadratic term:

1. $\sigma_{B,i}^2$ is independent of intensity, usually called the baseline or background noise,
2. $Q_i y_i$ is linear in intensity, usually called the Poisson or photoelectron noise (here the coefficient $Q_i$ is just the conversion factor from measurement units to statistical photoelectrons, SPE, for detector $i$), and
3. $\left(CV_{0,i}\right)^2 y_i^2$ is quadratic in intensity, where $CV_{0,i}$ describes time-varying sources of measurement variance such as fluidic/laser fluctuations.

Baseline and Poisson errors between detectors are uncorrelated, but the same assumption cannot be made about $CV_0$. Since detectors are grouped by laser line, all detectors on a given laser line sample the same point in time, meaning that time-dependent errors will be correlated to some extent among detectors on the same laser line. Moreover, other random variations in fluidics (affecting particle position) or laser intensity/position may be correlated or anticorrelated across laser lines, while chromatic aberration effects in the collection optics may lead different detectors on the same laser line to have-less-then perfect correlation.

Therefore (where the operator diag($z$) converts the column vector $z$ to a diagonal matrix):

$$\mathbb{V}[y] = \begin{bmatrix} \mathbb{V}[y_1] & \mathrm{cov}(y_1,y_2) & \cdots & \mathrm{cov}(y_1,y_m) \\ \mathrm{cov}(y_2,y_1) & \mathbb{V}[y_2] & \cdots & \mathrm{cov}(y_2,y_m) \\ \vdots & \vdots & \ddots & \vdots \\ \mathrm{cov}(y_m,y_1) & \mathrm{cov}(y_m,y_2) & \cdots & \mathbb{V}[y_m] \end{bmatrix}$$

$$= \mathrm{diag}(\sigma_B^2) + \mathrm{diag}(Q)\mathrm{diag}(y) + \begin{bmatrix} CV_{0,1}^2 y_1^2 & \mathrm{cov}(y_1,y_2) & \cdots & \mathrm{cov}(y_1,y_m) \\ \mathrm{cov}(y_2,y_1) & CV_{0,2}^2 y_2^2 & \cdots & \mathrm{cov}(y_2,y_m) \\ \vdots & \vdots & \ddots & \vdots \\ \mathrm{cov}(y_m,y_1) & \mathrm{cov}(y_m,y_2) & \cdots & CV_{0,p}^2 y_m^2 \end{bmatrix}$$

Detector Covariance in the Absence of Biological Spread

The total raw variance-covariance matrix in the absence of biological spread $\Sigma^{y0}$ is given by the following expression:

$$\Sigma^{y0} = \Sigma_B^y + \Sigma_Q^y + \Sigma_{CV}^y$$

$$= \mathrm{diag}(B) + \mathrm{diag}(Q)\mathrm{diag}(y) + \mathrm{diag}(y)\mathrm{diag}(CV)\mathrm{Corr}^y\mathrm{diag}(CV)\mathrm{diag}(y)$$

$$= \begin{bmatrix} \sigma_{B0}^2 & & \\ & \sigma_{B1}^2 & \\ & & \sigma_{Bm}^2 \end{bmatrix} + \begin{bmatrix} Q_0 y_0 & & \\ & Q_1 y_1 & \\ & & Q_m y_m \end{bmatrix}$$

$$+ \begin{bmatrix} CV_0^2 y_0^2 & CV_0 CV_1 y_0 y_1 \rho_{01} & CV_0 CV_m y_0 y_m \rho_{0m} \\ CV_0 CV_1 y_0 y_1 \rho_{01} & CV_1^2 y_1^2 & CV_1 CV_m y_1 y_m \rho_{1m} \\ CV_0 CV_m y_0 y_m \rho_{0m} & CV_1 CV_m y_1 y_m \rho_{1m} & CV_m^2 y_m^2 \end{bmatrix}$$

$$= \begin{bmatrix} \sigma_{B0}^2 & & \\ & \sigma_{B1}^2 & \\ & & \sigma_{Bm}^2 \end{bmatrix} + \begin{bmatrix} Q_0 y_0 & & \\ & Q_1 y_1 & \\ & & Q_m y_m \end{bmatrix}$$

$$+ \begin{bmatrix} y_0 & & \\ & y_1 & \\ & & y_m \end{bmatrix} \begin{bmatrix} CV_0 & & \\ & CV_1 & \\ & & CV_m \end{bmatrix} \begin{bmatrix} 1 & \rho_{01} & \rho_{0m} \\ \rho_{01} & 1 & \rho_{1m} \\ \rho_{0m} & \rho_{1m} & 1 \end{bmatrix} \begin{bmatrix} CV_0 & & \\ & CV_1 & \\ & & CV_m \end{bmatrix} \begin{bmatrix} y_0 & & \\ & y_1 & \\ & & y_m \end{bmatrix}$$

Notwithstanding the appended claims, the disclosure is also defined by the following clauses:
1. A method comprising:

   detecting light with a light detection system from a sample comprising a plurality of fluorophores having overlapping fluorescence spectra;
   spectrally resolving light from each fluorophore in the sample using a generalized least squares algorithm.

2. The method according to clause 1, wherein light is detected by the light detection system in a plurality of photodetector channels.
3. The method according to any one of clauses 1-2, wherein the light detection system comprises a plurality of photodetectors.
4. The method according to any one of clauses 2-3, wherein the method comprises generating data signals in each of the photodetector channels in response to the detected light.
5. The method according to clause 4, wherein the method comprises determining data signal covariance in each photodetector channel.

6. The method according to clause 5, wherein the data signal covariance in each photodetector channel comprises:

an intrinsic sample variability component; and
a measurement variability component.

7. The method according to any one of clauses 5-6, wherein the data signal covariance comprises electronic noise in each photodetector channel.

8. The method according to any one of clauses 5-6, wherein the data signal covariance comprises shot noise in each photodetector channel.

9. The method according to any one of clauses 5-8, wherein the data signal covariance varies linearly with the generated data signals.

10. The method according to any one of clauses 5-8, wherein the data signal covariance varies quadratically with the generated data signals.

11. The method according to any one of clauses 5-10, wherein the data signal covariance is correlated across two or more of the plurality of photodetector channels.

12. The method according to any one of clauses 5-11, wherein the data signal covariance is calculated according to:

$$V[y_i] = \sigma_{B,i}^2 + Q_i y_i + (CV_{0,i})^2 y_i^2$$

wherein:

$y_i$ is a measured detector signal;

$\sigma_{B,i}^2$ is a baseline noise component;
$Q_i y_i$ is a Poisson noise component;
$Q_i$ is a Poisson noise component;
$(CV_{0,i})^2 y_i^2$ is a quadratic noise component; and
$CV_{0,i}$ is a quadratic noise coefficient.

13. The method according to any one of clauses 5-12, wherein the method comprises calculating the data signal covariance using a covariance matrix.

14. The method according to clause 13, wherein the covariance matrix contains non-zero diagonal values.

15. The method according to any one of clauses 12-13, wherein the covariance is calculated with a covariance matrix according to:

$$V[y] = \Sigma^y = \begin{bmatrix} V[y_1] & cov(y_1, y_2) & \cdots & cov(y_1, y_m) \\ cov(y_2, y_1) & V[y_2] & \cdots & cov(y_2, y_m) \\ \vdots & \vdots & \ddots & \vdots \\ cov(y_m, y_1) & cov(y_m, y_2) & \cdots & V[y_m] \end{bmatrix}$$

$$= diag(\sigma_B^2) + diag(Q)diag(y) + \begin{bmatrix} CV_{0,1}^2 y_1^2 & cov(y_1, y_2) & \cdots & cov(y_1, y_m) \\ cov(y_2, y_1) & CV_{0,2}^2 y_2^2 & \cdots & cov(y_2, y_m) \\ \vdots & \vdots & \ddots & \vdots \\ cov(y_m, y_1) & cov(y_m, y_2) & \cdots & CV_{0,m}^2 y_m^2 \end{bmatrix}$$

wherein *diag(x)* indicates generating a diagonal matrix from the column vector *x*, and *cov(a, b)* denotes the covariance between *a* and *b*.

16. The method according to any one of clauses 13-15, wherein the generalized least squares algorithm comprises solving a generalized least squares problem by multiplying by the inverse of the calculated covariance matrix.

17. The method according to any one of clauses 5-16, wherein the method comprises estimating data signal covariance based on fluorescence intensity across two or more photodetectors of the light detection system.

18. The method according to clause 17, wherein the method comprises estimating data signal covariance based on fluorescence intensity across each of the photodetection channels.

19. The method according to any one of clauses 1-18, wherein solving the generalized least squares problem comprises calculating according to:

$$\hat{f} = \arg\min_{f}(Xf - y)^T G(Xf - y)$$

wherein:

$G = (\Sigma^y)^{-1}$ is a weight matrix;
$\Sigma^y$ is a covariance matrix;
$X$ is a spectral matrix (spillover matrix);
$y$ is measured detector values from the plurality of photodetectors of the light detection system for each cell;
$f$ is true fluorophore abundances for each cell; and
$\hat{f}$ is estimated (unmixed) fluorophore abundances for each cell.

20. The method according to any one of clauses 1-19, wherein the method further comprises generating *a priori* an estimated covariance matrix.

21. The method according to any one of clauses 13-20, wherein data signal covariance is determined by estimating by iterative optimization with a covariance matrix that minimizes variance of unmixed data signals.

22. The method according to any one of clauses 13-20, wherein solving the generalized least squares problem comprises Cholesky decomposition of the covariance matrix.

23. The method according to any one of clauses 1-22, wherein the method comprises finding the least-squares solution of the generalized least squares problem.

24. The method according to any one of clauses 1-23, wherein the method comprises minimizing the least-squares solution of the generalized least squares problem by one or more of matrix decomposition, matrix factorization, QR factorization, Cholesky decomposition, singular value decomposition, LDL decomposition, and forward- and back-substitution.

25. The method according to clause 24, wherein the method comprises finding the least-squares solution of the generalized least squares problem by Cholesky or LDL decomposition.

26. The method according to clause 25, wherein the method comprises minimizing the least-squares solution of the generalized least squares problem by Cholesky decomposition according to:

$$X^T G X \hat{f} = X^T G y$$

wherein:

$y$ is measured detector values from the plurality of photodetectors of the light detection system for each cell;
$X$ is spillover; and

$$G \text{ is } \begin{bmatrix} G_{1,1} & G_{1,2} & \cdots & G_{1,m} \\ G_{2,1} & G_{2,2} & \cdots & G_{2,m} \\ \vdots & \vdots & \ddots & \vdots \\ G_{m,1} & G_{m,2} & \cdots & G_{m,m} \end{bmatrix}$$

27. The method according to any one of clauses 25-26, wherein the method comprises finding the least-squares solution of the generalized least squares problem by Cholesky or LDL decomposition according to:

$$X^T G X \hat{f} = X^T G y$$

$A\hat{f} = B$
$LDL^T\hat{f} = B$                      LDL decomposition
$Lz = B$ where $z = DL^T\hat{f}$         Lower-triangular matrix solution
$Dx = z$ where $x = L_T\hat{f}$          Diagonal matrix solution
$L_T\hat{f} = x$, solve for $\hat{f}$    Upper-triangular matrix solution

28. The method according to clause 23, wherein the method comprises finding the least-squares solution of the generalized least squares problem by matrix decomposition.

29. The method according to clause 23, wherein the method comprises finding the least-squares solution of the generalized least squares problem by matrix factorization.

30. The method according to clause 23, wherein the method comprises finding the least-squares solution of the generalized least squares problem by QR factorization.

31. The method according to clause 30, wherein the generalized least squares problem is calculated using transformed $\overline{X}$ and $\overline{y}$ using QR factorization according to:

$$\overline{X}^T\overline{X}f = \overline{X}^T\overline{y} \qquad \text{Normal equations for transformed GLS problem}$$
$$QR = \overline{X} \qquad \text{QR decomposition of transformed } X$$
$$(QR)^TQR\hat{f} = (QR)^T\overline{y} \qquad \text{Substitution}$$

$$R^TQ^TQR\hat{f} = R^TQ^T\overline{y} \qquad \text{Expand transpose of } QR$$
$$R^TR\hat{f} = R^TQ^T\overline{y}Q^TQ \text{ cancels} \qquad \text{due to orthogonality}$$
$$R\hat{f} = Q^T\overline{y}. \qquad \text{Triangular solve for } \hat{f}.$$

32. The method according to clause 23, wherein the method comprises finding the least-squares solution of the generalized least squares problem by singular value decomposition.

33. The method according to clause 32, wherein the generalized least squares problem is calculated using singular value decomposition according to:

$$z = U^T\overline{y}$$

$$\Sigma w = z$$

$$\hat{f} = Vw,$$

wherein $U$ and $V$ are orthogonal matrices and $\Sigma$ is a diagonal matrix containing singular values of $\overline{X}$.

34. The method according to clause 23, wherein the method comprises finding the least-squares solution of the generalized least squares problem by LDL decomposition.

35. The method according to clause 23, wherein the method comprises finding the least-squares solution of the generalized least squares problem by forward- and back-substitution.

36. The method according to any one of clauses 1-35, wherein the method comprises spectrally resolving light from each fluorophore in real time.

37. The method according to clause 36, wherein light from each fluorophore is spectrally resolved in real time with an integrated circuit.

38. The method according to clause 37, wherein the integrated circuit comprises a field programmable gate array (FPGA).

39. The method according to any one of clauses 1-38, wherein the fluorescence spectra of each fluorophore overlaps with the fluorescence spectra of at least one other fluorophore in the sample.

40. The method according to clause 39, wherein the fluorescence spectra of each fluorophore overlaps with the fluorescence spectra of at least one other fluorophore in the sample by 10 nm or more.

41. The method according to clause 39, wherein the fluorescence spectra of each fluorophore overlaps with the fluorescence spectra of at least one other fluorophore in the sample by 25 nm or more.

42. The method according to clause 39, wherein the fluorescence spectra of at least one fluorophore in the sample overlaps with the fluorescence spectra of two different fluorophores in the sample.

43. The method according to clause 42, wherein the fluorescence spectra of at least one fluorophore in the sample overlaps with the fluorescence spectra of two different fluorophores in the sample by 10 nm or more.

44. The method according to clause 42, wherein the fluorescence spectra of at least one fluorophore in the sample overlaps with the fluorescence spectra of two different fluorophores in the sample by 25 nm or more.

45. The method according to any one of clauses 1-44, further comprising irradiating the sample with a light source.

46. The method according to clause 45, wherein the light source comprises a laser.

47. The method according to 46, wherein the light source comprises a plurality of lasers.

48. A system comprising:

a light source configured to irradiate a sample comprising a plurality of fluorophores having overlapping fluorescence spectra;

a light detection system comprising a plurality of photodetectors; and

a processor comprising memory operably coupled to the processor wherein the memory comprises instructions stored thereon, which when executed by the processor, cause the processor to spectrally resolve light from each fluorophore in the sample using a generalized least squares algorithm.

49. The system according to clause 48, wherein the system is configured to detect light by the light detection system in a plurality of photodetector channels.

50. The system according to any one of clauses 48-49, wherein the light detection system comprises a plurality of photodetectors.

51. The system according to clause 50, wherein the photodetectors comprise one or more photomultiplier tubes.

52. The system according to any one of clauses 48-51, wherein the light detection system comprises a photodetector array.

53. The system according to clause 52, wherein the photodetector array comprises photodiodes.

54. The system according to clause 53, wherein the photodetector array comprises charge coupled devices.

55. The system according to any one of clauses 48-54, wherein the memory comprises instructions stored thereon, which when executed by the processor, cause the processor to determine data signal covariance in each photodetector channel.

56. The system according to clause 55, wherein the data signal covariance in each photodetector channel comprises:

an intrinsic sample variability component; and

a measurement variability component.

57. The system according to any one of clauses 55-56, wherein the data signal covariance comprises electronic noise in each photodetector channel.

58. The system according to any one of clauses 55-56, wherein the data signal covariance comprises shot noise in each photodetector channel.

59. The system according to any one of clauses 55-58, wherein the data signal covariance varies linearly with the generated data signals.

60. The system according to any one of clauses 55-58, wherein the data signal covariance varies quadratically with the generated data signals.

61. The system according to any one of clauses 55-60, wherein the data signal covariance is correlated across two or more of the plurality of photodetector channels.

62. The system according to any one of clauses 48-61, wherein the memory comprises instructions stored thereon, which when executed by the processor, cause the processor to calculate covariance according to:

$$V[y_i] = \sigma_{B,i}^2 + Q_i y_i + (CV_{0,i})^2 y_i^2$$

wherein:

$y_i$ is a measured detector signal;

$\sigma_{B,i}^2$ is a baseline noise component;

$Q_i y_i$ is a Poisson noise component;

$Q_i$ is a Poisson noise coefficient;

$(CV_{0,i})^2 y_i^2$ is a quadratic noise component; and

$CV_{0,i}$ is a quadratic noise coefficient.

63. The system according to any one of clauses 48-62, wherein the memory comprises instructions stored thereon, which when executed by the processor, cause the processor to calculate the data signal covariance using a covariance matrix.

64. The system according to clause 63, wherein the covariance matrix contains non-zero diagonal values.

65. The system according to any one of clauses 63-64, wherein the memory comprises instructions stored thereon, which when executed by the processor, cause the processor to calculate covariance with a covariance matrix according to:

$$V[y] = \Sigma^y = \begin{bmatrix} V[y_1] & cov(y_1, y_2) & \cdots & cov(y_1, y_m) \\ cov(y_2, y_1) & V[y_2] & \cdots & cov(y_2, y_m) \\ \vdots & \vdots & \ddots & \vdots \\ cov(y_m, y_1) & cov(y_m, y_2) & \cdots & V[y_m] \end{bmatrix}$$

$$= diag(\sigma_B^2) + diag(Q)diag(y) + \begin{bmatrix} CV_{0,1}^2 y_1^2 & cov(y_1, y_2) & \cdots & cov(y_1, y_m) \\ cov(y_2, y_1) & CV_{0,2}^2 y_2^2 & \cdots & cov(y_2, y_m) \\ \vdots & \vdots & \ddots & \vdots \\ cov(y_m, y_1) & cov(y_m, y_2) & \cdots & CV_{0,m}^2 y_m^2 \end{bmatrix}$$

wherein *diag(x)* indicates generating a diagonal matrix from the column vector *x,* and *cov(a, b)* denotes the covariance between *a* and *b*.

66. The system according to any one of clauses 63-65, wherein the memory comprises instructions stored thereon, which when executed by the processor, cause the processor to calculate the generalized least squares problem by multiplying the data signals by the inverse of the calculated covariance matrix.

67. The system according to any one of clauses 55-66, wherein the memory comprises instructions stored thereon, which when executed by the processor, cause the processor to estimate data signal covariance based on fluorescence intensity across two or more photodetectors of the light detection system.

68. The system according to any one of clauses 55-66, wherein the memory comprises instructions stored thereon, which when executed by the processor, cause the processor to estimate data signal covariance based on fluorescence intensity across each of the photodetection channels.

69. The system according to any one of clauses 48-68, wherein the memory comprises instructions stored thereon, which when executed by the processor, cause the processor to calculate the generalized least squares problem according to:

$$\hat{f} = \arg\min_f (Xf - y)^T G(Xf - y)$$

wherein:

   $G = (\Sigma^y)^{-1}$ is a weight matrix;
   $\Sigma^y$ is a covariance matrix;
   *X* is a spectral matrix (spillover matrix);
   *y* is measured detector values from the plurality of photodetectors of the light detection system for each cell;
   *f* is true fluorophore abundances for each cell; and
   $\hat{f}$ is estimated (unmixed) fluorophore abundances for each cell.

70. The system according to any one of clauses 48-69, wherein the memory comprises instructions stored thereon, which when executed by the processor, cause the processor to generate a *priori* an estimated covariance matrix.

71. The system according to any one of clauses 48-70, wherein the memory comprises instructions stored thereon, which when executed by the processor, cause the processor to determine data signal covariance by estimating by iterative optimization with a covariance matrix that minimizes variance of unmixed data signals.

72. The system according to any one of clauses 48-71, wherein the generalized least squares problem comprises Cholesky decomposition of the covariance matrix.

73. The system according to any one of clauses 48-72, wherein the memory comprises instructions stored thereon, which when executed by the processor, cause the processor to find the least-squares solution of the generalized least squares problem.

74. The system according to any one of clauses 48-73, wherein the memory comprises instructions stored thereon, which when executed by the processor, cause the processor to find the least-squares solution of the generalized least squares problem by one or more of matrix decomposition, matrix factorization, QR factorization, Cholesky decomposition, singular value decomposition, LDL decomposition, and forward- and back-substitution.

75. The system according to clause 74, wherein the memory comprises instructions stored thereon, which when executed by the processor, cause the processor to find the least-squares solution of the generalized least squares problem by Cholesky decomposition.

76. The system according to clause 75, wherein the memory comprises instructions stored thereon, which when executed by the processor, cause the processor to find the least-squares solution of the generalized least squares problem by Cholesky decomposition according to:

$$X^T G X \hat{f} = X^T G y$$

wherein:

$y$ is measured detector values from the plurality of photodetectors of the light detection system for each cell;
$X$ is spillover; and

$$G \text{ is } \begin{bmatrix} G_{1,1} & G_{1,2} & \cdots & G_{1,m} \\ G_{2,1} & G_{2,2} & \cdots & G_{2,m} \\ \vdots & \vdots & \ddots & \vdots \\ G_{m,1} & G_{m,2} & \cdots & G_{m,m} \end{bmatrix}$$

77. The system according to any one of clauses 75-76, wherein the memory comprises instructors stored thereon, which when executed by the processor, cause the processor to find the least-squares solution of the generalized least squares problem by Cholesky or LDL decomposition according to:

$$X^T G X \hat{f} = X^T G y$$

| | |
|---|---|
| $A\hat{f} = B$ | |
| $LDL^T \hat{f} = B$ | LDL decomposition |
| $Lz = B$ where $z = DL^T \hat{f}$ | Lower-triangular matrix solution |
| $Dx = z$ where $x = L^T \hat{f}$ | Diagonal matrix solution |
| $L^T \hat{f} = x$, solve for $\hat{f}$ | Upper-triangular matrix solution |

78. The system according to clause 74, wherein the memory comprises instructions stored thereon, which when executed by the processor, cause the processor to find the least-squares solution of the generalized least squares problem by matrix decomposition.

79. The system according to clause 74, wherein the memory comprises instructions stored thereon, which when executed by the processor, cause the processor to find the least-squares solution of the generalized least squares problem by matrix factorization.

80. The system according to clause 74, wherein the memory comprises instructions stored thereon, which when executed by the processor, cause the processor to find the least-squares solution of the generalized least squares problem by QR factorization.

81. The system according to clause 80, wherein the memory comprises instructions stored thereon, which when executed by the processor, cause the processor to calculate the generalized least squares problem using transformed $\overline{X}$ and $\overline{y}$ using QR factorization according to:

| | |
|---|---|
| $\overline{X}^T \overline{X} f = \overline{X}^T \overline{y}$ | Normal equations for transformed GLS problem |
| $QR = \overline{X}$ | QR decomposition of transformed $X$ |
| $(QR)^T QR \hat{f} = (QR)^T \overline{y}$ | Substitution |
| $R^T Q^T QR \hat{f} = R^T Q^T \overline{y}$ | Expand transpose of $QR$ |
| $R^T R \hat{f} = R^T Q^T \overline{y} Q^T Q$ cancels | due to orthogonality |
| $R \hat{f} = Q^T \overline{y}.$ | Triangular solve for $\hat{f}$. |

82. The system according to clause 74, wherein the memory comprises instructions stored thereon, which when executed by the processor, cause the processor to find the least-squares solution of the generalized least squares problem by singular value decomposition.

83. The system according to clause 82, wherein the memory comprises instructions stored thereon, which when executed by the processor, cause the processor to calculate the generalized least squares problem using singular value decomposition according to:

$$z = U^T \overline{y}$$
$$\Sigma w = z$$

$$\hat{f} = Vw,$$

wherein *U* and *V* are orthogonal matrices and *Σ* is a diagonal matrix containing singular values of $\overline{X}$.

84. The system according to clause 74, wherein the memory comprises instructions stored thereon, which when executed by the processor, cause the processor to find the least-squares solution of the generalized least squares problem by LDL decomposition.

85. The system according to clause 74, wherein the memory comprises instructions stored thereon, which when executed by the processor, cause the processor to find the least-squares solution of the generalized least squares problem by forward- and back-substitution.

86. The system according to any one of clauses 48-85, wherein the system is configured to spectrally resolve light from each fluorophore in real time.

87. The system according to any one of clauses 48-86, wherein the system further comprises an integrated circuit.

88. The system according to clause 87, wherein the integrated circuit comprises a field programmable gate array (FPGA).

89. The system according to any one of clauses 48-88, wherein the light source comprises a laser.

90. The system according to 89, wherein the light source comprises a plurality of lasers.

91. An integrated circuit programmed to spectrally resolve light from each fluorophore in a sample comprising a plurality of fluorophores having overlapping fluorescence spectra using a generalized least squares problem.

92. The integrated circuit according to clause 91, wherein the integrated circuit is a field programmable gate array (FPGA).

93. The integrated circuit according to clause 91, wherein the integrated circuit is an application specific integrated circuit (ASIC).

94. The integrated circuit according to clause 91, wherein the integrated circuit is a complex programmable logic device (CPLD).

95. The integrated circuit according to any one of clauses 91-94, wherein the integrated circuit is programmed to determine data signal covariance in each photodetector channel of a light detection system.

96. The integrated circuit according to clause 95, wherein the data signal covariance in each photodetector channel comprises:

an intrinsic sample variability component; and
a measurement variability component.

97. The integrated circuit according to any one of clauses 95-96, wherein the data signal covariance comprises electronic noise in each photodetector channel.

98. The integrated circuit according to any one of clauses 95-96, wherein the data signal covariance comprises shot noise in each photodetector channel.

99. The integrated circuit according to any one of clauses 95-98, wherein the data signal covariance varies linearly with the generated data signals.

100. The integrated circuit according to any one of clauses 95-98, wherein the data signal covariance varies quadratically with the generated data signals.

101. The integrated circuit according to any one of clauses 95-98, wherein the data signal covariance is correlated across two or more of the plurality of photodetector channels.

102. The integrated circuit according to any one of clauses 95-101, wherein the integrated circuit is programmed to calculate covariance according to:

$$V[y_i] = \sigma_{B,i}^2 + Q_i y_i + (CV_{0,i})^2 y_i^2$$

wherein:

$y_i$ is a measured detector signal;

$\sigma_{B,i}^2$ is a baseline noise component;

$Q_i y_i$ is a Poisson noise component;

$Q_i$ is a Poisson noise coefficient;

$(CV_{0,i})^2 y_i^2$ is a quadratic noise component; and

$CV_{0,i}$ is a quadratic noise coefficient.

103. The integrated circuit according to any one of clauses 95-102, wherein the integrated circuit is programmed to calculate the data signal covariance using a covariance matrix.

104. The integrated circuit according to clause 103, wherein the covariance matrix contains non-zero diagonal values.

105. The integrated circuit according to any one of clauses 103-104, wherein the integrated circuit is programmed to calculate covariance with a covariance matrix according to:

$$V[y] = \Sigma^y = \begin{bmatrix} V[y_1] & cov(y_1, y_2) & \cdots & cov(y_1, y_m) \\ cov(y_2, y_1) & V[y_2] & \cdots & cov(y_2, y_m) \\ \vdots & \vdots & \ddots & \vdots \\ cov(y_m, y_1) & cov(y_m, y_2) & \cdots & V[y_m] \end{bmatrix}$$

$$= diag(\sigma_B^2) + diag(Q)diag(y) + \begin{bmatrix} CV_{0,1}^2 y_1^2 & cov(y_1, y_2) & \cdots & cov(y_1, y_m) \\ cov(y_2, y_1) & CV_{0,2}^2 y_2^2 & \cdots & cov(y_2, y_m) \\ \vdots & \vdots & \ddots & \vdots \\ cov(y_m, y_1) & cov(y_m, y_2) & \cdots & CV_{0,m}^2 y_m^2 \end{bmatrix}$$

wherein $diag(x)$ indicates generating a diagonal matrix from the column vector $x$, and $cov(a, b)$ denotes the covariance between $a$ and $b$.

106. The integrated circuit according to any one of clauses 103-105, wherein the integrated circuit is programmed to calculate the generalized least squares problem by multiplying the data signals by the inverse of the calculated covariance matrix.

107. The integrated circuit according to any one of clauses 91-106, wherein the integrated circuit is programmed to estimate data signal covariance based on fluorescence intensity across two or more photodetectors of the light detection system.

108. The integrated circuit according to any one of clauses 91-106, wherein the integrated circuit is programmed to estimate data signal covariance based on fluorescence intensity across each photodetection channel of the light detection system.

109. The integrated circuit according to any one of clauses 91-108, wherein the integrated circuit is programmed to calculate the generalized least squares problem is according to:

$$\hat{f} = \arg\min_f (Xf - y)^T G(Xf - y)$$

wherein:

$G = (\Sigma^y)^{-1}$ is a weight matrix;
$\Sigma^y$ is a covariance matrix;
$X$ is a spectral matrix (spillover matrix);
$y$ is measured detector values from the plurality of photodetectors of the light detection system for each cell;
$f$ is true fluorophore abundances for each cell; and

$\hat{f}$ is estimated (unmixed) fluorophore abundances for each cell.

110. The integrated circuit according to any one of clauses 91-109, wherein the integrated circuit is programmed to generate a *priori* an estimated covariance matrix.

111. The integrated circuit according to any one of clauses 91-110, wherein the integrated circuit is programmed to determine data signal covariance by estimating by iterative optimization with a covariance matrix that minimizes variance of unmixed data signals.

112. The integrated circuit according to any one of clauses 91-111, wherein the generalized least squares problem comprises Cholesky decomposition of the covariance matrix.

113. The integrated circuit according to any one of clauses 91-112, wherein the integrated circuit is programmed to find the least-squares solution of the generalized least squares problem.

114. The integrated circuit according to any one of clauses 91-113, wherein the integrated circuit is programmed to find the least-squares solution of the generalized least squares problem by one or more of matrix decomposition, matrix factorization, QR factorization, Cholesky decomposition, singular value decomposition, LDL decomposition, and forward- and back-substitution.

115. The integrated circuit according to clause 114, wherein the integrated circuit is programmed to find the least-squares solution of the generalized least squares problem by Cholesky or LDL decomposition.

116. The integrated circuit according to clause 115, wherein the integrated circuit is programmed to find the least-squares solution of the generalized least squares problem by Cholesky decomposition according to:

$$X^T G X \hat{f} = X^T G y$$

wherein:

y is measured detector values from the plurality of photodetectors of the light detection system for each cell;
X is spillover; and

$$G \text{ is } \begin{bmatrix} G_{1,1} & G_{1,2} & \cdots & G_{1,m} \\ G_{2,1} & G_{2,2} & \cdots & G_{2,m} \\ \vdots & \vdots & \ddots & \vdots \\ G_{m,1} & G_{m,2} & \cdots & G_{m,m} \end{bmatrix}$$

117. The integrated circuit according to any one of clauses 115-116, wherein the integrated circuit is programmed to find the least-squares solution of the generalized least squares problem by Cholesky or LDL decomposition according to:

$$X^T G X \hat{f} = X^T G y$$

| | |
|---|---|
| $A\hat{f} = B$ | |
| $LDL^T \hat{f} = B$ | LDL decomposition |
| $Lz = B$ where $z = DL^T\hat{f}$ | Lower-triangular matrix solution |
| $Dx = z$ where $x = L^T\hat{f}$ | Diagonal matrix solution |
| $L^T\hat{f} = x$, solve for $\hat{f}$ | Upper-triangular matrix solution |

118. The integrated circuit according to 114, wherein the integrated circuit is programmed to find the least-squares solution of the generalized least squares problem by matrix decomposition.

119. The integrated circuit according to clause 114, wherein the integrated circuit is programmed to find the least-squares solution of the generalized least squares problem by matrix factorization.

120. The integrated circuit according to clause 114, wherein the integrated circuit is programmed to find the least-squares solution of the generalized least squares problem by QR factorization.

121. The integrated circuit according to clause 120, wherein the generalized least squares problem is calculated using transformed $\overline{X}$ and $\overline{y}$ using QR factorization according to:

| | |
|---|---|
| $\overline{X}^T\overline{X}f = \overline{X}^T\overline{y}$ | Normal equations for transformed GLS problem |
| $QR = X$ | QR decomposition of transformed X |
| $(QR)^T QR\hat{f} = (QR)^T\overline{y}$ | Substitution |
| $R^T Q^T QR\hat{f} = R^T Q^T\overline{y}$ | Expand transpose of QR |
| $R^T R\hat{f} = R^T Q^T\overline{y}Q^T Q$ | cancels due to orthogonality |
| $R\hat{f} = Q^T\overline{y}$. | Triangular solve for $\hat{f}$. |

122. The integrated circuit according to clause 114, wherein the integrated circuit is programmed to find the least-squares solution of the generalized least squares problem by singular value decomposition.

123. The integrated circuit according to clause 122, wherein the integrated circuit is programmed to calculate the generalized least squares problem using singular value decomposition according to:

$$z = U^T\overline{y}$$

$$\Sigma w = z$$

$$\hat{f} = Vw,$$

wherein $U$ and $V$ are orthogonal matrices and $\Sigma$ is a diagonal matrix containing singular values of $\overline{X}$.

124. The integrated circuit according to clause 114, wherein the integrated circuit is programmed to find the least-squares solution of the generalized least squares problem by LDL decomposition.

125. The integrated circuit according to clause 114, wherein the integrated circuit is programmed to find the least-squares solution of the generalized least squares problem by forward- and back-substitution.

126. The integrated circuit according to any one of clauses 91-125, wherein the integrated circuit is programmed to spectrally resolve light from each fluorophore in real time.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

Accordingly, the preceding merely illustrates the principles of the invention. It will be appreciated that those skilled in the art will be able to devise various arrangements which, although not explicitly described or shown herein, embody the principles of the invention and are included within its spirit and scope. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. Moreover, nothing disclosed herein is intended to be dedicated to the public regardless of whether such disclosure is explicitly recited in the claims.

The scope of the present invention, therefore, is not intended to be limited to the exemplary embodiments shown and described herein. Rather, the scope and spirit of present invention is embodied by the appended claims. In the claims, 35 U.S.C. §112(f) or 35 U.S.C. §112(6) is expressly defined as being invoked for a limitation in the claim only when the exact phrase "means for" or the exact phrase "step for" is recited at the beginning of such limitation in the claim; if such exact phrase is not used in a limitation in the claim, then 35 U.S.C. § 112 (f) or 35 U.S.C. §112(6) is not invoked.

## Claims

1. A method comprising:

   detecting light with a light detection system from a sample comprising a plurality of fluorophores having overlapping fluorescence spectra;
   spectrally resolving light from each fluorophore in the sample using a generalized least squares algorithm.

2. The method according to claim 1, wherein light is detected by the light detection system in a plurality of photodetector channels.

3. The method according to any one of claims 1-2, wherein the light detection system comprises a plurality of photodetectors.

4. The method according to any one of claims 2-3, wherein the method comprises generating data signals in each of the photodetector channels in response to the detected light.

5. The method according to claim 4, wherein the method comprises determining data signal covariance in each photodetector channel.

6. The method according to claim 5, wherein the data signal covariance in each photodetector channel comprises:

   an intrinsic sample variability component; and
   a measurement variability component.

7. The method according to any one of claims 5-6, wherein the data signal covariance is correlated across two or more of the plurality of photodetector channels.

8. The method according to any one of claims 5-7, wherein the method comprises calculating the data signal covariance using a covariance matrix.

9. The method according to claim 8, wherein the covariance matrix contains non-zero diagonal values.

10. The method according to any one of claims 5-9, wherein the method comprises estimating data signal covariance based on fluorescence intensity across two or more photodetectors of the light detection system.

11. The method according to any one of claims 1-10, wherein the method comprises finding the least-squares solution of the generalized least squares problem.

12. The method according to any one of claims 1-11, wherein the method comprises minimizing the least-squares solution of the generalized least squares problem by one or more of matrix decomposition, matrix factorization, QR factorization, Cholesky decomposition, singular value decomposition, LDL decomposition, and forward- and back-substitution.

13. The method according to any one of claims 1-12, wherein the method comprises spectrally resolving light from each fluorophore in real time.

14. A system comprising:

a light source configured to irradiate a sample comprising a plurality of fluorophores having overlapping fluorescence spectra;
a light detection system comprising a plurality of photodetectors; and
a processor comprising memory operably coupled to the processor wherein the memory comprises instructions stored thereon, which when executed by the processor, cause the processor to spectrally resolve light from each fluorophore in the sample using a generalized least squares algorithm.

15. An integrated circuit programmed to spectrally resolve light from each fluorophore in a sample comprising a plurality of fluorophores having overlapping fluorescence spectra using a generalized least squares problem.

Data signals from
Detected Light        **101**

**Determine Covariance between Photodetectors**
Include non-zero quadratic covariance terms in noise
model        **102**

Spectrally
Resolved
Data signals        **103**

**Figure 1A**

# Raw measurement variance is a quadratic function of intensity

$$\mathbb{V}[y_i] = \underbrace{\sigma^2_{B,i}}_{\substack{\text{baseline}\\\text{noise}}} + \underbrace{Q_i y_i}_{\substack{\text{Poisson}\\\text{noise}}} + \underbrace{(CV_{0,i})^2 y_i^2}_{\substack{\text{Quadratic CV}\\\text{noise}}}$$

**Figure 1B**

# The quadratic noise coefficients (CV terms) can be inferred by fitting a curve of variance vs median intensity

EP 4 589 282 A1

$$\text{baseline noise} \qquad \text{Poisson noise} \qquad \text{Quadratic CV noise}$$

$$\mathbb{V}[y_i] = \sigma_{B,i}^2 + Q_i y_i + (CV_{0,i})^2 y_i^2$$

**Figure 1C**

# Inclusion of the quadratic term with zero covariance consistently overestimates unmixed spread

Variance normalized to source fluorophore intensity

- X-axis: predicted spread (variance) assuming zero covariance
- Y-axis: measured spread
- Each point corresponds to a different fluorophore whose expression causes spread (measured on a sample of CD4+ cells exclusively expression that fluorophore)

**Without correlated noise:**
Simulation based on model overestimates spread

**Figure 1D**

Including nonzero quadratic covariance terms in the noise model leads to accurate prediction of unmixed spread

Figure 1D continued

# Mechanism for correlation and anticorrelation in quadratic noise due to nonuniform illumination and fluidic fluctuations

**Positive correlation:**
- Detectors on the same laser line will tend to all get brighter or dimmer together
- If lasers are aligned relative to each other, a particle's trajectory will tend to pass through a brighter area or a dimmer area on all laser lines together, leading to correlation across laser lines

**Negative correlation:**
- If lasers are not aligned to each other perfectly, depending on particle trajectory, detectors on some lasers may get dimmer at the same time as detectors on other lasers get brighter

Trajectory 1:
Brighter on Laser 1
Brighter on Laser 2
Brighter on Laser 3

Trajectory 2:
Dimmer on Laser 1
Dimmer on Laser 2
Dimmer on Laser 3

Laser 1

Laser 2

Laser 3

Trajectory 1:
Brighter on Laser 1
Dimmer on Laser 2
Brighter on Laser 3

Trajectory 2:
Dimmer on Laser 1
Brighter on Laser 2
Dimmer on Laser 3

**Figure 2A**

EP 4 589 282 A1

Example of generalized least squares unmixing compared to ordinary least squares and weighted least squares in the presence of nonzero noise covariance in a simulated panel

Figure 2B

EP 4 589 282 A1

Figure 3A

**Figure 3B**

**Figure 4A**

EP 4 589 282 A1

**Figure 4B**

EP 4 589 282 A1

Figure 5

500

506

508

510

590

504

502

**Figure 6A**

**Figure 6B**

COMPUTING SYSTEM 700

PROCESSING UNIT 710

NETWORK INTERFACE 720

COMPUTER READABLE MEDIUM 730

INPUT/OUTPUT DEVICE INTERFACE 740

MEMORY 770

OPERATING SYSTEM 772

DISPLAY (OPTIONAL) 750

INPUT DEVICE (OPTIONAL) 760

DATA STORE 790

**Figure 7**

EP 4 589 282 A1

## EUROPEAN SEARCH REPORT

**Application Number**

EP 24 22 2775

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/062339 A1 (MAGE PETER [US] ET AL) 2 March 2023 (2023-03-02) * figure 2 * * paragraph [0005] - paragraph [0007] * * paragraph [0084] - paragraph [0101] * * paragraph [0113] - paragraph [0133] * * paragraph [0166] - paragraph [0182] * | 1-9, 11-15 | INV. G01N15/1429 G01N15/149 G01N21/64 |
| X | EBERHARD W L ET AL: "LIDAR DISCRIMINATION OF MULTIPLE FLUORESCENT TRACERS OF ATMOSPHERICMOTIONS", APPLIED OPTICS, OPTICAL SOCIETY OF AMERICA, WASHINGTON, DC, US, vol. 28, no. 15, 1 August 1989 (1989-08-01), pages 2996-3007, XP000073226, ISSN: 0003-6935, DOI: 10.1364/AO.28.002996 * figure 1 * * page 2996 - page 3005 * | 1-15 | |
| A | Anonymous: "Generalized least squares - Wikipedia", , 13 December 2023 (2023-12-13), XP093281069, Retrieved from the Internet: URL:https://web.archive.org/web/2023121309 4056/https://en.wikipedia.org/wiki/General ized_least_squares * the whole document * | 1-15 | **TECHNICAL FIELDS SEARCHED (IPC)** G01N |
| A | US 2023/393049 A1 (MAGE PETER [US]) 7 December 2023 (2023-12-07) * paragraph [0064] - paragraph [0065] * | 1-15 | |
| A | EP 1 063 502 A1 (EVOTEC BIOSYSTEMS AG [DE]) 27 December 2000 (2000-12-27) * paragraph [0050] - paragraph [0053] * | 1-15 | |

The present search report has been drawn up for all claims

| Place of search | Date of completion of the search | Examiner |
|---|---|---|
| The Hague | 27 May 2025 | Lefortier, Stéphanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 22 2775

This annex lists the patent family members relating to the patent documents cited in the above-mentioned European search report.
The members are as contained in the European Patent Office EDP file on
The European Patent Office is in no way liable for these particulars which are merely given for the purpose of information.

27-05-2025

| Patent document cited in search report | | Publication date | Patent family member(s) | | Publication date |
|---|---|---|---|---|---|
| US 2023062339 | A1 | 02-03-2023 | AU | 2019416121 A1 | 20-05-2021 |
| | | | CN | 113439205 A | 24-09-2021 |
| | | | CN | 118980626 A | 19-11-2024 |
| | | | EP | 3903095 A1 | 03-11-2021 |
| | | | JP | 7528087 B2 | 05-08-2024 |
| | | | JP | 2022516233 A | 25-02-2022 |
| | | | JP | 2024156737 A | 06-11-2024 |
| | | | KR | 20210097807 A | 09-08-2021 |
| | | | US | 2020209064 A1 | 02-07-2020 |
| | | | US | 2021239530 A1 | 05-08-2021 |
| | | | US | 2023062339 A1 | 02-03-2023 |
| | | | US | 2024369411 A1 | 07-11-2024 |
| | | | WO | 2020139848 A1 | 02-07-2020 |
| US 2023393049 | A1 | 07-12-2023 | CN | 119422203 A | 11-02-2025 |
| | | | EP | 4533465 A1 | 09-04-2025 |
| | | | US | 2023393049 A1 | 07-12-2023 |
| | | | WO | 2023235203 A1 | 07-12-2023 |
| EP 1063502 | A1 | 27-12-2000 | NONE | | |

EPO FORM P0459

For more details about this annex : see Official Journal of the European Patent Office, No. 12/82

## REFERENCES CITED IN THE DESCRIPTION

**Patent documents cited in the description**

- US 63622370
- US 9423353 B
- US 9784661 B
- US 9983132 B
- US 10006852 B
- US 10078045 B
- US 10036699 B
- US 10222316 B
- US 10288546 B
- US 10324019 B
- US 10408758 B
- US 10451538 B
- US 10620111 B
- US 20170133857 A
- US 20170328826 A
- US 20170350803 A
- US 20180275042 A
- US 20190376895 A
- US 20190376894 A
- US 20170299493 A
- US 62803264
- US 10663476 B
- US 10613017 B
- US 10605713 B
- US 10585031 B
- US 10578542 B
- US 10578469 B
- US 10481074 B
- US 10302545 B
- US 10145793 B
- US 10113967 B
- US 9952076 B
- US 9933341 B
- US 9726527 B
- US 9453789 B
- US 9200334 B
- US 9097640 B
- US 9095494 B
- US 9092034 B
- US 8975595 B
- US 8753573 B
- US 8233146 B
- US 8140300 B
- US 7544326 B
- US 7201875 B
- US 7129505 B
- US 6821740 B
- US 6813017 B
- US 6809804 B
- US 6372506 B
- US 5700692 A
- US 5643796 A
- US 5627040 A
- US 5620842 A
- US 5602039 A
- US 4987086 A
- US 4498766 A
- US 20200256781 A
- US 20210404943 A
- US 7679039 B

**Non-patent literature cited in the description**

- **DIEBOLD et al.** *Nature Photonics*, 2013, vol. 7 (10), 806-810
- Flow Cytometry: A Practical Approach. Oxford Univ. Press, 1997
- Flow Cytometry Protocols, Methods in Molecular Biology. Humana Press, 1997
- Practical Flow Cytometry. Wiley-Liss, 1995
- **VIRGO et al.** *Ann Clin Biochem.*, 2012, vol. 49, 17-28
- **LINDEN**. *Semin Throm Hemost.*, October 2004, vol. 30 (5), 502-11
- **ALISON et al.** *J Pathol*, December 2010, vol. 222 (4), 335-344
- **HERBIG**. *Crit Rev Ther Drug Carrier Syst.*, 2007, vol. 24 (3), 203-255
- **SCHRAIVOGEL et al.** High-speed fluorescence image-enabled cell sorting. *Science*, 2022, vol. 375 (6578), 315-320